(19) Europäisches Patentamt
European Patent Office
Office européen des brevets

(11) **EP 3 834 789 B1**

(12) **EUROPEAN PATENT SPECIFICATION**

(45) Date of publication and mention
of the grant of the patent:
**18.09.2024 Bulletin 2024/38**

(21) Application number: **19846609.6**

(22) Date of filing: **09.08.2019**

(51) International Patent Classification (IPC):
*A61F 13/53* (2006.01)   *A61L 15/60* (2006.01)
*B01J 20/26* (2006.01)   *B01J 20/28* (2006.01)
*C08L 101/14* (2006.01)   *A61F 13/534* (2006.01)

(52) Cooperative Patent Classification (CPC):
**A61F 13/534; A61L 15/60; B01J 20/267;**
**B01J 20/28011; B01J 20/28038; B01J 20/28054;**
**B01J 20/28057;** A61F 2013/530547;
A61F 2013/53445; B01J 2220/68

(86) International application number:
**PCT/JP2019/031782**

(87) International publication number:
**WO 2020/032280 (13.02.2020 Gazette 2020/07)**

(54) **WATER-ABSORBING SHEET AND WATER-ABSORBING ARTICLE INCLUDING THE SAME**

WASSERABSORBIERENDE FOLIE UND DIESE ENTHALTENDER WASSERABSORBIERENDER ARTIKEL

FEUILLE ABSORBANT L'EAU ET ARTICLE ABSORBANT L'EAU COMPRENANT CELLE-CI

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **09.08.2018 JP 2018150124**
**09.08.2018 JP 2018150125**
**09.08.2018 JP 2018150129**
**28.09.2018 JP 2018185701**
**28.09.2018 JP 2018185706**

(43) Date of publication of application:
**16.06.2021 Bulletin 2021/24**

(73) Proprietor: **Nippon Shokubai Co., Ltd.**
**Osaka-shi, Osaka 541-0043 (JP)**

(72) Inventors:
• **NODA, Yuika**
**Himeji-shi, Hyogo 671-1282 (JP)**
• **TORII, Kazushi**
**Himeji-shi, Hyogo 671-1282 (JP)**
• **UEDA, Hiroko**
**Himeji-shi, Hyogo 671-1282 (JP)**
• **KITANO, Takahiro**
**Himeji-shi, Hyogo 671-1282 (JP)**
• **HIRAUCHI, Tatsushi**
**Himeji-shi, Hyogo 671-1282 (JP)**
• **HORIMOTO, Yuichiro**
**Himeji-shi, Hyogo 671-1282 (JP)**
• **HIRAOKA, Ryuichi**
**Himeji-shi, Hyogo 671-1282 (JP)**
• **IKEUCHI, Hiroyuki**
**Himeji-shi, Hyogo 671-1282 (JP)**

(74) Representative: **Hoffmann Eitle**
**Patent- und Rechtsanwälte PartmbB**
**Arabellastraße 30**
**81925 München (DE)**

(56) References cited:
**EP-A1- 1 730 219**      **EP-B1- 1 730 219**
**WO-A1-2013/099634**   **WO-A1-2013/099634**
**WO-A1-2016/204302**   **JP-A- 2006 055 833**
**JP-A- 2012 218 320**    **JP-U- H0 659 039**
**US-A1- 2012 203 191**

## Description

### TECHNICAL FIELD

The present invention relates to a water-absorbing sheet and an absorbent article including the same.

### BACKGROUND ART

A water-absorbing resin (SAP/Super Absorbent polymer) is a water-swellable, water-insoluble polymer gelling agent. Water-absorbing resins are utilized in various use applications, including hygienic materials such as paper diapers, sanitary napkins, and incontinence products for adults, soil water-retaining agents for agricultural and horticultural use, industrial waterproofing agents, and the like.

These absorbent articles are generally produced as absorbent bodies obtained by mixing a water-absorbing resin and a fiber material and individually mold-making each absorbent article in a paper diaper manufacturing plant, and the absorbent body is processed into absorbent bodies having various shapes (for example, an hourglass shape, a fox shape, an oval shape, and the like as viewed in plan view) according to the purpose . Since the method for producing these absorbent bodies performs mold-making for individual articles, the absorbent bodies can be processed into any arbitrary form, and since it is also easy to adjust the amount of fibers or the water-absorbing resins for each absorbent article, the absorbent articles constitute the current mainstream of paper diapers.

However, in recent years, in the production of paper diapers, paper diapers that use an absorbent body produced by cutting a long water-absorbing sheet having a water-absorbing resin immobilized between two pieces of a sheet, by a production process for a sanitary material (usually, cut into a rectangular shape having a width of approximately 10 cm and a length of several dozen cm), have been produced. Paper diaper manufacturers can simplify the production process for paper diapers by purchasing or producing a long continuous water-absorbing sheet and can also reduce the thickness of paper diapers without using pulp. A water-absorbing sheet adopts a configuration in which water-absorbing resin particles are sandwiched between upper and lower sheets (particularly, nonwoven fabric sheets) and immobilized. Usually, after a long continuous sheet is produced, the long continuous sheet is cut into a rectangular shape having a width of approximately 10 cm and a length of several dozen cm, and the cut sheet is incorporated into a paper diaper (for example, Patent Literature 1).

Unlike conventional sanitary materials (paper diapers), paper diapers based on water-absorbing sheets have a short history, and it is the current situation that the development of water-absorbing resins appropriate for the water-absorbing sheets and the suggestion of parameters mostly have not been achieved. Thus, water-absorbing resins that are exclusively used for conventional paper diapers are also directly used in water-absorbing sheets.
Patent Literatures 2-4 describe **a water-absorbing sheet comprising two base material layers, a water-absorbing layer being water-permeable and two particulate water absorbents.**

Citation List

Patent Literatures

>   Patent Literature 1: WO 2010/143635 A
>   Patent Literature 2: WO 2013/099634 A1
>   Patent Literature 3: US 2012/0203191 A1
>   Patent Literature 4: WO 2005/092956 A1

### SUMMARY OF INVENTION

The inventors of the present invention found that due to the structure in which being thin is characteristic of the mainstream water-absorbing sheets, so-called"reversion" may occur, by which a liquid that has been absorbed is released. Further, the inventors found that when introduction of a liquid occurs intermittently for several times (particularly, three or more times) under a situation with no load, and the amount of introduction of the liquid becomes large, the problem is notable.

Therefore, an object of the present invention is to provide a novel water-absorbing sheet that can meaningfully reduce the return amount even though the environment for liquid introduction is as described above.

The problems described above are solved by using a water-absorbing sheet having a first base material, a second base material, and a water-absorbing layer positioned between the first base material and the second base material, wherein the first base material is a water-permeable sheet positioned on a side where a liquid to be absorbed is introduced, the water-absorbing layer has a first particulate water absorbent localized on a side of a surface of the first base material, the surface being arranged to face the second base material; and a second particulate water absorbent localized on a side of a surface of the second base material, the surface being arranged to face the first base material, an overall absorption amount as represented by the following formula:

[Mathematical Formula 1]

CRC g/g + 0.44 × AAP2.1kPa g/g

of the second particulate water absorbent is 48 g/g or more, and a water absorption time determined according to a vortex method is 35 seconds or more,
a CRC of the second particulate water absorbent is 30 to 50 g/g, and an AAP2.1kPa is 15 to 35 g/g, wherein the CRC is measured according to ERT441.2-02 and the AAP2.1kPa is measured according to ERT442.2-02, and
the first and second particulate water absorbents are fixed to the first and second base materials by means of an adhesive.

## BRIEF DESCRIPTION OF DRAWINGS

Fig. 1 is a schematic diagram illustrating a cross-section of a water-absorbing sheet according to an embodiment of the present invention according to a first aspect of the present invention.
Fig. 2 is a schematic diagram illustrating an apparatus for measuring GPR according to the first aspect of the present invention.
Fig. 3 is a plan view and a right side view illustrating a sample used for an evaluation of the reversion amount according to the first aspect of the present invention and are diagrams illustrating the appearance of a water-absorbing sheet produced in an Example wrapped with a liquid-impermeable sheet.
Fig. 4 is a plan view and a front view of a liquid injection cylinder used for the evaluation of the reversion amount according to the first aspect of the present invention.
Fig. 5 is a front view illustrating the appearance of the liquid injection cylinder placed on the water-absorbing sheet used in the Examples of the present application, according to the first aspect of the present invention.
Fig. 6 is a front view and a right side view illustrating the appearance of the process of charging an aqueous solution of sodium chloride from the liquid injection cylinder to the water-absorbing sheet using a funnel in Fig. 5 according to the first aspect of the present invention.
Fig. 7 is a schematic diagram illustrating a cross-section of a water-absorbing sheet according to an embodiment of the present invention according to a second aspect of the present description.
Fig. 8 is a schematic diagram illustrating an apparatus for measuring GPR according to the second aspect of the present description.
Fig. 9 is a plan view and a right side view illustrating a sample used for an evaluation of the reversion amount according to the second aspect of the present description and are diagrams illustrating the appearance of a water-absorbing sheet produced in an Example wrapped with a liquid-impermeable sheet.
Fig. 10 is a plan view and a front view of a liquid injection cylinder used for the evaluation of the reversion amount according to the second aspect of the present description.
Fig. 11 is a front view illustrating the appearance of the liquid injection cylinder placed on the water-absorbing sheet used in the Examples of the present application, according to the second aspect of the present description.
Fig. 12 is a front view and a right side view illustrating the appearance of the process of charging an aqueous solution of sodium chloride from the liquid injection cylinder to the water-absorbing sheet using a funnel in Fig. 11 according to the second aspect of the present description.
Fig. 13 is an outline diagram illustrating an apparatus used at the time of measuring the permeability according to the second aspect of the present description.
Fig. 14 is a schematic diagram illustrating a cross-section of a water-absorbing sheet according to an embodiment of the present invention according to a third aspect of the present description.
Fig. 15 is a schematic diagram illustrating an apparatus for measuring GPR according to the third aspect of the present description.
Fig. 16 is a plan view and a right side view illustrating a sample used for an evaluation of the reversion amount according to the third aspect of the present description and are diagrams illustrating the appearance of a water-absorbing sheet produced in an Example wrapped with a liquid-impermeable sheet.

Fig. 17 is a plan view and a front view of a liquid injection cylinder used for the evaluation of the reversion amount according to the third aspect of the present description.

Fig. 18 is a front view illustrating the appearance of the liquid injection cylinder placed on the water-absorbing sheet used in the Examples of the present application, according to the third aspect of the present description.

Fig. 19 is a front view and a right side view illustrating the appearance of the process of charging an aqueous solution of sodium chloride from the liquid injection cylinder to the water-absorbing sheet using a funnel in Fig. 18 according to the third aspect of the present description.

Fig. 20 is an outline diagram illustrating an apparatus used at the time of measuring the permeability according to the third aspect of the present description.

## DESCRIPTION OF EMBODIMENTS

<First aspect of present invention>

Hereinafter, the present invention will be described bywayofthebestmodes. Throughout the present specification, unless particularly stated otherwise, any expression in a singular form should be understood to encompass the concept of its plural form. Therefore, unless particularly stated otherwise, the article specifying a single form (for example, "a", "an", "the", and the like in the case of English language) should be understood to encompass the concept of its plural form. Furthermore, unless particularly stated otherwise, any term used in the present specification should be understood as a term that is used to have the meaning conventionally used in the relevant technical field. Therefore, unless defined otherwise, all the technical terms and scientific terms used in the present specification have the same meaning as generally understood by a person ordinarily skilled in the art to which the present invention is pertained. If there is any conflict in meaning, the present specification (including the definitions) takes priority. The present invention is not intended to be limited to the following embodiments, and various modifications can be made within the scope of the claims.

[1. Definitions of terms]

[1-1. Water-absorbing sheet]

The "water-absorbing sheet" according to the present invention refers to a structure in which a water-absorbing resin (particulate water absorbent) is supported between two or more sheets of long base materials. The water-absorbing sheet may use an adhesive, or may use a hot melt adhesive, for the adhesion between the base materials and/or the adhesion between the base material and the particulate water absorbent. The water-absorbing sheet may include other components (a fiber component, an antibacterial agent, a deodorant, and the like) in addition to the particulate water absorbent. Furthermore, the water-absorbing sheet may also include another sheet in addition to the two pieces of sheets having the particulate water absorbent and the like interposed therebetween.

Usually, a water-absorbing sheet is in the form of a continuous sheet or a roll form obtained by winding the continuous sheet. When the water-absorbing sheet is used, a continuous sheet is cut out into an appropriate shape (rectangular shape or the like) and then is used as an absorbent body such as a disposable diaper. On the other hand, a conventional disposable diaper of a water-absorbing resin at a high concentration (for example, a disposable diaper in which the absorbent body is pulpless) uses an absorbent body that is mold-made for each sheet of the disposable diaper. Therefore, such an absorbent body differs from the water-absorbing sheet of the present invention in view of the technical properties.

[1-2. Water-absorbing resin]

The "water-absorbing resin" according to the present specification refers to a polymer gelling agent in which the water-swellability (CRC) defined in ERT441.2-02 is 5 g/g or more, and the water-soluble component (Ext) defined in ERT470.2-02 is 50% by mass or less.

The water-absorbing resin is preferably a hydrophilic crosslinked polymer by crosslink polymerizing an unsaturated monomer having a carboxyl group. The shape of the water-absorbing resin is a sheet form, a fibrous form, a film form, a particulate form, a gel form, or the like. The water-absorbing sheet according to an embodiment of the present invention uses a particulate water-absorbing resin.

The "water-absorbing resin" according to the present specification is not limited to an embodiment in which the total amount (100% by mass) is composed only of the water-absorbing resin. The water-absorbing resin may also be a water-absorbing resin composition including additives and the like. Furthermore, the "water-absorbing resin" according to the present specification is a concept that also includes intermediate bodies in the production process for the water-absorbing resin. For example, a hydrated gel-like crosslinked polymer after polymerization, a dried polymer after drying, a water-absorbing resin powder before surface crosslinking, and the like may also be described as the "water-absorbing resin".

As such, in the present specification, in addition to the water-absorbing resin itself, a water-absorbing resin composition and intermediate bodies may also be collectively referred to and described as "water-absorbing resin".

[1-3. Water absorbent, particulate water absorbent]

The "water absorbent" according to the present specification means an absorbent gelling agent for absorbing an aqueous liquid (liquid), the absorbent gelling agent including a water-absorbing resin as a main component. Here, the aqueous liquid (liquid) is not particularly limited so long as it is water as well as a liquid including water. Examples of the aqueous liquid that the water-absorbing sheet according to an embodiment of the present invention absorbs include urine, menstrual blood, sweat, and other body fluids.

The "particulate water absorbent" according to the present specification means a water absorbent in the form of particle (powdery form) (since the water-absorbing resin is included as a main component in the water absorbent, the particulate water absorbent corresponds to a water-absorbing resin in the form of particle) . In the concept of the "particulate water absorbent", a single grain of the particulate water absorbent and an aggregate of a plurality of particulate water absorbents are all included. The term "particulate" according to the present specification means having the form of particle. Here, the "particle" refers to a relatively small divided body of a material and has a size of several Å to several mm (see "Particles", "McGraw-Hill Dictionary of Scientific and Technical Terms, 3rd Edition", edited by McGraw-Hill Dictionary of Scientific and Technical Terms Editorial Board, Nikkan Kogyo Shimbun, Ltd., 1996, p. 1929) . Meanwhile, in the present specification, the "particulate water absorbent" may be described simply as "water absorbent".

The particulate water absorbent includes a water-absorbing resin as a polymer (alternatively, also referred to as particulate water-absorbing resin or water-absorbing resin particles) as a main component. The particulate water absorbent includes the water-absorbing resin as a polymer at a proportion of 60% to 100% by mass, preferably 70% to 100% by mass, more preferably 80% to 100% by mass, even more preferably 90 to 100% by mass, and particularly preferably 95% to 100% by mass. The remaining portion of the particulate water absorbent may optionally include water, additives (inorganic fine particles, polyvalent metal cations, and the like), and the like. Incidentally, the particulate water absorbents used in the Examples of the present application include 80% to 100% by mass of a water-absorbing resin.

That is, the upper limit of the water-absorbing resin in the particulate water absorbent is, for example, 100% by mass, 99% by mass, 97% by mass, 95% by mass, or 90% by mass. Preferably, the particulate water absorbent further includes, in addition to the water-absorbing resin, 0% to 10% by mass of components, particularly water, additives (inorganic fine particles, polyvalent metal cations), and the like.

Incidentally, a preferred percentage water content of the particulate water absorbent is 0.2% to 30% by mass. As described above, a water-absorbing resin composition in which components such as water and additives are integrated and/or mixed with the water-absorbing resin, is also included in the "particulate water absorbent".

Examples of the water-absorbing resin that serves as a main component of the particulate water absorbent include a polyacrylic acid (salt)-based resin, a polysulfonic acid (salt)-based resin, a maleic anhydride (salt)-based resin, a polyacrylamide-based resin, a polyvinylalcohol-based resin, a polyethylene oxide-based resin, a polyaspartic acid (salt)-based resin, a polyglutamic acid (salt)-based resin, a polyalginic acid (salt) -based resin, a starch-based resin, and a cellulose-based resin. Among these, preferably, a polyacrylic acid (salt)-based resin is used as the water-absorbing resin.

[1-4. Polyacrylic acid (salt)]

The "polyacrylic acid (salt)" according to the present specification refers to polyacrylic acid and/or a salt thereof . The polyacrylic acid (salt) is a polymer that includes a repeating unit of acrylic acid and/or a salt thereof (hereinafter, referred to as "acrylic acid (salt) ") as a main component and further includes a graft component as an optional component. The polyacrylic acid (salt) is obtained by polymerization of acrylic acid (salt), hydrolysis of polyacrylamide, polyacrylonitrile, or the like, and the like. Preferably, the polyacrylic acid (salt) is obtained by polymerization of acrylic acid (salt).

Here, the phrase "includes as a main component" implies that the amount of use of acrylic acid (salt) at the time of polymerizing polyacrylic acid (salt) is usually 50 mol% to 100 mol%, preferably 70 mol% to 100 mol%, more preferably 90 mol% to 100 mol%, and even more preferably substantially 100mol%, with respect to the entirety of the monomers (however, excluding an internal crosslinking agent) used for polymerization.

[1-5. EDANA and ERT]

"EDANA" is an acronym for the European Disposables and Nonwovens Associations. "ERT" is an acronym for methods for measuring a water-absorbing resin of the European standards (substantially, international standards) enacted by EDANA (EDANA Recommended Test Methods). In the present specification, unless particularly stated otherwise, the physical properties of the water-absorbing resin are measured according to ERT of the version of year 2002.

[1-6. Others]

According to the present specification, the expression "X to Y" representing a range means "X or more and Y or less" .

According to the present specification, unless particularly annotated otherwise, a unit of mass, "t (ton) ", means "Metric ton". The unit "ppm" means "ppm by mass". The "mass" and "weight", "parts by mass" and "parts by weight", "% by mass" and "% by weight", and "ppm by mass" and "ppm by weight" are respectively considered to have the same meanings.

According to the present specification, the term "acid (salt)" means "acid and/or a salt thereof". The term "(meth)acryl" means "acryl and/or methacryl".

According to the present specification, the volume unit "liter" may be described as "I" or "L". The unit "% by mass" may be described as "wt%". In a case in which measurement of trace components is carried out, the detection limit or less is described as N.D. (Non Detected).

[2. Water-absorbing sheet]

The water-absorbing sheet of the present invention is a water-absorbing sheet having a first base material, a second base material, and a water-absorbing layer positioned between the first base material and the second base material, wherein the first base material is a water-permeable sheet positioned on the side where a liquid to be absorbed is introduced, the water-absorbing layer has a first particulate water absorbent localized on the side of a surface of the first base material, the surface being arranged to face the second base material; and a second particulate water absorbent localized on the side of a surface of the second base material, the surface being arranged to face the first base material, the overall absorption amount as represented by the following formula:

$$[Mathematical\ Formula\ 2]$$
$$CRC\ g/g + 0.44 \times AAP2.1kPa\ g/g$$

of the second particulate water absorbent is 48 g/g or more, and the water absorption time determined by a vortex method is 35 seconds or more,
a CRC of the second particulate water absorbent is 30 to 50 g/g, and an AAP2.1kPa is 15 to 35 g/g, wherein the CRC is measured according to ERT441.2-02 and the AAP2.1kPa is measured according to ERT442.2-02, and
the first and second particulate water absorbents are fixed to the first and second base materials by means of an adhesive. Due to such a configuration, even when introduction of a liquid occurs intermittently for several times (particularly, three or more times) under a situation with no load and the amount of introduction of the liquid becomes large, the return amount can be meaningfully reduced. Particularly, due to such a configuration, very excellent results can be obtained in the measurement of the return amount under particular conditions in the Examples of the present application (in the present specification, also referred to as "evaluation of the specific return amount") . That is, when there is intermittent introduction of a liquid for several times (particularly, three or more times), in a conventional configuration, the liquid amount is more than or equal to the set amount of absorption, and excessive "return" occurs. In contrast, in the present invention, by utilizing an advantage that a particulate water absorbent is formed into a sheet (layer) form, the present invention includes a configuration of maximally utilizing the area of the particulate water absorbent (particularly, a layer of the second particulate water absorbent). More specifically speaking, since the water absorption time of the second particulate water absorbent as determined according to a vortex method is meaningfully slow, the diffusibility is high, and the second particulate water absorbent whose total amount of absorption is meaningfully high, so to speak, accomplishing a tank-like function, absorbs a diffused liquid (for example, urine) so as not to spill. Then, even if the liquid that the second particulate water absorbent could not absorb tries to go back, due to the presence of the first particulate water absorbent, the liquid can be prevented from rising up to the skin. Therefore, the "evaluation of the specific return amount" can be achieved with excellence. Here, it should be additionally remarked that a water-absorbing sheet or an absorbent article, which has been designed so as to suppress the return amount under general conditions, does not necessarily give excellent results in regard to the "evaluation of the specific return amount" of the present application. Furthermore, the water-absorbing sheet according to an embodiment of the present invention is suitable as, for example, an absorbent article (for example, a diaper) which an infant who has just learned to run and still has a small urinary bladder uses during a period of time for actively moving around, such as daytime; however, of course, the usage form is not limited to this. Incidentally, the first particulate water absorbent and the second particulate water absorbent are not limited to be of the same type, under the premise that the overall absorption amount of the second particulate water absorbent and the water absorption time determined according to a vortex method are defined as described above. Furthermore, the mechanisms and the like described in the present specification are not intended to limit the technical scope of the claims of the present application.

In the following description, embodiments of the present invention will be described with reference to the attached drawings. Incidentally, identical reference signs will be assigned to identical elements in the description of the drawings, and any overlapping explanations will not be repeated. Furthermore, the dimensional ratios of the drawings are exaggerated for the convenience of explanation and may be different from the actual ratios.

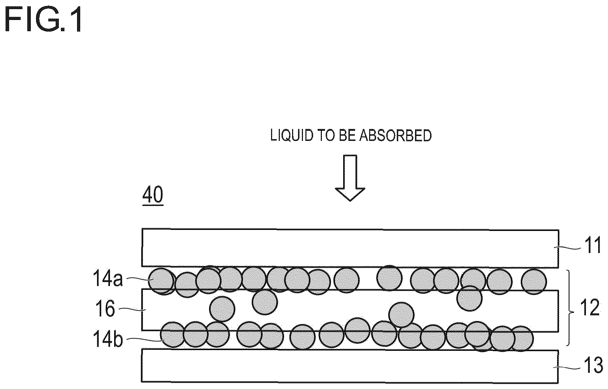

Fig. 1 is a schematic diagram illustrating a cross-section of a water-absorbing sheet 40 according to an embodiment of the present invention. A first base material 11 is positioned on the side where a liquid to be absorbed is introduced. That is, at least the first base material is disposed on the discharge side of the liquid (for example, the skin side in a paper diaper). A water-absorbing layer 12 is disposed between the first base material 11 and a second base material 13. Incidentally, in Fig. 1, the second base material 13 is positioned only on the side opposite to the side where the liquid to be absorbed is introduced, with respect to the water-absorbing layer 12 interposed therebetween; however, for example, the area of the second base material 13 may be designed to be larger than that of the first base material 11 so that the second base material 13 may be folded up so as to wrap the first base material 11. By doing so, fall-off of a first particulate water absorbent 14a and a second particulate water absorbent 14b can be suppressed. In the present specification, the first particulate water absorbent and the second particulate water absorbent may be collectively referred to simply as particulate water absorbents. The water-absorbing layer 12 has the particulate water absorbents 14 and may have an intermediate sheet 16, if necessary. In the water-absorbing sheet 40 of Fig. 1, the first particulate water absorbent 14a localized on the side of a surface of the first base material 11, the surface being arranged to face the second base material 13, and the second particulate water absorbent 14b localized on the side of a surface of the second base material 13, the surface being arranged to face the first base material 11, exist so as to interpose an intermediate sheet 16. Within this intermediate sheet 16, for example, the water absorbents 14 fixed by an adhesive may be detached, and thereby water absorbents 14 captured in the intermediate sheet 16 may exist. In the form of Fig. 1, the water-absorbing layer 12 has a particulate water absorbent 14a fixed to the first base material 11 and a particulate water absorbent 14b fixed to the second base material. Portions of the particulate water absorbents 14a and 14b may have been detached from the respective sheets. Therefore, the water-absorbing "layer" does not refer only to a continuous body such as a sheet but may have any form so long as it exists with a certain thickness between the first base material 11 and the second base material 13. Regarding the method of fixing to the respective base materials, an adhesive is used. A method for producing a water-absorbing sheet using an adhesive will be described in detail in [3.].

The water-absorbing sheet according to an embodiment of the present invention can be made thinner than the absorbent bodies used in the conventional type absorbent articles. In a case in which the water-absorbing sheet is used for a disposable diaper, the thickness is, for example, at 40%RH to 50%RH, preferably 15 mm or less, more preferably 10 mm or less, even more preferably 7 mm or less, particularly preferably 5 mm or less, and most preferably 4 mm or less. On the other hand, in view of the strength of the water-absorbing sheet and the diameters of the particulate water absorbents, the lower limit of the thickness is 0.2 mm or more, preferably 0.3 mm or more, and more preferably 0.5 mm or more. The thickness of the water-absorbing sheet used in the Examples of the present application was 3 mm to 5 mm under the above-described conditions.

Incidentally, the thickness of the water-absorbing sheet is measured using a Dial Thickness Gauge large-sized type (thickness measuring machine) (manufactured by Ozaki Manufacturing Co., Ltd., product No. : J-B, gauge head: anvils upper and lower $\phi$ 50 mm) . Regarding the measurement sites, an absorbent body was divided into three equal parts in the longitudinal direction, and the respective center parts (a point positioned at an intersection point obtained by drawing diagonal lines from the corners of the absorbent body) were set as the measurement sites. For example, in the case of a water-absorbing sheet measuring 36 cm in the longitudinal direction and 10 cm in the width direction, the measurement positions are such that, with respect to the length of 36 cm in the longitudinal direction, three points such as a point 6 cm away from the left end in the longitudinal direction and 5 cm away from both ends in the width direction (designated as left) ; a point 18 cm away from the left end in the longitudinal direction and 5 cm away from both ends in the width direction (designated as center) ; and a point 30 cm away from the left end in the longitudinal direction and 5 cm away from both ends in the width direction (designated as right), correspond to the measurement sites. The measurement score is obtained by making measurement two times at each site, and the measured value of thickness is designated as the average value of six points in total. Regarding a specific procedure, a water-absorbing sheet is stuck flatly on a plate having a uniform thickness so that no crease or distortion is produced at the measurement sites of the water-absorbing sheet, and the plate is mounted on a lower gauge head of a thickness measuring machine . Next, an upper gauge head of the thickness measuring machine is approached to a position 2 to 3 mm high from the water-absorbing sheet, subsequently the handle is slowly released from the hands, and the thickness combining the water-absorbing sheet and the plate is measured. The thickness of the water-absorbing sheet is determined by formula: $T1 = T2 - T0$ (T0: thickness of plate (mm), T1: thickness of water-absorbing sheet (mm), T2: thickness of water-absorbing sheet and plate (mm)).

In order to further impart liquid passability, diffusibility, flexibility, and the like to the water-absorbing sheet, the surface of the water-absorbing sheet may be appropriately subjected to embossing processing. The region that is subjected to embossing processing may be the entire face of the water-absorbing sheet surface or may be a portion thereof. By providing a continuously embossing-processed region in the longitudinal direction of the water-absorbing sheet, a liquid can be caused to diffuse easily in the longitudinal direction. Furthermore, the particulate water absorbents may be scattered on the entire surface of the water-absorbing sheet, or particulate water absorbent-absent regions may be provided in some parts. In the case of providing particulate water absorbent-absent regions, it is preferable to provide the absent regions in a channel form (striped shape) in the longitudinal direction of the water-absorbing sheet. As such, when embossing-processed regions and/or particulate water absorbent-absent regions are provided consecutively in the longitudinal direction, those regions accomplish the role as passages for allowing large quantities of liquid to flow through (liquid conveyance passages). The embossing-processed regions and/or the particulate water absorbent-absent regions may be provided in a linear shape, may be provided in a curved shape, or may be provided in a wavy shape.

In the following description, various members constituting the water-absorbing sheet will be described in detail.

[2-1. First base material, second base material, and intermediate sheet]

The first base material is a water-permeable sheet positioned on the side where a liquid to be absorbed is introduced. Incidentally, the liquid to be absorbed is not limited to water and may be urine, blood, sweat, feces, wastewater, moisture, ice, a mixture of water and an organic solvent and/or an inorganic solvent, rain water, underground water, or the like. The liquid to be absorbed is not particularly limited so long as it includes water. Preferred examples include urine, menstrual blood, sweat, and other body fluids.

The material for the first base material, the second base material, and the intermediate sheet used for the water-absorbing sheet according to an embodiment of the present invention is preferably a nonwoven fabric. The raw material of the nonwoven fabric is not particularly limited; however, from the viewpoints of liquid penetrability, flexibility, and the strength of the water-absorbing sheet, polyolefin fibers (polyethylene (PE), polypropylene (PP), and the like), polyester fibers (polyethylene terephthalate (PET), polytrimethylene terephthalate (PTT), polyethylene naphthalate (PEN), and the like), polyamide fibers (nylon and the like), rayon fibers, pulp (cellulose) fibers, and the like are preferred. Furthermore, nonwoven fabrics of other synthetic fibers, and nonwoven fabrics produced by mixing synthetic fibers with cotton, silk, hemp, pulp (cellulose) fibers and the like are also similarly preferred. The nonwoven fabric described above may be a nonwoven fabric including only one kind of the above-mentioned fibers or a nonwoven fabric combining two or more kinds of fibers. It is preferable that the nonwoven fabrics used for the first base material and the second base material are produced by anair-laidmethod. Furthermore, pulp (cellulose) fibers are preferred.

As mentioned above, the nonwoven fabric used for the water-absorbing sheet according to an embodiment of the present invention is preferably a hydrophilic nonwoven fabric in order to increase the water permeability; however, the nonwoven fabric or the fibers as the material of the nonwoven fabric may be hydrophilized using a hydrophilizing agent (surfactant or the like).

Regarding examples of the hydrophilizing agent include anionic surfactants (aliphatic sulfonic acid salts, higher alcohol sulfuric acid ester salts, and the like), cationic surfactants (quaternary ammonium salts and the like), nonionic surfactants (polyethylene glycol fatty acid esters, polyglycerin fatty acid esters, sorbitan fatty acid esters, and the like), silicone-based surfactants (polyoxyalkylene-modified silicone, and the like), stain release agents including polyester-based, polyamide-based, acrylic, and urethane-based resins, and the like are used. Regarding examples of the hydrophilizing agent include anionic surfactants (aliphatic sulfonic acid salts, higher alcohol sulfuric acid ester salts, and the like), cationic surfactants (quaternary ammonium salts and the like), nonionic surfactants (polyethylene glycol fatty acid esters, polyglycerin fatty acid esters, sorbitan fatty acid esters, and the like), silicone-based surfactants (polyoxyalkylene-modified silicone, and the like), stain release agents including polyester-based, polyamide-based, acrylic, and urethane-based resins, and the like are used.

The first base material used for the water-absorbing sheet according to an embodiment of the present invention is a water-permeable sheet because it is located on the side where the liquid to be absorbed is introduced; however, it is preferable that the second base material and the intermediate sheet are also water-permeable sheets having water permeability, and the two may be of the same type or may be of different types. With regard to the water-permeability to the water-permeable sheet, the water permeability coefficient (JIS A1218:2009) is preferably $1\times10^{-5}$ cm/sec or higher. This water permeability coefficient is more preferably $1 \times 10^{-4}$ cm/sec or higher, even more preferably $1 \times 10^{-3}$ cm/sec or higher, still more preferably $1 \times 10^{-2}$ cm/sec or higher, and even more preferably $1 \times 10^{-1}$ cm/sec or higher. The water-permeable sheet used for the water-absorbing sheet according to an embodiment of the present invention is preferably a hydrophilic nonwoven fabric. By being a hydrophilic nonwoven fabric, the intended effects of the present invention can be efficiently provided.

The porosities of the first base material, the second base material, and the intermediate sheet (for example, nonwoven fabric) can be measured by the following formula. The basis weight A ($g/m^2$) used for a base material (or a sheet), the thickness B (mm) of a base material (or a sheet), the densityC ($g/cm^3$) of the rawmaterial (for example, polyolefin) used for a base material (or a sheet) Porosity (%) of base material (or sheet) = 100 - {(A/10000) / (B/10)} / C*100

It is more desirable that the thicknesses of the first base material and the second base material are thinner to the extent of having strength as a water-absorbing sheet, and for each sheet of the base material, the thicknesses are each independently appropriately selected in the ranges of 0.01 to 2 mm, 0.02 to 1 mm, 0.03 to 0.6 mm, and 0.05 to 0.5 mm. The basis weight is preferably 5 to 300 $g/m^2$, more preferably 8 to 200 $g/m^2$, even more preferably 10 to 100 $g/m^2$, and still more preferably 11 to 50 $g/m^2$, per sheet of the base material.

[2-2. Water-absorbing layer]

The water-absorbing layer in the water-absorbing sheet according to an embodiment of the present invention includes particulate water absorbents and optionally an intermediate sheet.

(Particulate water absorbents)

The water-absorbing layer in the water-absorbing sheet according to an embodiment of the present invention includes a first particulate water absorbent and a second particulate water absorbent. Incidentally, unless particularly stated otherwise, in a case in which the first particulate water absorbent and/or the second particulate water absorbent are mixtures of a plurality of kinds of particulate water absorbents, the following description is an explanation of the physical properties of the mixtures.

"Overall absorption amount"

According to the present invention, the overall absorption amount as represented by the following formula:

$$[\text{Mathematical Formula 3}]$$
$$\text{CRC g/g} + 0.44 \times \text{AAP2.1kPa g/g}$$

of the second particulate water absorbent is 48 g/g or more. When the overall absorption amount is less than 48 g/g, the problems of the present invention cannot be solved. As such, by adjusting the value of the overall absorption amount that defines the relationship between CRC and AAP2.1kPa to 48 g/g or more, excellent results are obtained in the evaluation of the specific return amount.

The overall absorption amount of the second particulate water absorbent in the water-absorbing sheet according to an embodiment of the present invention needs to be 48 g/g or more; however, the overall absorption amount is preferably 49 g/g or more, more preferably 50.0 g/g or more, even more preferably 51.0 g/g or more, and still more preferably 52.0 g/g or more. When the overall absorption amount of the second particulate water absorbent becomes larger, since the action as a tank of the second particulate water absorbent can be effectively exhibited, excellent results are obtained in the evaluation of the specific return amount. Meanwhile, the upper limit of the overall absorption amount of the second particulate water absorbent is not particularly limited; however, practically, the upper limit is about 60 g/g or less, 58 g/g or less, or 56 g/g or less.

The lower limit of the overall absorption amount of the first particulate water absorbent in the water-absorbing sheet according to an embodiment of the present invention is not particularly limited; however, the lower limit is preferably 30 g/g or more, more preferably 35 g/g or more, and even more preferably 40 g/g or more. Incidentally, incidentally, the upper limit of the overall absorption amount of the first particulate water absorbent is not particularly limited; however, practically, the upper limit is about 60 g/g or less, 58 g/g or less, or 56 g/g or less.

Incidentally, CRC in the overall absorption amount can be controlled by means of the amount of an internal crosslinking agent, and the like. Furthermore, AAP can be controlled by means of the crosslinking density of the surface crosslinked layer, and the like. These can be controlled by known technologies.

Incidentally, according to embodiments of the present invention, there is also provided a water-absorbing sheet having a first base material, a second base material, and a water-absorbing layer positioned between the first base material and the second base material, wherein the first base material is a water-permeable sheet positioned on the side where a liquid to be absorbed is introduced, the water-absorbing layer has a first particulate water absorbent localized on the side of a surface of the first base material, the surface being arranged to face the second base material, and a second particulate water absorbent localized on the side of a surface of the second base material, the surface being arranged to face the first base material, and the water absorption time of the second particulate water absorbent as determined according to a vortex method is 35 seconds or more. The description including, for example, the physical properties related to the second particulate water absorbent (for example, CRC and AAP2. 1kpa) as described in the present specification can also be applied to the second particulate water absorbent included in the water-absorbing sheet according to this embodiment. According to the present embodiment, a second particulate water absorbent for which CRC is high to a certain extent and AAP is high to a certain extent, can be used. According to the present embodiment, it is preferable that the lower limit of CRC is 34 g/g or more, and the lower limit of AAP2.1kpa is 32 g/g or more. According to the present embodiment, it is preferable that the lower limit of CRC is 35 g/g or more, and the lower limit of AAP2.1kpa is 30 g/g or more. According to the present embodiment, it is preferable that the lower limit of CRC is 36 g/g or more, and the lower limit of AAP2.1kpa is 28 g/g or more. According to the present embodiment, it is preferable that the lower limit of CRC is 37.2 g/g or more, and the lower limit of AAP2.1kpa is 24.5 g/g or more. According to the present embodiment, it is preferable that the lower limit of CRC is 38 g/g or more, and the lower limit of AAP2.1kpa is 23 g/g or more. According to the present embodiment, it is preferable that the lower limit of CRC is 39 g/g or more, and the lower limit of AAP2.1kpa is 21 g/g or more. Furthermore, the description including, for example, the physical properties related to the first particulate water absorbent (for example, the water absorption time determined according to a vortex method, and GPR) as described in the present specification can also be applied to the first particulate water absorbent included in the water-absorbing sheet according to this embodiment.

"CRC" (ERT441.2-02)

The term "CRC" is an acronym for "Centrifuge Retention Capacity" and means a water absorption ratio with no load (may also be referred to as "water absorption ratio") of a particulate water absorbent. Specifically, CRC refers to a water absorption ratio (unit: g/g) obtained by introducing 0.2 g of a particulate water absorbent into a nonwoven fabric bag, subsequently immersing the bag in a large excess of a 0.9 mass% aqueous solution of sodium chloride for 30 minutes so as to cause the particulate water absorbent to freely swell, and then dehydrating the particulate water absorbent in a centrifuge (250 G).

The CRC of the second particulate water absorbent in the water-absorbing sheet according to the present invention is 30 to 50 g/g, preferably 36 to 45 g/g, and more preferably 38 to 42 g/g. Thereby, excellent results are obtained in the evaluation of the specific return amount.

The CRC of the first particulate water absorbent in the water-absorbing sheet according to an embodiment of the present invention is preferably 30 to 50 g/g, more preferably 31 to 48 g/g, and even more preferably 32 to 45 g/g. By being such an embodiment, excellent results are obtained in the evaluation of the specific return amount.

"AAP" (ERT442.2-02)

"AAP" is an acronym for "Absorption Against Pressure" and means the water absorption ratio under pressure of a particulate water absorbent. Specifically, AAP refers to the water absorption ratio (unit: g/g) obtained after swelling 0.9 g of a particulate water absorbent in a large excess of a 0.9 mass% aqueous solution of sodium chloride for one hour under a load of 2.06 kPa (21 g/cm$^2$, 0.3 psi). Furthermore, in ERT442.2-02, it is described as Absorption Under Pressure (AUP); however, the terms substantially have the same contents.

The AAP2.1kPa of the second particulate water absorbent in the water-absorbing sheet according to the present invention is 15 to 35 g/g, preferably 23 to 33 g/g, and more preferably 24 to 32 g/g. Thereby, excellent results are obtained in the evaluation of the specific return amount.

The AAP2.1kPa of the first particulate water absorbent in the water-absorbing sheet according to an embodiment of the present invention is preferably 18 to 40 g/g, more preferably 23 to 33 g/g, and even more preferably 24 to 32 g/g. By being such an embodiment, excellent results are obtained in the evaluation of the specific return amount. Therefore, in the water-absorbing sheet according to an embodiment of the present invention, the CRC of the second particulate water absorbent is 30 to 50 g/g, and the AAP2.1kPa is 15 to 35 g/g.

"Vortex"

The term "Vortex" according to the present invention is the water absorption time determined according to the "Testing method for water absorption rate of super absorbent polymers" described in JIS K7224-1996. Regarding a specific measurement method, 0.02 parts by weight of Edible Blue No. 1, which is a food additive, is added to 1, 000 parts by weight of a 0.90 wt% aqueous solution of sodium chloride that has been prepared in advance, and the mixture is adjusted to a liquid temperature of 30°C. 50 ml of the 0.90 wt% aqueous solution of sodium chloride that has been colored blue is weighed in a 100-ml beaker, and in the middle of stirring the aqueous solution at 600 rpm with a cylindrical stirring bar having a length of 40 mm and a width of 8 mm, 2.00 g of a particulate water absorbent is charged thereinto. The water absorption time (seconds) is measured. Regarding the termination point, according to the criteria described in the "Manual for testing method for water absorption rate of super absorbent polymers" described in JIS K7224-year 1996, the time taken by the water absorbent to absorb physiological saline until the test liquid covers the stirrer tip is measured as the water absorption time (seconds).

With regard to the water-absorbing sheet according to an embodiment of the present invention, the water absorption time of the second particulate water absorbent as determined according to the vortex method is 35 seconds or more, preferably 40 seconds or more, and more preferably 43 seconds or more. Here, when the water absorption time of the second particulate water absorbent as determined according to the vortex method is less than 35 seconds, the intended object of the present invention cannot be solved. As such, by regulating the water absorption time of the second particulate water absorbent as determined according to the vortex method to be meaningfully long, the diffusibility in the planar direction of the second particulate water absorbent can be increased, and the reaction volume possessed by the second particulate water absorbent can be effectively utilized. Thus, excellent results are obtained in the evaluation of the specific return amount.

With regard to the water-absorbing sheet according to an embodiment of the present invention, the water absorption time of the first particulate water absorbent as determined according to the vortex method is preferably 25 seconds or more, more preferably 35 seconds or more, and even more preferably 48 seconds or more. As such, by regulating the water absorption time of the first particulate water absorbent as determined according to the vortex method to be meaningfully long, the diffusibility in the planar direction can be increased, and the reaction volume possessed by the first particulate water absorbent can be effectively utilized. Thus, excellent results are obtained in the evaluation of the specific return amount. With regard to the water-absorbing sheet according to an embodiment of the present invention, the upper limit of the water absorption time of the first particulate water absorbent as determined according to the vortex method is not particularly limited; however, from the viewpoint of preventing liquid leakage, for example, the upper limit may be 100 seconds or less, 80 seconds or less, or 60 seconds or less. Incidentally, with regard to the water-absorbing sheet of the present invention, as long as the water absorption time of the second particulate water absorbent as determined according to the vortex method is 35 seconds or more, the water absorption time being faster than the water absorption time of the first particulate water absorbent as determined according to the vortex method is not obstructed. The water absorption time determined according to the vortex method can be controlled by the specific surface area and the like of the water-absorbing resin particles. When the specific surface area of the water-absorbing resin particles becomes large, the water absorption time tends to be shortened.

"GPR"

Gel permeation rate (Gel PermeationPermeability Rate: GPR)

According to the present specification, the "liquid passability" of a particulate water absorbent refers to the flowability of a liquid that passes between swollen gel particles under a load. As an index of this, the gel permeation rate (GPR) is used. The gel permeation rate (GPR) of the particulate water absorbents (first particulate water absorbent and second particulate water absorbent) included in the water-absorbing sheet according to an embodiment of the present invention is carried out by the following procedure by referring to the saline flow conductivity (SFC) test described in US Patent No. 5849405 and by changing the measurement conditions.

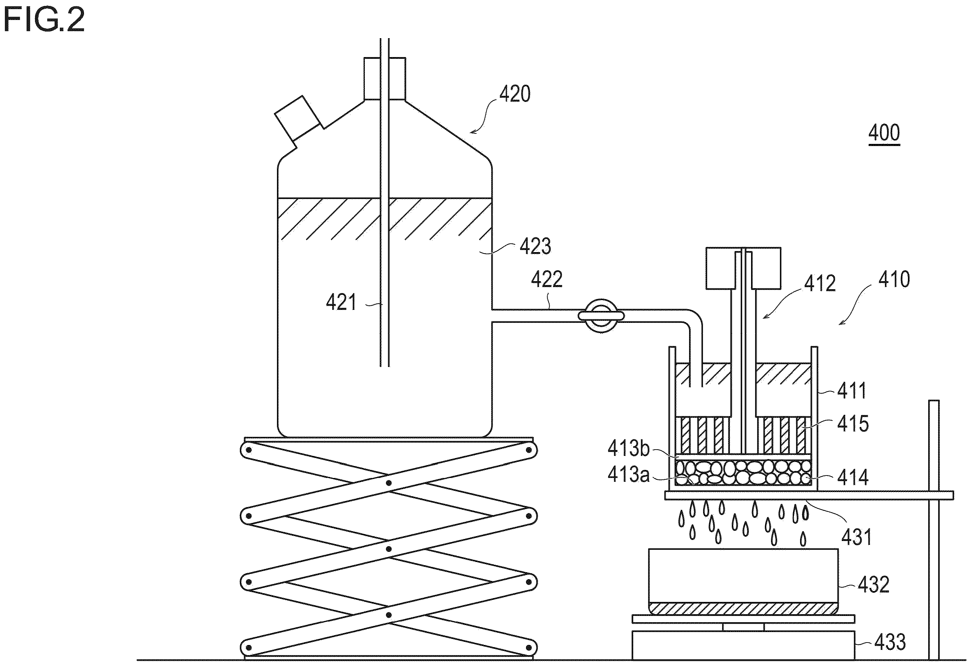

As an apparatus for measurement, an apparatus 400 illustrated in Fig. 2 is used. The apparatus 400 is broadly composed of a container 410 and a tank 420. In the container 410, a cell 411 (inner diameter 6 cm) is provided, and inside the cell 411, a swollen gel 414 (product obtained by causing a particulate water absorbent to absorb water) can be stored, while a liquid 423 can be introduced. Furthermore, by fitting a piston 412 into the cell 411, pressure can be exerted to the swollen gel 414. At the bottom face of the cell 411 and the bottom face of the piston 412, wire gauzes 413a and 413b (wire gauzes made of No. 400 stainless steel, sieve opening 38 $\mu$m) are stretched so that the swollen gel 414 (and a particulate water absorbent) cannot pass therethrough. Here, as the liquid 423, a 0.00 mass% to 0.90 mass% aqueous solution (0.9 mass% in the present application) of sodium chloride is used. The tank 420 has the liquid 423 accumulated inside. The liquid 423 is introduced into the cell 411 through an L-shaped tube with a cock 422. Furthermore, a glass tube 421 is inserted into the tank 420, and the interior of the glass tube 421 is filled with air. Thereby, the lower end of the glass tube 421 and the liquid surface inside the cell 411 can be made even. That is, so long as the liquid surface of the liquid 423 inside the tank 420 remains upper to the lower end of the glass tube 421, it is possible to maintain the liquid surface inside the cell 411 at a constant level. In the measurement of this time, the difference in elevation between the lower liquid surface of the liquid 423 (that is, lower end of the glass tube 421) inside the tank 420 and the bottom face of the swollen gel 414 was adjusted to 4 cm. That is, according to the apparatus 400, the liquid 423 at a constant positive hydrostatic pressure can be introduced into the cell 411. Since the piston 412 has a hole 415 formed therein, the liquid 423 flows through the hole 415, also further flows through the swollen gel 414 layer, and flows out to the outside of the cell 411. The container 410 is mounted on a stainless steel wire gauze 431 that does not interrupt the passage of the liquid 423. Therefore, the liquid 423 that has flowed out from the cell 411 is finally collected in a capturing container 432. Then, the amount of the liquid 423 collected in the capturing container 432 can be weighed using a Roberval balance 433.

A specific method for measuring the gel permeation rate (GPR) is as follows. Meanwhile, the following operation is carried out at room temperature (20°C to 25°C).

(1) A particulate water absorbent (0.900 g) is uniformly introduced into a cell 411.

(2) The particulate water absorbent is caused to absorb a liquid (0.00 mass% to 0.90 mass% (0.9 mass% in the present application) aqueous solution of sodium chloride) with no load for 60 minutes, and a swollen gel 414 is obtained.

(3) A piston is placed on the swollen gel 414, and the system is brought into a pressurized state at 0.3 psi (2.07 kPa) .

(4) While the hydrostatic pressure is maintained at a constant value of 3, 923 dyne/cm$^2$, a liquid 423 is introduced into the cell 411 and is caused to pass through the swollen gel 414 layer.

(5) The amount of the liquid 423 that passes through the swollen gel 414 layer is recorded for 3 minutes at an interval of 5 seconds. That is, the flow rate of the liquid 423 that passes through the swollen gel 414 layer is measured. For the measurement, a Roberval balance 433 and a computer (not illustrated in the diagram) are used.

(6) The flow rates after one minute to after three minutes from the initiation of the flow-through of the liquid 423 are averaged, and the gel permeation rate (GPR) [g/min] is calculated.

With regard to the water-absorbing sheet according to an embodiment of the present invention, the GPR of the first particulate water absorbent is preferably 5 g/min or more, more preferably 35 g/min or more, even more preferably 55 g/min or more, still more preferably 75 g/min or more, even more preferably 95 g/min or more, and still more preferably 118 g/min or more. By being such an embodiment, the liquid to be absorbed may be easily sent to the second particulate water absorbent after being introduced on the first base material side, and the second particulate water absorbent can be effectively utilized. Thus, excellent results are obtained in the evaluation of the specific return amount. With regard to the water-absorbing sheet according to an embodiment of the present invention, the upper limit of the GPR of the first particulate water absorbent is not particularly limited; however, from the viewpoint of preventing liquid leakage, the upper limit is 500 g/min or less, 400 g/min or less, or 300 g/min or less.

With regard to the water-absorbing sheet according to an embodiment of the present invention, the GPR of the second particulate water absorbent is preferably 1 g/min or more, more preferably 3 g/min or more, and even more preferably 5 g/min or more. By having such a lower limit, gel blocking is suppressed, diffusibility is increased, and a liquid can be easily absorbed by the absorbent body. With regard to the water-absorbing sheet according to an embodiment of the present invention, the upper limit of the GPR of the first particulate water absorbent is not particularly limited; however, from the viewpoint of preventing liquid leakage, the upper limit is 500 g/min or less, 400 g/min or less, or 300 g/min or less.

From the above, with regard to the water-absorbing sheet according to an embodiment of the present invention, the overall absorption amount of the second particulate water absorbent is 50.0 g/g or more, the GPR of the first particulate water absorbent is 5 g/min or more, and the content weight of the second particulate water absorbent with respect to the content weight of the first particulate water absorbent is 0.4 or less. By being such an embodiment, the intended effects of the present invention can be notably provided. Furthermore, with regard to the water-absorbing sheet according to an embodiment of the present invention, the water absorption time of the second particulate water absorbent as determined according to the vortex method is 43 seconds or more, and the overall absorption amount of the second particulate water absorbent is 52.0 g/g or more. By being such an embodiment, the intended effects of the present invention can be notably provided.

"Degradable soluble content"

The upper limits of the degradable soluble content of the first particulate water absorbent and the degradable soluble content of the second particulate water absorbent in the water-absorbing sheet according to an embodiment of the present invention are not particularly limited; however, from the viewpoint of liquid leakage during long-term use, the upper limits are each independently preferably less than 50%, more preferably 25% or less, and even more preferably 15% or less. The lower limits are not particularly limited; however, from the viewpoint of liquid leakage during long-term use, for example, the lower limits are about 1% or more, 3% or more, and 5% or more. Incidentally, a method for measuring the degradable soluble content is as follows.

L-ascorbic acid is added to physiological saline that has been prepared in advance so as to obtain a concentration of 0.05% by mass, and a degradation test liquid is produced. Specifically, 0.50 g of L-ascorbic acid was dissolved in 999.5 g of physiological saline, and thereby a degradation test liquid was prepared. 25 ml of the degradation test liquid is added into a glass beaker container having a capacity of 250 ml, 1.0 g of a particulate water absorbent is added thereto, and thereby a swollen gel is formed. The container is tightly sealed by covering up with a plastic wrap, and the swollen gel is left to stand for 16 hours in an atmosphere at 37°C (manufactured by Kusumoto Chemicals, Ltd., ETAC trade name, HISPEC series, used HT320, set to 37°C, set to a wind velocity variator scale of 30). After 16 hours, 175 ml of physiological saline and a cylindrical stirring bar having a length of 30 mm and a width of 8 mm are charged therein, the swollen gel is degraded and then stirred at 500 rpm for 10 minutes, and extraction from hydrated gel is performed.

20.0 g of a filtrate that is obtained by filtering this extract using a filter paper (Advantec Toyo Kaisha, Ltd., product name: (JIS P 3801, No. 2), thickness 0.26 mm, retained particle size 5 pm) is weighed, 30 g of physiological saline is further added thereto, and the mixture is used as a measurement solution.

Regarding the measurement method, physiological saline is subjected to titration up to pH 10 using a 0.1 N aqueous solution of NaOH, subsequently the physiological saline is titrated to pH 2.7 using a 0.1 N aqueous solution of HCl, and blank titers ([bNaOH] ml, [bHCl] ml) are obtained. A similar titration operation is also carried out for the measurement solution, and thereby titers ([NaOH]ml, [HCl]ml) are determined. For example, in the case of a particulate water absorbent including known amounts of acrylic acid and sodium salt thereof, a soluble content in the particulate water absorbent can be calculated by the following calculation formula, based on the average molecular weight of the monomer and the titers obtained by the above-described operation:

Degradable soluble content (mass%) = $0.1 \times$ (average molecular weight) $\times 200 \times 100 \times$ ([HCl] - [bHCl]/1000/1.0/20.0.

In the case of an unknown amount, the average molecular weight of the monomer is calculated using a neutralization ratio determined by titration.

"Surface tension"

Surface tension is the work (free energy) required to increase the surface area of a solid or a liquid, expressed in per unit area. The surface tension as used in the present application refers to the surface tension of an aqueous solution when a particulate water absorbent is dispersed in a 0.90 mass% aqueous solution of sodium chloride. Incidentally, the surface tension of a water absorbent is measured by the following procedure. That is, 50 ml of physiological saline that has been adjusted to 20°C is introduced into a sufficiently washed 100-ml beaker, and first, the surface tension of the physiological saline is measured using a surface tensiometer (K11 automatic surface tensiometer manufactured by Kruss GmbH). Next, into the beaker including the physiological saline after surface tension measurement, which has been adjusted to 20°C, a sufficiently washed stirring bar made of a fluororesin, 25 mm in length, and 0.5 g of a particulate water absorbent are charged, and the mixture is stirred for 4 minutes under the conditions of 500 rpm. After 4 minutes, after stirring is stopped and the hydrated particulate water absorbent settles, the surface tension of the supernatant is measured by performing a similar operation again. Incidentally, in the present invention, a plate method of using a platinum plate is employed, and the plate is sufficiently washed with deionized water before each measurement and is used after being heated with a gas burner and washed. With regard to the water-absorbing sheet according to an embodiment of the present invention, the surface tension of at least one of the first particulate water absorbent and the second particulate water absorbent is preferably, in the following order, 65 mN/m or more, 66 mN/m or more, 67 mN/m or more, 69 mN/m or more, 70 mN/m or more, or 71 mN/m or more, and most preferably 72 mN/m or more. In the application of a particulate water absorbent to a water-absorbing sheet, the influence of surface tension is more easily exhibited than conventional paper diapers, and as the surface tension satisfies the above-described conditions, the return amount to the paper diaper can be reduced.

With regard to the water-absorbing sheet according to an embodiment of the present invention, the upper limit of the surface tension of at least one of the first particulate water absorbent and the second particulate water absorbent is not particularly limited; however, the upper limit is 73 mN/m or less.

"Diffusivity"

Diffusivity represents liquid diffusibility of a water-absorbing sheet, and as the diffusivity is higher, the liquid diffusibility is higher. A method for measuring the diffusivity is based on the method described in the Examples of the present application.

The diffusivity of the water-absorbing sheet according to an embodiment of the present invention is preferably 38% or higher, more preferably 39% or higher, even more preferably 40% or higher, still more preferably 41% or higher, even more preferably 42% or higher, and still more preferably 43% or higher. By being such an embodiment, the intended effects of the present invention can be notably provided. The upper limit of the diffusivity of the water-absorbing sheet according to an embodiment of the present invention is not particularly limited; however, from the viewpoint of liquid leakage, the upper limit is practically 80% or lower.

"Particle shape"

The particulate water absorbent according to an embodiment of the present invention is preferably such that the particle shape is an irregular crushed shape. Therefore, at least one of the shapes of the first particulate water absorbent and the second particulate water absorbent is an irregular crushed shape. Here, the irregular crushed shape is a crushed-shaped particle whose shape is not regular. Compared to spherical particles obtained by reverse phase suspension polymerization or gas phase polymerization, the irregular crushed shape allows easy fixation to the base material. The particulate water absorbent according to an embodiment of the present invention is preferably a pulverized product in aqueous solution polymerization. On the other hand, in a case in which the particulate water absorbent is not subjected to a pulverization process, representatively, spherical particles obtainable by reverse phase suspension polymerization, liquid droplet polymerization, by which monomers to be polymerized are sprayed and polymerized, or the like, or a granulated product of spherical particles do not have an irregular crushed shape . According to embodiments of the present invention, when the shape of the particulate water absorbent is an irregular crushed shape, shape retention of the water-absorbing sheet is easily achieved as compared to particles whose average degree of circularity is high (for example, spherical particles) . According to embodiments of the present invention, the average degree of circularity of the particulate water absorbent is preferably 0.70 or less, more preferably 0.60 or less, and even more preferably 0.55 or less.

The method for calculating the average degree of circularity is as follows. One hundred or more particles of a particulate water absorbent were randomly selected, various particles of the particulate water absorbent were imaged with an electron microscope (VE-9800 manufactured by KEYENCE CORPORATION) (magnification ratio 50 times), images of the particulate water absorbent were obtained, and the circumference and the area were calculated for each particle using an affiliated image analysis software. The degree of circularity of each particle is determined by the following formula:

$$[\text{Mathematical Formula 4}]$$
$$\text{Degree of circularity} = 4 \times \pi \times \text{area}/(\text{circumference})^2$$

and the average value of the values thus obtained is calculated as the average degree of circularity.

"Particle size"

The particle size of the particulate water absorbent (or a particulate water-absorbing resin, water-absorbing resin particles) according to an embodiment of the present invention is a weight average particle size obtained by the method for measuring "PSD" as defined in ERT420.2-02, and the particle size may be 150 to 600 pm.

With regard to the water-absorbing sheet according to an embodiment of the present invention, the lower limit of the content of the particulate water absorbents per unit volume of the water-absorbing sheet is, in a preferable order, 50 mg/cm$^3$ or more, 51 mg/cm$^3$ or more, 52 mg/cm$^3$ or more, 53 mg/cm$^3$ or more, 54 mg/cm$^3$ or more, 55 mg/cm$^3$ or more, 57 mg/cm$^3$ or more, 59 mg/cm$^3$ or more, or 60 mg/cm$^3$ or more. With regard to the water-absorbing sheet according to an embodiment of the present invention, the upper limit of the content of the particulate water absorbents per unit volume of the water-absorbing sheet is also not particularly limited; however, the upper limit is practically 600 mg/cm$^3$ or less, preferably 500 mg/cm$^3$ or less, more preferably 400 mg/cm$^3$ or less, even more preferably 300 mg/cm$^3$ or less, and still more preferably 150 mg/cm$^3$ or less. By adjusting the content of the particulate water absorbents to the ranges described above, the water-absorbing sheet efficiently exhibits the effects of the present invention. The contents of particulate water absorbents per unit volume of the water-absorbing sheet of the water-absorbing sheets used in Examples of the present application were 50 mg/cm$^3$ or more.

The method for producing a particulate water absorbent is not particularly limited as long as it is a method for producing a water absorbent having desired physical properties, and for example, a particulate water absorbent can be appropriately produced by referring to the publications described in the Examples and the like.

Furthermore, in the water-absorbing sheet according to an embodiment of the present invention, the content weight of the first particulate water absorbent with respect to the content weight of the second particulate water absorbent is preferably 1.0 or less, more preferably 0.5 or less, and even more preferably 0.4 or less. By making the content of the second particulate water absorbent to be equal to, and preferably larger than, the content of the first particulate water absorbent, the second particulate water absorbent that is considered to have a so-called tank-like operating function effectively functions. Thus, excellent results are obtained in the evaluation of the specific return amount.

[3. Method for producing water-absorbing sheet]

The method for producing a water-absorbing sheet according to an embodiment of the present invention includes at least one of: (1) a step of scattering a first particulate water absorbent on a first base material, (2) a step of scattering a second particulate water absorbent on a second base material, and (3) a step of scattering a first particulate water absorbent and/or a second particulate water absorbent on an intermediate sheet. As more specific examples of the production method, the following production methods of (a) to (c) may be mentioned.

(a) A first particulate water absorbent (and preferably an adhesive) is uniformly scattered on a first base material. A second base material and a second particulate water absorbent are also subjected to a similar operation. The first base material and the second base material are superposed such that the surfaces on which the particulate water absorbents have been scattered are paired, and the base materials are pressure-bonded. Pressure-bonding is preferably heated pressure-bonding at near the melting temperature of the adhesive.

(b) A first particulate water absorbent (and preferably an adhesive) is scattered on a first base material, the first base material is passed through a heating furnace, and thereby the first particulate water absorbent is fixed to the extent that the first particulate water absorbent is not scattered and lost. A second base material and a second particulate water absorbent are also subjected to a similar operation. The first base material and the second base material are superposed such that the surfaces on which the particulate water absorbents have been scattered are paired, and the base materials are heated and pressure-bonded.

(c) An adhesive is melted and applied on a first base material, subsequently a particulate water absorbent is uniformly scattered thereon, and thereby a layer is formed. A second base material and a second particulate water absorbent are also subjected to a similar operation. The first base material and the second base material are superposed such that the surfaces on which the particulate water absorbents have been scattered are paired, and the base materials are pressure-bonded using a roll press or the like.

Among these methods, from the viewpoints of the convenience of the production method and the level of production efficiency, methods (a) and (c) are preferred. Incidentally, it is also possible to produce a water-absorbing sheet by using the methods (a) to (c) in combination.

Furthermore, as examples of a method for producing a water-absorbing sheet including an intermediate sheet, the following production methods of (d) to (g) are available.

(d) A first particulate water absorbent (and preferably an adhesive) is uniformly scattered on a first base material. An intermediate sheet is superposed thereon, and the assembly is pressure-bonded. Furthermore, on a surface of the intermediate sheet on the side that does not face the first particulate water absorbent, a second particulate water absorbent (and preferably an adhesive) is uniformly scattered. An intermediate sheet is superposed thereon, and (preferably, under heated conditions in which a hot melt is melted, or in a state in which a hot melt is melting) the assembly is pressure-bonded.

(e) A second particulate water absorbent is uniformly scattered on an intermediate sheet. Furthermore, an adhesive is scattered on a second base material. Then, the surface of the intermediate sheet, on which the second particulate water absorbent has been scattered, and the surface of the second base material, on which the adhesive has been scattered, are pressure-bonded. Next, in the intermediate sheet after pressure-bonding, on the surface on the opposite side of the surface on which the second particulate water absorbent has been scattered, a first particulate water absorbent is uniformly scattered. Furthermore, an adhesive is scattered on the first base material. Then, the surface of the intermediate sheet, on which the first particulate water absorbent has been scattered, and the surface of the first base material, on which the adhesive has been scattered, are pressure-bonded. It is preferable that the above-described pressure-bonding is carried out under heated conditions in which a hot melt is melted, or in a state in which a hot melt is melting.

(f) An adhesive is scattered on a second base material. Next, a second particulate water absorbent is uniformly scattered thereon. Next, an intermediate sheet is placed thereon, and the assembly is pressure-bonded. Next, an adhesive is scattered on a surface of the intermediate sheet on the side that does not face the second particulate water absorbent. Next, a first particulate water absorbent is uniformly scattered thereon. Next, a first base material is placed thereon, and the assembly is pressure-bonded. It is preferable that the above-described pressure-bonding is carried out under heated conditions in which a hot melt is melted, or in a state in which a hot melt is melting.

(g) An adhesive is scattered on a second base material. Next, a second particulate water absorbent is uniformly scattered thereon. Next, an intermediate sheet is placed thereon, and the assembly is pressure-bonded. Next, on the surface of the intermediate sheet on the side that does not face the second particulate water absorbent, a first particulate water absorbent is uniformly scattered. Furthermore, an adhesive is scattered on a first base material. Then, the surface of the intermediate sheet, on which the first particulate water absorbent has been scattered, and the surface of the first base material, on which the adhesive has been scattered, are pressure-bonded. It is preferable that the above-described pressure-bonding is carried out under heated conditions in which a hot melt is melted, or in a state in which a hot melt is melting.

As a step other than those described above, for the purpose of improving the feel of the water-absorbing sheet to the touch and enhancing the liquid absorption performance, the water-absorbing sheet may be subjected to embossing processing. The embossing processing may be carried out simultaneously when the first base material and the second base material are pressure-bonded or may be carried out after sheet production.

In the method for producing a water-absorbing sheet according to an embodiment of the present invention, additives (a deodorant, fibers, an antibacterial agent, a gel stabilizer, and the like) may be appropriately incorporated. The amount of incorporation of the additives is preferably 0% to 50% by mass, and more preferably 0% to 10% by mass, with respect to the mass of the particulate water absorbents. In the above-described production method, particulate water absorbents that have been mixed with additives in advance may be used, or additives may be added in the middle of the production process.

The dimension of the water-absorbing sheet to be produced can be appropriately designed. Usually, the transverse width is 3 to 10 m, and the length is several dozen meters (m) to several thousand meters (m) (for a continuous sheet or a roll form) . The water-absorbing sheet thus produced is used after being cut out according to the purpose (size of the absorbent body to be used).

In addition to the examples described above, the method for producing a water-absorbing sheet is disclosed in the following patent literatures: WO 2012/174026 A, WO 2013/078109 A, WO 2015/041784 A, WO 2011/117187 A, WO 2012/001117 A, WO 2012/024445 A, WO 2010/004894 A, WO 2010/004895 A, WO 2010/076857 A, WO 2010/082373 A, WO 2010/113754 A, WO 2010/143635 A, WO 2011/043256 A, WO 2011/086841 A, WO 2011/086842 A, WO 2011/086843 A, WO 2011/086844 A, WO 2011/117997 A, WO 2011/118409 A, WO 2011/136087 A, WO 2012/043546 A, WO 2013/099634 A, WO 2013/099635A, JP2010-115406A, JP2002-345883A, JPH6-315501 A, JP H6-190003 A, JP H6-190002 A, JP H6-190001 A, JP H2-252558 A, JP H2-252560 A, and JP H2-252561 A. The methods for producing a water-absorbing sheet disclosed in these literatures are also appropriately referred to.

With regard to the water-absorbing sheet according to an embodiment of the present invention, as a method of fixing base materials together or fixing a base material with a particulate water absorbent, (i) pressure-bonding may be used, (ii) various binders dissolved or dispersed in water, a water-soluble polymer, or a solvent may be used, or (iii) base materials may be heat-sealed at the melting point of the material of the base materials themselves. Fixing is achieved using an adhesive.

The adhesive to be used may be a solution type; however, from the viewpoint of the effort to remove the solvent, the problem of remaining solvent, and the problem of productivity, a hot melt adhesive that exhibits high productivity and does not have a problem of residual solvent is preferred. In the present invention, the hot melt adhesive may be incorporated in advance at the surface of the base material or the particulate water absorbent, or separately, a hot melt adhesive may be used during the production process for a water-absorbing sheet. The form or melting point of the hot melt adhesive can be appropriately selected, and the hot melt adhesive may be in a particulate form, may be in a fibrous form, may be in a net form, may be in a film form, or may be in a liquid form melted by heating. The melting temperature or the softening point of the hot melt adhesive is preferably 50°C to 200°C, or 60°C to 180°C. In the case of using a particulate adhesive, a particulate adhesive in which the particle size is about 0.01 to 2 times, 0.02 to 1 time, or 0.05 to 0.5 times, the average particle size of the particulate water absorbents, is used.

Regarding the method of using a hot melt adhesive in the production of the water-absorbing sheet according to an embodiment of the present invention, the following example may be mentioned. A water-absorbing sheet can be produced by uniformly scattering a mixture of a particulate water absorbent and a hot melt adhesive on a base material (for example, a nonwoven fabric), laminating another sheet of base material thereon, and heating and pressure-bonding the laminate at near the melting temperature of the hot melt adhesive.

The hot melt adhesive to be used for the present invention can be appropriately selected; however, preferably, one or more kinds selected from an ethylene-vinyl acetate copolymer adhesive, a styrene-based elastomer adhesive, a polyolefin-based adhesive, a polyester-based adhesive, and the like can be appropriately used.

Specific examples include, as polyolefin-based adhesives, polyethylene, polypropylene, and atactic polypropylene; as styrene-based elastomer adhesives, a styrene-isoprene block copolymer (SIS), a styrene-butadiene block copolymer (SBS), a styrene-isobutylene block copolymer (SIBS), a styrene-ethylene-butylene-styrene block copolymer (SEES), a styrene-butadiene rubber (SBR), and the like; copolymerized polyolefins, and the like; as polyester-based adhesives, polyethylene terephthalate (PET), polybutylene terephthalate (PBT), copolymerized polyesters, and the like; as ethylene-vinyl acetate copolymer adhesives, an ethylene-vinyl acetate copolymer (EVA) adhesive; an ethylene-ethyl acrylate copolymer (EEA), an ethylene-butyl acrylate copolymer (EBA), and the like.

With regard to the water-absorbing sheet according to an embodiment of the present invention and/or a method for producing the same, it is preferable that the water-absorbing sheet includes an adhesive, and the adhesive is preferably a hot melt adhesive. The amount of use (content) of the adhesive (for example, a hot melt adhesive) is preferably more than 0 and 3.0 times, and more preferably 0.05 to 2.0 times, the total mass of the first particulate water absorbent and the second particulate water absorbent. When the content of the adhesive (particularly, a hot melt melt adhesive) is too large, not only it is disadvantageous in view of the cost and in view of the mass of the water-absorbing sheet (increase in the mass of a paper diaper), but also there is a possibility that the particulate water absorbent may be subjected to the regulation of swelling and lower the water absorption power of the water-absorbing sheet.

[4. Absorbent article]

The absorbent article according to an embodiment of the present invention has a structure in which the water-absorbing sheet described in [2] is interposed between a liquid-permeable sheet and a liquid-impermeable sheet. Therefore, according to the present invention, there is provided an absorbent article having the water-absorbing sheet interposed between a liquid-permeable sheet and a liquid-impermeable sheet, the liquid-permeable sheet being positioned on the first base material side, and the liquid-impermeable sheet being positioned on the second base material side. Specific examples of the absorbent article include a disposable diaper, an incontinence pad, a sanitary napkin, a pet sheet, a drip sheet for food, a water sealant for electric power cables, and the like. By having such a configuration, excellent results are obtained in the evaluation of the specific return amount.

Regarding the liquid-permeable sheet and the liquid-impermeable sheet, those known in the technical field of absorbent articles can be used without particular limitations. Furthermore, the absorbent article can be produced according to a known method.

EXAMPLES

The present invention will be described in more detail using the following Examples and Comparative Examples. However, the technical scope of the present invention is not intended to be limited to the following Examples only. Furthermore, in the following Examples, unless particularly stated otherwise, operations were carried out under the conditions of room temperature (25°C)/relative humidity of 40% to 50%RH.

<Production Example>

With reference to the Production Examples, Examples, and Comparative Examples described in the following patent literatures, particulate water absorbents (1) to (7) of polyacrylic acid (salt)-based resins were obtained by appropriately adjusting CRC by means of the amount of an internal crosslinking agent. The physical properties of the particulate water absorbents thus obtained are presented in Table 1.

WO 2014/034897 A
WO 2017/170605 A
WO 2016/204302 A
WO 2014/054656 A
WO 2015/152299 A
WO 2018/062539 A
WO 2012/043821 A.

[Production Example of acrylic acid]

Commercially available acrylic acid (acrylic acid dimer 2, 000 ppm, acetic acid 500 ppm, propionic acid 500 ppm, and p-methoxyphenol 200 ppm) was supplied to the column bottom of a high-boiling point impurities separating column having fifty weirless perforated plates and was distilled at a reflux ratio of 1. After removal of maleic acid, a dimer including acrylic acid (acrylic acid dimer), and the like, crystallization is further carried out, and thereby acrylic acid (acrylic acid dimer 20 ppm, acetic acid 50 ppm, propionic acid 50 ppm, furfural 1 ppm or less, and protoanemonin 1 ppm or less) was obtained. After performing further distillation, 50 ppm of p-methoxyphenol was added thereto.

[Method for producing aqueous solution of sodium acrylate]

1,390 g of the acrylic acid was neutralized at 20°C to 40°C using 48% caustic soda according to Example 9 of US Patent No. 5210298, and thereby a 100% neutralized aqueous solution of sodium acrylate at a concentration of 37% was obtained.

<Particulate water absorbent 1>

In 5,500 g (monomer concentration 33.0% by mass) of an aqueous solution of sodium acrylate having a neutralization ratio of 75 mol%, which was obtained by mixing the acrylic acid obtained in the above-described Production Example of acrylic acid, an aqueous solution of sodium acrylate obtained by the above-described method for producing an aqueous solution of sodium acrylate using the acrylic acid, and deionized water, 4.37 g of polyethylene glycol diacrylate (average number of added moles of ethylene oxide 9) was dissolved, and a reaction liquid was obtained. Next, the reaction liquid was supplied to a reactor formed by attaching a lid to a jacketed stainless steel double-arm type kneader having an internal volume of 10 L and having two sigma-type blades, the system was purged with nitrogen gas while maintaining the reaction liquid at 30°C, and dissolved oxygen in the reaction liquid was removed. Subsequently, while the reaction liquid was stirred, 26.65 g of a 10 mass% aqueous solution of sodium persulfate and 32.79 g of a 1 mass% aqueous solution of L-ascorbic acid were added thereto, and after approximately 1 minute, polymerization was initiated. Forty minutes after the initiation of polymerization, 181.5 g of a water-absorbing resin fine powder having a size of 150 $\mu$m or less was added thereto, the gel was cracked for 10 minutes by rotating the blades of the kneader at a high speed (130 rpm), and then a hydrated gel-like polymer was taken out. The hydrated gel-like polymer thus obtained was finely divided into particles that measured about 1 to 2 mm. This finely divided hydrated gel-like polymer was spread on a 50-mesh (mesh size 300 pm) wire gauze and was subjected to hot air drying for 65 minutes at 175°C. Next, the dried product was pulverized using a roll mill, further classified with a wire gauze having a sieve opening of 600 pm, and compounded. Thereby, water-absorbing resin (1-1) having an irregular crushed shape with an average particle size of 350 $\mu$m was obtained.

Into 100 parts by mass of the water-absorbing resin (1-1) thus obtained, 3.83 parts by mass of an aqueous solution of a surface crosslinking agent including 0.03 parts by mass of ethylene glycol diglycidyl ether, 0.3 parts by mass of 1,4-butanediol, 0.5 parts by mass of propylene glycol, and 3.0 parts by mass of water was sprayed and mixed. The mixture was subjected to a heating treatment for 40 minutes at a heat medium temperature of 195°C using a paddle type mixing and heat-treating machine, and surface-crosslinked water-absorbing resin (1-2) was obtained. 1.0 part by mass of water was sprayed and mixed into 100 parts by mass of the surface-crosslinked water-absorbing resin (1-2) thus obtained, the mixture was cured in a sealed container for one hour at 60°C and then was passed through a sieve having a sieve opening of 710 $\mu$m. Thus, water-absorbing resin (1-3) was obtained. 0.3 parts by mass of Aerosil 200 (hydrophilic amorphous silica, manufactured by Nippon Aerosil Co., Ltd.) was added to the water-absorbing resin (1-3) and mixed, and thereby the water-absorbing resin thus obtained was produced as particulate water absorbent (1).

<Particulate water absorbent 2>

In 5,500 g (monomer concentration 38.0% by mass) of an aqueous solution of sodium acrylate having a neutralization ratio of 75 mol%, which was obtained by mixing the acrylic acid obtained in the above-described Production Example of acrylic acid, an aqueous solution of sodium acrylate obtained by the above-described method for producing an aqueous solution of sodium acrylate using the acrylic acid, and deionized water, 5.03 g of polyethylene glycol diacrylate (average number of added moles of ethylene oxide 9) was dissolved, and a reaction liquid was obtained. Next, the reaction liquid was supplied to a reactor formed by attaching a lid to a jacketed stainless steel double-arm type kneader having an internal volume of 10 L and having two sigma-type blades, the system was purged with nitrogen gas while maintaining the reaction liquid at 30°C, and dissolved oxygen in the reaction liquid was removed. Subsequently, while the reaction liquid was stirred, 30.68 g of a 10 mass% aqueous solution of sodium persulfate and 37. 7 6 g of a 1 mass% aqueous solution of L-ascorbic acid were added thereto, and after approximately 1 minute, polymerization was initiated. Forty minutes after the initiation of polymerization, a hydrated gel-like polymer was taken out. The hydrated gel-like polymer thus obtained was finely divided into particles that measured about 2 to 4 mm. This finely divided hydrated gel-like polymer was spread on a 50-mesh (mesh size 300 pm) wire gauze and was subjected to hot air drying for 65 minutes at 175°C. Next, the dried product was pulverized using a roll mill, further classified with a wire gauze having a sieve opening of 600 pm, and compounded. Thereby, water-absorbing resin (2-1) having an irregular crushed shape with an average particle size of 380 $\mu$m was obtained.

Into 100 parts by mass of the water-absorbing resin (2-1) thus obtained, 3.83 parts by mass of an aqueous solution of a surface crosslinking agent including 0.03 parts by mass of ethylene glycol diglycidyl ether, 0.3 parts by mass of 1,4-butanediol, 0.5 parts by mass of propylene glycol, and 3.0 parts by mass of water was sprayed and mixed. The mixture was subjected to a heating treatment for 40 minutes at a heat medium temperature of 210°C using a paddle type mixing and heat-treating machine, and surface-crosslinked water-absorbing resin (2-2) was obtained. 1.0 part by mass of water was spray and mixed into 100 parts by mass of the surface-crosslinked water-absorbing resin (2-2) thus obtained, the mixture was cured in a sealed container for one hour at 60°C and then was passed through a sieve having a sieve opening of 710 $\mu$m. Thus, water-absorbing resin (2-3) was obtained. 0.3 parts by mass of Aerosil 200 (hydrophilic amorphous silica, manufactured by Nippon Aerosil Co., Ltd.) was added to the water-absorbing resin (2-3) and mixed, and thereby the water-absorbing resin thus obtained was produced as particulate water absorbent (2).

<Particulate water absorbent 3>

In 5,500 g (monomer concentration 38.0% by mass) of an aqueous solution of sodium acrylate having a neutralization ratio of 75 mol%, which was obtained by mixing the acrylic acid obtained in the above-described Production Example of acrylic acid, an aqueous solution of sodium acrylate obtained by the above-described method for producing an aqueous solution of sodium acrylate using the acrylic acid, and deionized water, 3.77 g of polyethylene glycol diacrylate (average number of added moles of ethylene oxide 9) was dissolved, and a reaction liquid was obtained. Next, the reaction liquid was supplied to a reactor formed by attaching a lid to a jacketed stainless steel double-arm type kneader having an internal volume of 10 L and having two sigma-type blades, the system was purged with nitrogen gas while maintaining the reaction liquid at 30°C, and dissolved oxygen in the reaction liquid was removed. Subsequently, while the reaction liquid was stirred, 30.68 g of a 10 mass% aqueous solution of sodium persulfate and 37. 7 6 g of a 1 mass% aqueous solution of L-ascorbic acid were added thereto, and after approximately 1 minute, polymerization was initiated. Forty minutes after the initiation of polymerization, a hydrated gel-like polymer was taken out. The hydrated gel-like polymer thus obtained was finely divided into particles that measured about 2 to 4 mm. This finely divided hydrated gel-like polymer was spread on a 50-mesh (mesh size 300 pm) wire gauze and was subjected to hot air drying for 65 minutes at 175°C. Next, the dried product was pulverized using a roll mill, further classified with a wire gauze having a sieve opening of 600 pm, and compounded. Thereby, water-absorbing resin (3-1) having an irregular crushed shape with an average particle size of 350 $\mu$m was obtained.

Into 100 parts by mass of the water-absorbing resin (3-1) thus obtained, 3.83 parts by mass of an aqueous solution of a surface crosslinking agent including 0.03 parts by mass of ethylene glycol diglycidyl ether, 0.3 parts by mass of 1,4-butanediol, 0.5 parts by mass of propylene glycol, and 3.0 parts by mass of water was sprayed and mixed. The mixture was subjected to a heating treatment for 40 minutes at a heat medium temperature of 195°C using a paddle type mixing and heat-treating machine, and surface-crosslinked water-absorbing resin (3-2) was obtained. 1.0 part by mass of water was sprayed and mixed into 100 parts by mass of the surface-crosslinked water-absorbing resin (3-2) thus obtained, the mixture was cured in a sealed container for one hour at 60°C and then was passed through a sieve having a sieve opening of 710 $\mu$m. Thus, water-absorbing resin (3-3) was obtained. 0.3 parts by mass of Aerosil 200 (hydrophilic amorphous silica, manufactured by Nippon Aerosil Co., Ltd.) was added to the water-absorbing resin (3-3) and mixed, and thereby the water-absorbing resin thus obtained was produced as particulate water absorbent (3).

<Particulate water absorbent 4>

In 5,500 g (monomer concentration 39.0% by mass) of an aqueous solution of sodium acrylate having a neutralization ratio of 75 mol%, which was obtained by mixing the acrylic acid obtained in the above-described Production Example of acrylic acid, an aqueous solution of sodium acrylate obtained by the above-described method for producing an aqueous solution of sodium acrylate using the acrylic acid, and deionized water, 4.80 g of polyethylene glycol diacrylate (average number of added moles of ethylene oxide 9) was dissolved, and a reaction liquid was obtained. Next, the reaction liquid was supplied to a reactor formed by attaching a lid to a jacketed stainless steel double-arm type kneader having an internal volume of 10 L and having two sigma-type blades, the system was purged with nitrogen gas while maintaining the reaction liquid at 30°C, and dissolved oxygen in the reaction liquid was removed. Subsequently, while the reaction liquid was stirred, 31.65 g of a 10 mass% aqueous solution of sodium persulfate and 38.95 g of a 1 mass% aqueous solution of L-ascorbic acid were added thereto, and after approximately 1 minute, polymerization was initiated. Forty minutes after the initiation of polymerization, a hydrated gel-like polymer was taken out. The hydrated gel-like polymer thus obtained was finely divided into particles that measured about 2 to 4 mm. This finely divided hydrated gel-like polymer was spread on a 50-mesh (mesh size 300 pm) wire gauze and was subjected to hot air drying for 65 minutes at 175°C. Next, the dried product was pulverized using a roll mill, further classified with a wire gauze having a sieve opening of 600 pm, and compounded. Thereby, water-absorbing resin (4-1) having an irregular crushed shape with an average particle size of 380 $\mu$m was obtained.

Into 100 parts by mass of the water-absorbing resin (4-1) thus obtained, 3.83 parts by mass of an aqueous solution of a surface crosslinking agent including 0.03 parts by mass of ethylene glycol diglycidyl ether, 0.3 parts by mass of 1,4-butanediol, 0.5 parts by mass of propylene glycol, and 3.0 parts by mass of water was sprayed and mixed. The mixture was subjected to a heating treatment for 40 minutes at a heat medium temperature of 210°C using a paddle type mixing and heat-treating machine, and surface-crosslinked water-absorbing resin (4-2) was obtained. 1.0 part by mass of water was sprayed and mixed into 100 parts by mass of the surface-crosslinked water-absorbing resin (4-2) thus obtained, the mixture was cured in a sealed container for one hour at 60°C and then was passed through a sieve having a sieve opening of 710 μm. Thus, water-absorbing resin (4-3) was obtained. 0.3 parts by mass of Aerosil 200 (hydrophilic amorphous silica, manufactured by Nippon Aerosil Co., Ltd.) was added to the water-absorbing resin (4-3) and mixed, and thereby the water-absorbing resin thus obtained was produced as particulate water absorbent (4).

<Particulate water absorbent 5>

In 5,500 g (monomer concentration 35.5% by mass) of an aqueous solution of sodium acrylate having a neutralization ratio of 75 mol%, which was obtained by mixing the acrylic acid obtained in the above-described Production Example of acrylic acid, an aqueous solution of sodium acrylate obtained by the above-described method for producing an aqueous solution of sodium acrylate using the acrylic acid, and deionized water, 4.00 g of polyethylene glycol diacrylate (average number of added moles of ethylene oxide 9) was dissolved, and a reaction liquid was obtained. Next, the reaction liquid was supplied to a reactor formed by attaching a lid to a jacketed stainless steel double-arm type kneader having an internal volume of 10 L and having two sigma-type blades, the system was purged with nitrogen gas while maintaining the reaction liquid at 30°C, and dissolved oxygen in the reaction liquid was removed. Subsequently, while the reaction liquid was stirred, 28.66 g of a 10 mass% aqueous solution of sodium persulfate and 35.28 g of a 1 mass% aqueous solution of L-ascorbic acid were added thereto, and after approximately 1 minute, polymerization was initiated. Forty minutes after the initiation of polymerization, a hydrated gel-like polymer was taken out. The hydrated gel-like polymer thus obtained was finely divided into particles that measured about 2 to 4 mm. This finely divided hydrated gel-like polymer was spread on a 50-mesh (mesh size 300 pm) wire gauze and was subjected to hot air drying for 65 minutes at 175°C. Next, the dried product was pulverized using a roll mill, further classified with a wire gauze having a sieve opening of 600 pm, and compounded. Thereby, water-absorbing resin (5-1) having an irregular crushed shape with an average particle size of 350 μm was obtained.

Into 100 parts by mass of the water-absorbing resin (5-1) thus obtained, 4.03 parts by mass of an aqueous solution of a surface crosslinking agent including 0.03 parts by mass of ethylene glycol diglycidyl ether, 1.0 part by mass of propylene glycol, and 3.0 parts by mass of water was sprayed and mixed. The mixture was subjected to a heating treatment for 40 minutes at a heat medium temperature of 100°C using a paddle type mixing and heat-treating machine, and surface-crosslinked water-absorbing resin (5-2) was obtained. 3.0 part by mass of water was sprayed and mixed into 100 parts by mass of the surface-crosslinked water-absorbing resin (5-2) thus obtained, the mixture was cured in a sealed container for one hour at 60°C and then was passed through a sieve having a sieve opening of 710 μm. Thus, water-absorbing resin (5-3) was obtained. 0.3 parts by mass of Aerosil 90G (hydrophilic amorphous silica, manufactured by Nippon Aerosil Co., Ltd.) was added to the water-absorbing resin (5-3) and mixed, and thereby the water-absorbing resin thus obtained was produced as particulate water absorbent (5).

<Particulate water absorbent 6>

In 5,500 g (monomer concentration 35.5% by mass) of an aqueous solution of sodium acrylate having a neutralization ratio of 75 mol%, which was obtained by mixing the acrylic acid obtained in the above-described Production Example of acrylic acid, an aqueous solution of sodium acrylate obtained by the above-described method for producing an aqueous solution of sodium acrylate using the acrylic acid, and deionized water, 5.29 g of polyethylene glycol diacrylate (average number of added moles of ethylene oxide 9) was dissolved, and a reaction liquid was obtained. Next, the reaction liquid was supplied to a reactor formed by attaching a lid to a jacketed stainless steel double-arm type kneader having an internal volume of 10 L and having two sigma-type blades, the system was purged with nitrogen gas while maintaining the reaction liquid at 30°C, and dissolved oxygen in the reaction liquid was removed. Subsequently, while the reaction liquid was stirred, 28.66 g of a 10 mass% aqueous solution of sodium persulfate and 35.28 g of a 1 mass% aqueous solution of L-ascorbic acid were added thereto, and after approximately 1 minute, polymerization was initiated. Forty minutes after the initiation of polymerization, a hydrated gel-like polymer was taken out. The hydrated gel-like polymer thus obtained was finely divided into particles that measured about 2 to 4 mm. This finely divided hydrated gel-like polymer was spread on a 50-mesh (mesh size 300 pm) wire gauze and was subjected to hot air drying for 65 minutes at 175°C. Next, the dried product was pulverized using a roll mill, further classified with a wire gauze having a sieve opening of 600 pm, and compounded. Thereby, water-absorbing resin (6-1) having an irregular crushed shape with an average particle size of 350 μm was obtained.

Into 100 parts by mass of the water-absorbing resin (6-1) thus obtained, 4.03 parts by mass of an aqueous solution of a surface crosslinking agent including 0.03 parts by mass of ethylene glycol diglycidyl ether, 1.0 part by mass of propylene glycol, and 3.0 parts by mass of water was sprayed and mixed. The mixture was subjected to a heating treatment for 40 minutes at a heat medium temperature of 100°C using a paddle type mixing and heat-treating machine, and surface-crosslinked water-absorbing resin (6-2) was obtained. 3.0 part by mass of water was sprayed and mixed into 100 parts by mass of the surface-crosslinked water-absorbing resin (6-2) thus obtained, the mixture was cured in a sealed container for one hour at 60°C and then was passed through a sieve having a sieve opening of 710 $\mu$m. Thus, water-absorbing resin (6-3) was obtained. 0.3 parts by mass of Aerosil 90G (hydrophilic amorphous silica, manufactured by Nippon Aerosil Co., Ltd.) was added to the water-absorbing resin (6-3) and mixed, and thereby the water-absorbing resin thus obtained was produced as particulate water absorbent (6).

<Particulate water absorbent 7>

In 5,500 g (monomer concentration 33.0% by mass) of an aqueous solution of sodium acrylate having a neutralization ratio of 75 mol%, which was obtained by mixing the acrylic acid obtained in the above-described Production Example of acrylic acid, an aqueous solution of sodium acrylate obtained by the above-described method for producing an aqueous solution of sodium acrylate using the acrylic acid, and deionized water, 6.01 g of polyethylene glycol diacrylate (average number of added moles of ethylene oxide 9) was dissolved, and a reaction liquid was obtained. Next, the reaction liquid was supplied to a reactor formed by attaching a lid to a jacketed stainless steel double-arm type kneader having an internal volume of 10 L and having two sigma-type blades, the system was purged with nitrogen gas while maintaining the reaction liquid at 30°C, and dissolved oxygen in the reaction liquid was removed. Subsequently, while the reaction liquid was stirred, 26.65 g of a 10 mass% aqueous solution of sodium persulfate and 32.79 g of a 1 mass% aqueous solution of L-ascorbic acid were added thereto, and after approximately 1 minute, polymerization was initiated. Forty minutes after the initiation of polymerization, 181.5 g of a water-absorbing resin fine powder having a size of 150 $\mu$m or less was added thereto, the gel was cracked for 10 minutes by rotating the blades of the kneader at a high speed (130 rpm), and then a hydrated gel-like polymer was taken out. The hydrated gel-like polymer thus obtained was finely divided into particles that measured about 1 to 2 mm. This finely divided hydrated gel-like polymer was spread on a 50-mesh (mesh size 300 pm) wire gauze and was subjected to hot air drying for 65 minutes at 175°C. Next, the dried product was pulverized using a roll mill, further classified with a wire gauze having a sieve opening of 600 pm, and compounded. Thereby, water-absorbing resin (7-1) having an irregular crushed shape with an average particle size of 350 $\mu$m was obtained.

Into 100 parts by mass of the water-absorbing resin (7-1) thus obtained, 3.83 parts by mass of an aqueous solution of a surface crosslinking agent including 0.03 parts by mass of ethylene glycol diglycidyl ether, 0.3 parts by mass of 1,4-butanediol, 0.5 parts by mass of propylene glycol, and 3.0 parts by mass of water was sprayed and mixed. The mixture was subjected to a heating treatment for 40 minutes at a heat medium temperature of 210°C using a paddle type mixing and heat-treating machine, and surface-crosslinked water-absorbing resin (7-2) was obtained. 1.0 part by mass of water was sprayed and mixed into 100 parts by mass of the surface-crosslinked water-absorbing resin (7-2) thus obtained, the mixture was cured in a sealed container for one hour at 60°C and then was passed through a sieve having a sieve opening of 710 $\mu$m. Thus, water-absorbing resin (7-3) was obtained. 0.3 parts by mass of Aerosil 200 (hydrophilic amorphous silica, manufactured by Nippon Aerosil Co., Ltd.) was added to the water-absorbing resin (7-3) and mixed, and thereby the water-absorbing resin thus obtained was produced as particulate water absorbent (7).

<Particulate water absorbent 8>

In 5,500 g (monomer concentration 38.0% by mass) of an aqueous solution of sodium acrylate having a neutralization ratio of 75 mol%, which was obtained by mixing the acrylic acid obtained in the above-described Production Example of acrylic acid, an aqueous solution of sodium acrylate obtained by the above-described method for producing an aqueous solution of sodium acrylate using the acrylic acid, and deionized water, 4.40 g of polyethylene glycol diacrylate (average number of added moles of ethylene oxide 9) was dissolved, and a reaction liquid was obtained. Next, the reaction liquid was supplied to a reactor formed by attaching a lid to a jacketed stainless steel double-arm type kneader having an internal volume of 10 L and having two sigma-type blades, the system was purged with nitrogen gas while maintaining the reaction liquid at 30°C, and dissolved oxygen in the reaction liquid was removed. Subsequently, while the reaction liquid was stirred, 30.68 g of a 10 mass% aqueous solution of sodium persulfate and 37. 7 6 g of a 1 mass% aqueous solution of L-ascorbic acid were added thereto, and after approximately 1 minute, polymerization was initiated. Forty minutes after the initiation of polymerization, a hydrated gel-like polymer was taken out. The hydrated gel-like polymer thus obtained was finely divided into particles that measured about 2 to 4 mm. This finely divided hydrated gel-like polymer was spread on a 50-mesh (mesh size 300 pm) wire gauze and was subjected to hot air drying for 65 minutes at 175°C. Next, the dried product was pulverized using a roll mill, further classified with a wire gauze having a sieve opening of 600 pm, and compounded. Thereby, water-absorbing resin (8-1) having an irregular crushed shape with an average particle size of 350 $\mu$m was obtained.

Into 100 parts by mass of the water-absorbing resin (8-1) thus obtained, 3.83 parts by mass of an aqueous solution of a surface crosslinking agent including 0.03 parts by mass of ethylene glycol diglycidyl ether, 0.3 parts by mass of 1,4-butanediol, 0.5 parts by mass of propylene glycol, and 3.0 parts by mass of water was sprayed and mixed. The mixture was subjected to a heating treatment for 40 minutes at a heat medium temperature of 195°C using a paddle type mixing and heat-treating machine, and surface-crosslinked water-absorbing resin (8-2) was obtained. 1.0 part by mass of water was sprayed and mixed into 100 parts by mass of the surface-crosslinked water-absorbing resin (8-2) thus obtained, the mixture was cured in a sealed container for one hour at 60°C and then was passed through a sieve having a sieve opening of 710 $\mu$m. Thus, water-absorbing resin (8-3) was obtained. 0.3 parts by mass of Aerosil 200 (hydrophilic amorphous silica, manufactured by Nippon Aerosil Co., Ltd.) was added to the water-absorbing resin (8-3) and mixed, and thereby the water-absorbing resin thus obtained was produced as particulate water absorbent (8).

<Particulate water absorbent 9>

In 5,500 g (monomer concentration 39.0% by mass) of an aqueous solution of sodium acrylate having a neutralization ratio of 75 mol%, which was obtained by mixing the acrylic acid obtained in the above-described Production Example of acrylic acid, an aqueous solution of sodium acrylate obtained by the above-described method for producing an aqueous solution of sodium acrylate using the acrylic acid, and deionized water, 3.87 g of polyethylene glycol diacrylate (average number of added moles of ethylene oxide 9) was dissolved, and a reaction liquid was obtained. Next, the reaction liquid was supplied to a reactor formed by attaching a lid to a jacketed stainless steel double-arm type kneader having an internal volume of 10 L and having two sigma-type blades, the system was purged with nitrogen gas while maintaining the reaction liquid at 30°C, and dissolved oxygen in the reaction liquid was removed. Subsequently, while the reaction liquid was stirred, 31.49 g of a 10 mass% aqueous solution of sodium persulfate and 38.7 6 g of a 1 mass% aqueous solution of L-ascorbic acid were added thereto, and after approximately 1 minute, polymerization was initiated. Forty minutes after the initiation of polymerization, a hydrated gel-like polymer was taken out. The hydrated gel-like polymer thus obtained was finely divided into particles that measured about 2 to 4 mm. This finely divided hydrated gel-like polymer was spread on a 50-mesh (mesh size 300 pm) wire gauze and was subjected to hot air drying for 65 minutes at 175°C. Next, the dried product was pulverized using a roll mill, further classified with a wire gauze having a sieve opening of 600 pm, and compounded. Thereby, water-absorbing resin (9-1) having an irregular crushed shape with an average particle size of 350 $\mu$m was obtained.

Into 100 parts by mass of the water-absorbing resin (9-1) thus obtained, 3.83 parts by mass of an aqueous solution of a surface crosslinking agent including 0.03 parts by mass of ethylene glycol diglycidyl ether, 0.3 parts by mass of 1,4-butanediol, 0.5 parts by mass of propylene glycol, and 3.0 parts by mass of water was sprayed and mixed. The mixture was subjected to a heating treatment for 40 minutes at a heat medium temperature of 195°C using a paddle type mixing and heat-treating machine, and surface-crosslinked water-absorbing resin (9-2) was obtained. 1.0 part by mass of water was sprayed and mixed into 100 parts by mass of the surface-crosslinked water-absorbing resin (9-2) thus obtained, the mixture was cured in a sealed container for one hour at 60°C and then was passed through a sieve having a sieve opening of 710 $\mu$m. Thus, water-absorbing resin (9-3) was obtained. 0.3 parts by mass of Aerosil 200 (hydrophilic amorphous silica, manufactured by Nippon Aerosil Co., Ltd.) was added to the water-absorbing resin (9-3) and mixed, and thereby the water-absorbing resin thus obtained was produced as particulate water absorbent (9).

[Extraction of water-absorbing resin from commercially available disposable diaper 1]

A water-absorbing resin was taken out from commercially available disposable diapers (MOONY AIRFIT (L size, Lot No. 201512163072), manufactured by Unicharm Corp., purchased in May 2 016) . At the time of extraction, only the water-absorbing resin was taken out so as not to be mixed with cotton-like pulp and the like. The extracted water-absorbing resin had a particle shape obtained by granulating spherical particles. This water-absorbing resin was designated as particulate water absorbent (T1). The physical properties of the particulate water absorbent (T1) are presented in Table 1.

[Extraction of water-absorbing resin from commercially available disposable diaper 2]

A water-absorbing resin was taken out from a commercially available disposable diaper (HUGGIES (4 Maxi size, Lot No. EXP30/04/19), manufactured by Kimberly-Clark Corporation, purchased in June 2016 from Turkey) . At the time of extraction, only the water-absorbing resin was taken out so as not to be mixed with cotton-like pulp and the like. The extracted water-absorbing resin had a crushed particle shape. This water-absorbing resin was designated as particulate water absorbent (T2) . The physical properties of the particulate water absorbent (T2) are presented in Table 1.

[EXAMPLES]

[Example 1]

On a surface of a nonwoven fabric (2) made of pulp fibers as a main component (product produced by an air-laid method, corresponding to a second base material, basis weight: 42 g/m$^2$) having a thickness of 0.70 mm under no load, which had been cut into a size of 10 cm in length and 40 cm in width, 0.3 to 0.5 g of an adhesive including styrene-butadiene rubber (spray adhesive 77, manufactured by 3M Japan, Ltd.) was uniformly scattered (scattering amount: 7.5 to 12.5 g/m$^2$).

Next, on a surface of a nonwoven fabric (1) where an adhesive had been scattered, 9.0 g (scattering amount: 225 g/m$^2$) of the particulate water absorbent (2) (corresponding to a second particulate water absorbent) was uniformly scattered. Furthermore, a nonwoven fabric (2) made of polyolefins as a main component (product produced by an air-through method, corresponding to an intermediate sheet, basis weight: 50.6 g/m$^2$, thickness was about 3 mm under no load (actual measured value 2.5 mm) , density of the rawmaterial of the nonwoven fabric (2) made of polyolefins as a main component: 0.91g/cm$^3$) was superposed thereon, and the assembly was pressurized and pressure-bonded.

Next, on the surface of the nonwoven fabric (2) on the side that did not face the particulate water absorbent (2), 0.3 to 0.5 g (scattering amount: 7.5 to 12.5 g/m$^2$) of an adhesive including styrene-butadiene rubber (spray adhesive 77, manufactured by 3M Japan, Ltd.) was uniformly scattered. Furthermore, 3.0 g (scattering amount: 75 g/m$^2$) of the particulate water absorbent (1) (corresponding to a first particulate water absorbent) was uniformly scattered thereon.

Finally, a nonwoven fabric (1) made of pulp fibers (product produced by an air-laid method, corresponding to a first base material, basis weight: 42 g/m$^2$) having a thickness of 0.70 mm under no load, which had been cut into a size of 10 cm in length and 40 cm in width as a main component, was superposed on the particulate water absorbent (1), and the assembly was pressurized and pressure-bonded. In this way, water-absorbing sheet (1) was obtained.

Meanwhile, the nonwoven fabric (1) and nonwoven fabric (2) used in the present Example were all water-permeable sheets.

[Example 2]

With regard to Example 1, the particulate water absorbent (3) was used instead of both the particulate water absorbent (1) and the particulate water absorbent (2), and thereby water-absorbing sheet (2) was obtained.

[Example 3]

With regard to Example 1, the particulate water absorbent (4) was used instead of the particulate water absorbent (1), while the particulate water absorbent (5) was used instead of the particulate water absorbent (2), and thereby water-absorbing sheet (3) was obtained.

[Example 4]

With regard to Example 3, the particulate water absorbent (6) was used instead of the particulate water absorbent (5), and thereby water-absorbing sheet (4) was obtained.

[Example 5]

With regard to Example 3, 6. 0 g (scattering amount: 150 g/m$^2$) of the particulate water absorbent (4) and 6.0 g (scattering amount: 150 g/m$^2$) of the particulate water absorbent (5) were used, and thereby water-absorbing sheet (5) was obtained.

[Example 6]

With regard to Example 1, the particulate water absorbent (5) was used instead of the particulate water absorbent (1), while the particulate water absorbent (3) was used instead of the particulate water absorbent (2), and thereby water-absorbing sheet (6) was obtained.

[Example 7]

With regard to Example 1, the particulate water absorbent (7) was used instead of the particulate water absorbent (1), while the particulate water absorbent (5) was used instead of the particulate water absorbent (2), and thereby water-absorbing sheet (7) was obtained.

[Example 8]

With regard to Example 1, the particulate absorbent (8) was used instead of the particulate water absorbent (1), while the particulate absorbent (3) was used instead of the particulate absorbent (2), and thereby water-absorbing sheet (8) was obtained.

[Comparative Example 1]

With regard to Example 1, the particulate water absorbent (7) was used instead of both the particulate water absorbent (1) and the particulate water absorbent (2), and thereby comparative water-absorbing sheet (1) was obtained.

[Comparative Example 2]

With regard to Example 1, the particulate water absorbent (T1) was used instead of the particulate water absorbent (1), while the particulate water absorbent (T2) was used instead of the particulate water absorbent (2), and thereby comparative water-absorbing sheet (2) was obtained.

[Comparative Example 3]

With regard to Example 1, the particulate water absorbent (T2) was used instead of the particulate water absorbent (1), while the particulate water absorbent (T1) was used instead of the particulate water absorbent (2), and thereby comparative water-absorbing sheet (3) was obtained.

[Comparative Example 4]

With regard to Example 1, the particulate water absorbent (1) was used instead of the particulate water absorbent (2), and thereby comparative water-absorbing sheet (4) was obtained.

[Comparative Example 5]

With regard to Example 1, the particulate water absorbent (4) was used instead of both the particulate water absorbent (1) and the particulate water absorbent (2), and thereby comparative water-absorbing sheet (5) was obtained.

[Comparative Example 6]

With regard to Example 1, the particulate absorbent (5) was used instead of the particulate water absorbent (1), while the particulate absorbent (9) was used instead of the particulate absorbent (2), and thereby comparative water-absorbing sheet (6) was obtained.

[Method for evaluating absorbent sheet]

<Reversion amount>

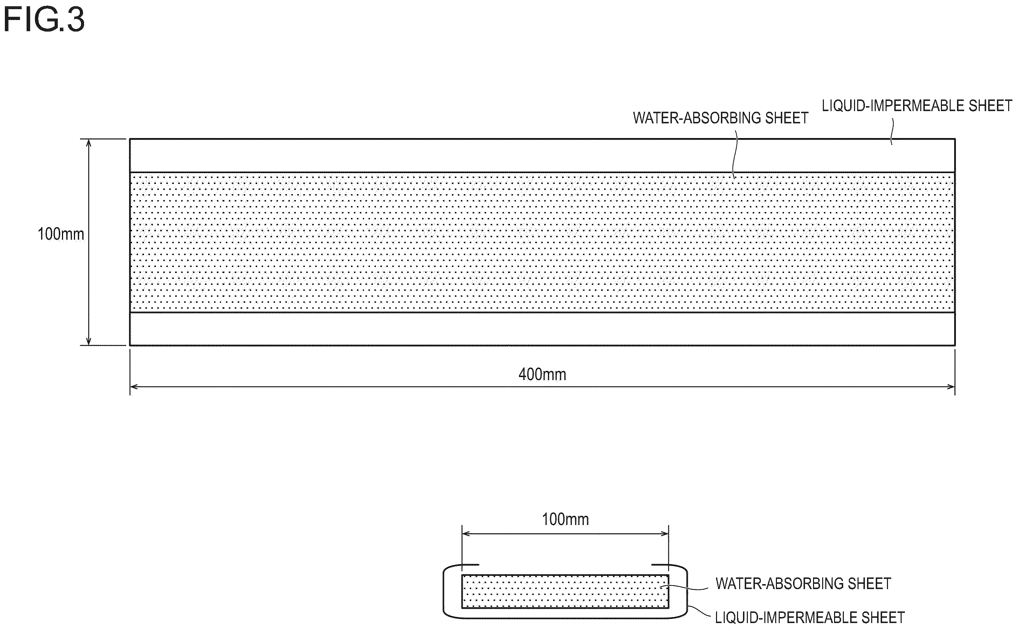

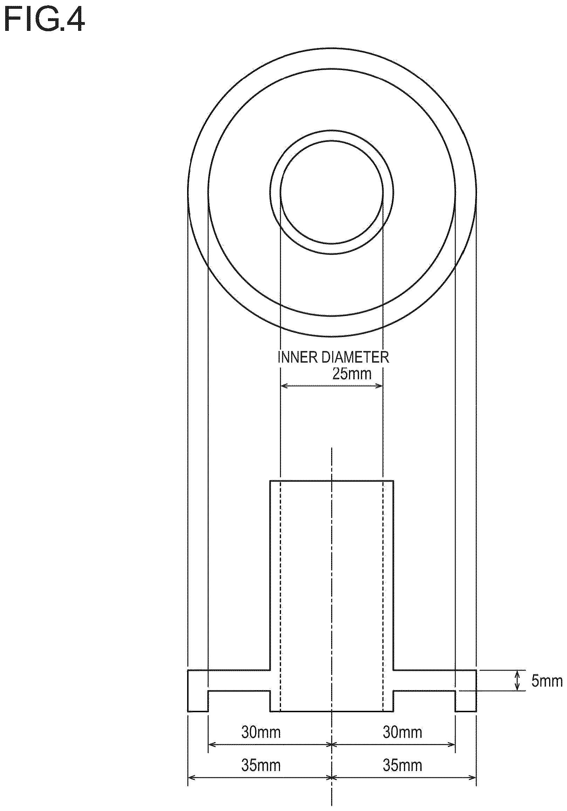

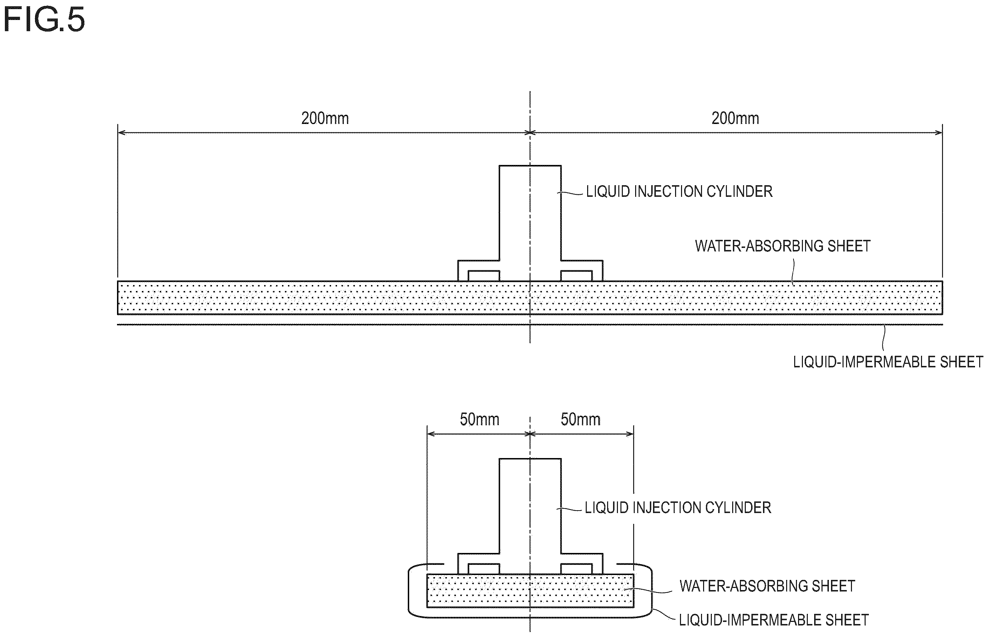

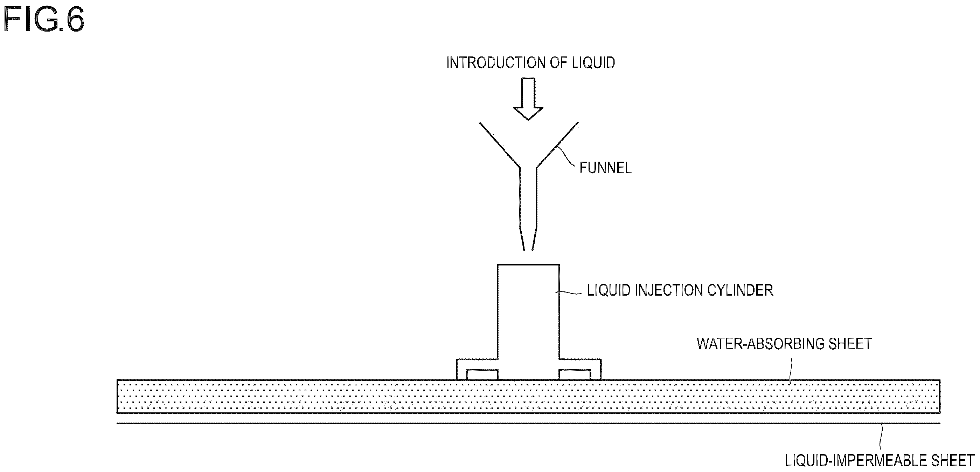

As illustrated in Fig. 3, a water-absorbing sheet that had been cut into a size of 10 cm in length and 40 cm in width was wrapped with a liquid-impermeable sheet having a size of 14 cm in length and 40 cm in width such that an opening was produced at the top. The water-absorbing sheet wrapped with the liquid-impermeable sheet was placed on a flat surface, and a liquid injection cylinder (Fig. 4) was placed thereon at the center of the water-absorbing sheet as illustrated in Fig. 5. In this state, 80 g of a 0.9 wt% aqueous solution of sodium chloride at 23 °C was charged into the liquid injection cylinder using a funnel that enabled liquid charge at a flow rate of 7 ml/second (Fig. 6). Ten minutes after the liquid charge, 20 sheets of filter paper (Model No. 2, manufactured by Advantec MFS, Inc.; circular-shaped product having a diameter of 110 mm), whose weight had been measured in advance, were placed at the center of the water-absorbing sheet, a circular-shaped weight (1,200 g) having a diameter of 100 mm was further placed thereon, and this was retained for 1 minute. After 1 minute, the weight was removed, and the reversion amount first time (g) was measured from a weight increment of the filter paper. One minute after the removal of the weight, a similar operation (10 minutes after the charge of liquid, filter paper and a weight (1,200 g) were placed and retained for 1 minute. After 1 minute, the weight was removed, the measurement of the reversion amount was repeated, and the reversion amount second time (g) and the reversion amount third time (g) were measured. Meanwhile, as the reversion amount third time (g) is smaller, the sample is evaluated to be superior. The reversion amounts of the various water-absorbing sheets and the various comparative water-absorbing sheets are presented in Table 2.

<Diffusivity>

The central site (10 cm in length and 12 cm in width) of the water-absorbing sheet after evaluation was cut out, and the weight was measured (W1 (g)). From the weight before water absorption of the nonwoven fabric used at the center part of the water-absorbing sheet (can be determined from the basis weight of the nonwoven fabric constituting the water-absorbing sheet (W2 (g)) and the weight before water absorption of the particulate water absorbent (can be determined from the scattering basis weight of the particulate water absorbent (W3 (g)), the liquid amount (W4 (g)) remaining at the central site of the water-absorbing sheet after evaluation is determined by the following Formula (i):

$$W4 \ (g) \ = \ W1 \ - \ W2 \ - \ W3 \ (g) \qquad\qquad (i)$$

When the total charged liquid amount is designated as V (g), the reversion amount first time is designated as R1 (g), the reversion amount second time is designated as R2 (g), and the reversion amount third time is designated as R3 (g), the diffusivity can be determined by the following formula (ii).

$$\text{Diffusivity} \ (\%) \ = \ (1 \ - \ (W4 \ + \ R1 \ + \ R2 \ + \ R3)/V) \ \times \ 100 \quad (ii)$$

The diffusivities of the various water-absorbing sheets and the various comparative water-absorbing sheets are presented in Table 2.

[Table 1]

Table 1

| | CRC [g/g] | AAP2.1 kPa [g/g] | Vortex [sec] | GPR [g/min] | Surface tension [g/min] |
|---|---|---|---|---|---|
| Particulate water absorbent 1 | 38.6 | 28.1 | 32 | 48 | 72 |
| Particulate water absorbent 2 | 35.3 | 29.4 | 50 | 116 | 72 |
| Particulate water absorbent 3 | 39.9 | 24.9 | 40 | 28 | 72 |
| Particulate water absorbent 4 | 35.3 | 28.1 | 50 | 120 | 72 |
| Particulate water absorbent 5 | 38.2 | 31.6 | 45 | 8 | 72 |
| Particulate water absorbent 6 | 35.0 | 31.0 | 53 | 17 | 72 |
| Particulate water absorbent 7 | 32.4 | 29.5 | 30 | 180 | 72 |
| Particulate water absorbent 8 | 37.4 | 26.9 | 37 | 46 | 72 |
| Particulate water absorbent 9 | 37.2 | 24.4 | 40 | 51 | 72 |
| Particulate water absorbent T1 | 33.4 | 33.1 | 30 | 2 | 58 |
| Particulate water absorbent T2 | 29.4 | 29.8 | 20 | 84 | 55 |

[Table 2]

Table 2

| | First particulate water absorbent | | | Second particulate water absorbent | | | | | First particulate water absorbent/ second particulate water absorbent (weight ratio) | Return amount [g] | | | Diffusivity [%] |
|---|---|---|---|---|---|---|---|---|---|---|---|---|---|
| | | Water absorption time [sec] | GPR [g/min] | | CRC [g/g] | AAP2.1k Pa [g/g] | Water absorption time [sec] | Overall absorption amount [g/g] | | First time | Second time | Third time | |
| Example 1 | Particulate water absorbent (1) | 32 | 48 | Particulate water absorbent (2) | 35.3 | 29.4 | 50 | 48.2 | 0.33 | 0.2 | 2.1 | 4.7 | 41.9 |
| Example 2 | Particulate water absorbent (3) | 40 | 28 | Particulate water absorbent (3) | 39.9 | 24.9 | 40 | 50.9 | 0.33 | 0.1 | 1.3 | 4.5 | 43 |
| Example 3 | Particulate water absorbent (4) | 50 | 120 | Particulate water absorbent (5) | 38.2 | 31.6 | 45 | 52.1 | 0.33 | 0.7 | 1.9 | 3.5 | 43.8 |
| Example 4 | Particulate water absorbent (4) | 50 | 120 | Particulate water absorbent (6) | 35.0 | 31.0 | 53 | 48.6 | 0.33 | 0.6 | 2.2 | 4.5 | 42.9 |
| Example 5 | Particulate water absorbent (4) | 50 | 120 | Particulate water absorbent (5) | 38.2 | 31.6 | 45 | 52.1 | 1.00 | 0.1 | 1.8 | 4.7 | 42 |

| | First particulate water absorbent | | | Second particulate water absorbent | | | | | First particulate water absorbent/ second particulate water absorbent (weight ratio) | Return amount [g] | | | Diffusivity [%] |
|---|---|---|---|---|---|---|---|---|---|---|---|---|---|
| | | Water absorption time [sec] | GPR [g/min] | | CRC [g/g] | AAP2.1k Pa [g/g] | Water absorption time [sec] | Overall absorption amount [g/g] | | First time | Second time | Third time | |
| Example 6 | Particulate water absorbent (5) | 45 | 8 | Particulate water absorbent (3) | 39.9 | 24.9 | 40 | 50.9 | 0.33 | 0.1 | 1.5 | 4.7 | 38.4 |
| Example 7 | Particulate water absorbent (7) | 30 | 180 | Particulate water absorbent (5) | 38.2 | 31.6 | 45 | 52.1 | 0.33 | 0.2 | 1.9 | 4.1 | 44.1 |
| Example 8 | Particulate water absorbent (8) | 37 | 46 | Particulate water absorbent (3) | 39.9 | 24.9 | 40 | 50.9 | 0.33 | 0.2 | 2.1 | 4.3 | 43.1 |
| Comparative Example 1 | Particulate water absorbent (7) | 30 | 180 | Particulate water absorbent (7) | 32.4 | 29.5 | 30 | 45.4 | 0.33 | 1.1 | 2.7 | 6.7 | 36.6 |
| Comparative Example 2 | Particulate water absorbent (T1) | 30 | 2 | Particulate water absorbent (T2) | 29.4 | 29.8 | 20 | 42.5 | 0.33 | 0.1 | 2.2 | 9.2 | 36.2 |
| Comparative Example 3 | Particulate water absorbent (T2) | 20 | 84 | Particulate water absorbent (T1) | 33.4 | 33.1 | 30 | 48.0 | 0.33 | 0.8 | 2.4 | 7.4 | 30 |

| | First particulate water absorbent | | | Second particulate water absorbent | | | | | First particulate water absorbent/ second particulate water absorbent (weight ratio) | Return amount [g] | | | |
| | | Water absorption time [sec] | GPR [g/min] | | CRC [g/g] | AAP2.1k Pa [g/g] | Water absorption time [sec] | Overall absorption amount [g/g] | | First time | Second time | Third time | Diffusivity [%] |
|---|---|---|---|---|---|---|---|---|---|---|---|---|---|
| Comparative Example 4 | Particulate water absorbent (1) | 32 | 48 | Particulate water absorbent (1) | 38.6 | 28.1 | 32 | 50.0 | 0.33 | 0.6 | 2.1 | 5.3 | 36.8 |
| Comparative Example 5 | Particulate water absorbent (4) | 50 | 120 | Particulate water absorbent (4) | 35.3 | 28.1 | 50 | 47.6 | 0.33 | 0.5 | 1.9 | 6.0 | 38 |
| Comparative Example 6 | Particulate water absorbent (5) | 45 | 8 | Particulate water absorbent (9) | 37.2 | 24.4 | 40 | 47.9 | 0.33 | 0.7 | 3.1 | 6.3 | 40.6 |

EP 3 834 789 B1

[Reference Signs List]

11: first base material
12: water-absorbing layer
13: second base material
14: particulate water absorbent
14a: first particulate water absorbent
14b: second particulate water absorbent
16: intermediate sheet
400: apparatus
410: container
411: cell
412: piston
413a, 413b: wire gauze
414: swollen gel (product obtained by causing a particulate water absorbent to absorb water)
415: hole
420: tank
421: glass tube
422: L-shaped tube with a cock glass tube
423: liquid
431: stainless steel wire gauze
432: capturing container
433: Roberval balance

The present application is based on Japanese Patent Application No. 2018-150124, Japanese Patent Application No. 2018-150125, and Japanese Patent Application No. 2018-150129, all filed August 9, 2018, Japanese Patent Application No. 2018-185701 and Japanese Patent Application No. 2018-185706, all filed September 28, 2018.

The above description is an explanation for the "first aspect of the present invention".

"Second aspect of the present description" and "third aspect of the present description", which are inventions separate from the "first aspect of the present invention", however not claimed, will be described below. The "second aspect of the present description" is an invention separate from the "first aspect of the present invention" and the "third aspect of the present description". Similarly, the "third aspect of the present description" is an invention separated from the "first aspect of the present invention" and the "second aspect of the present description".

<Second aspect of present description>

According to the second aspect of the present description, the above-described problems will be solved by a water-absorbing sheet having a first base material, a second base material, and a water-absorbing layer positioned between the first base material and the second base material, wherein the first base material is a water-permeable sheet positioned on the side where a liquid to be absorbed is introduced, the water-absorbing layer has a particulate water absorbent and an intermediate sheet, the weight average particle size of the particulate water absorbent is 200 to 600 $\mu$m, the CRC of the particulate water absorbent is 36 g/g or more, and the permeability of the particulate water absorbent to the intermediate sheet is 60% or higher.

[1. Definitions of terms]

[1-1. Water-absorbing sheet]

The "water-absorbing sheet" according to the second aspect of the description refers to a structure in which a water-absorbing resin (particulate water absorbent) and an intermediate sheet are supported between two or more sheets of long base materials. The water-absorbing sheet may use an adhesive, or may use a hot melt adhesive, for the adhesion between at least any of the base materials, the intermediate sheet, and the particulate water absorbent. The water-absorbing sheet may include other components (a fiber component, an antibacterial agent, a deodorant, and the like) in addition to the particulate water absorbent. Furthermore, the water-absorbing sheet may also include another sheet in addition to the two sheets of base material having the particulate water absorbent and the like interposed therebetween.

Usually, a water-absorbing sheet is in the form of a continuous sheet or a roll form obtained by winding the continuous sheet. When the water-absorbing sheet is used, a continuous sheet is cut out into an appropriate shape (rectangular shape or the like) and then is used as an absorbent body such as a disposable diaper. On the other hand, a conventional disposable diaper of a water-absorbing resin at a high concentration (for example, a disposable diaper in which the absorbent body is pulpless) uses an absorbent body that is mold-made for each sheet of the disposable diaper. Therefore, such an absorbent body differs from the water-absorbing sheet of the second aspect of the description in view of the technical properties.

[1-2. Water-absorbing resin]

The "water-absorbing resin" according to the present specification refers to a polymer gelling agent in which the water-swellability (CRC) defined in ERT441.2-02 is 5 g/g or more, and the water-soluble component (Ext) defined in ERT470.2-02 is 50% by mass or less.

The water-absorbing resin is preferably a hydrophilic crosslinked polymer by crosslink polymerizing an unsaturated monomer having a carboxyl group. The shape of the water-absorbing resin is a sheet form, a fibrous form, a film form, a particulate form, a gel form, or the like. The water-absorbing sheet according to an embodiment of the second aspect of the description uses a particulate water-absorbing resin.

The "water-absorbing resin" according to the present specification is not limited to an embodiment in which the total amount (100% by mass) is composed only of the water-absorbing resin. The water-absorbing resin may also be a water-absorbing resin composition including additives and the like. Furthermore, the "water-absorbing resin" according to the present specification is a concept that also includes intermediate bodies in the production process for the water-absorbing resin. For example, a hydrated gel-like crosslinked polymer after polymerization, a dried polymer after drying, a water-absorbing resin powder before surface crosslinking, and the like may also be described as the "water-absorbing resin".

As such, in the present specification, in addition to the water-absorbing resin itself, a water-absorbing resin composition and intermediate bodies may also be collectively referred to and described as "water-absorbing resin".

[1-3. Water absorbent, particulate water absorbent]

The "water absorbent" according to the present specification means an absorbent gelling agent for absorbing an aqueous liquid (liquid), the absorbent gelling agent including a water-absorbing resin as a main component. Here, the aqueous liquid (liquid) is not particularly limited so long as it is water as well as a liquid including water. Examples of the aqueous liquid that the water-absorbing sheet according to an embodiment of the second aspect of the description absorbs include urine, menstrual blood, sweat, and other body fluids.

The "particulate water absorbent" according to the present specification means a water absorbent in the form of particle (powdery form) (since the water-absorbing resin is included as a main component in the water absorbent, the particulate water absorbent corresponds to a water-absorbing resin in the form of particle). In the concept of the "particulate water absorbent", a single grain of the particulate water absorbent and an aggregate of a plurality of particulate water absorbents are all included. The term "particulate" according to the present specification means having the form of particle. Here, the "particle" refers to a relatively small divided body of a material and has a size of several Å to several mm (see "Particles", "McGraw-Hill Dictionary of Scientific and Technical Terms, 3rd Edition", edited by McGraw-Hill Dictionary of Scientific and Technical Terms Editorial Board, Nikkan Kogyo Shimbun, Ltd., 1996, p. 1929). Meanwhile, in the present specification, the "particulate water absorbent" may be described simply as "water absorbent". With regard to the water-absorbing sheet of the second aspect of the description, the weight average particle size of the particulate water absorbent is 200 to 600 $\mu$m. Here, when the weight average particle size of the particulate water absorbent is less than 200 pm, there is a risk that handleability may be deteriorated. Furthermore, when the weight average particle size of the particulate water absorbent is more than 600 pm, there is a risk that the feel of the water-absorbing sheet to the touch may be deteriorated. With regard to the water-absorbing sheet according to an embodiment of the second aspect of the description, the weight average particle size of the particulate water absorbent is preferably 250 to 500 pm, and more preferably 300 to 450 $\mu$m. Furthermore, with regard to the water-absorbing sheet of the second aspect of the description, it is preferable that 95% by mass or more of the entirety of the particulate water absorbent has a particle size of 850 $\mu$m or less, it is more preferable that 98% by mass or more of the entirety of the particulate water absorbent has a particle size of 850 $\mu$m or less, and it is even more preferable that substantially 100% by mass of the entirety of the particulate water absorbent has a particle size of 850 $\mu$m or less. Incidentally, in the Examples of the present application, substantially 100% by mass of the entirety of the particulate water absorbent has a particle size of 850 $\mu$m or less. In the present specification, the method for measuring the weight average particle size is calculated by a method similar to " (3) Mass-Average Particle Diameter (D50) and Logarithmic Standard Deviation ($\sigma\zeta$) of Particle Diameter Distribution" described in US Patent No. 7638570, based on the PSD obtained according to the method for measuring the "PSD" defined in ERT420.2-02.

With regard to the water-absorbing sheet according to an embodiment of the second aspect of the description, the weight average particle size of the particulate water absorbent is 400 μm or less. By being such an embodiment, the intended effects of the second aspect of the description can be enhanced.

The particulate water absorbent includes a water-absorbing resin as a polymer (alternatively, also referred to as particulate water-absorbing resin or water-absorbing resin particles) as a main component. The particulate water absorbent includes the water-absorbing resin as a polymer at a proportion of 60% to 100% by mass, preferably 70% to 100% by mass, more preferably 80% to 100% by mass, even more preferably 90% to 100% by mass, and particularly preferably 95% to 100% by mass. The remaining portion of the particulate water absorbent may optionally include water, additives (inorganic fine particles, polyvalent metal cations, and the like), and the like. Incidentally, the particulate water absorbents used in the Examples of the present application include about 95% to about 99% by mass of a water-absorbing resin. That is, the upper limit of the water-absorbing resin in the particulate water absorbent is, for example, 100% by mass, 99% by mass, 97% by mass, 95% by mass, or 90% by mass. Preferably, the particulate water absorbent further includes, in addition to the water-absorbing resin, 0% to 10% by mass of components, particularly water, additives (inorganic fine particles, polyvalent metal cation), and the like.

Incidentally, a preferred percentage water content of the particulate water absorbent is 0.2% to 30% by mass. As described above, a water-absorbing resin composition in which components such as water and additives are integrated with the water-absorbing resin and/or a water-absorbing resin composition in the form in which the components are mixed with the water-absorbing resin, is also included in the "particulate water absorbent".

Examples of the water-absorbing resin that serves as a main component of the particulate water absorbent include a polyacrylic acid (salt)-based resin, a polysulfonic acid (salt)-based resin, a maleic anhydride (salt)-based resin, a polyacrylamide-based resin, a polyvinylalcohol-based resin, a polyethylene oxide-based resin, a polyaspartic acid (salt)-based resin, a polyglutamic acid (salt)-based resin, a polyalginic acid (salt) -based resin, a starch-based resin, and a cellulose-based resin. Among these, preferably, a polyacrylic acid (salt)-based resin is used as the water-absorbing resin.

[1-4. Polyacrylic acid (salt)]

The "polyacrylic acid (salt)" according to the present specification refers to polyacrylic acid and/or a salt thereof . The polyacrylic acid (salt) is a polymer that includes a repeating unit of acrylic acid and/or a salt thereof (hereinafter, referred to as "acrylic acid (salt)") as a main component and further includes a graft component as an optional component. The polyacrylic acid (salt) is obtained by polymerization of acrylic acid (salt), hydrolysis of polyacrylamide, polyacrylonitrile, or the like, and the like. Preferably, the polyacrylic acid (salt) is obtained by polymerization of acrylic acid (salt).

Here, the phrase "includes as a main component" implies that the amount of use of acrylic acid (salt) at the time of polymerizing polyacrylic acid (salt) is usually 50 mol% to 100 mol%, preferably 70 mol% to 100 mol%, more preferably 90 mol% to 100 mol%, and even more preferably substantially 100mol%, with respect to the entirety of the monomers (however, excluding an internal crosslinking agent) used for polymerization.

[1-5. EDANA and ERT]

"EDANA" is an acronym for the European Disposables and Nonwovens Associations. "ERT" is an acronym for methods for measuring a water-absorbing resin of the European standards (substantially, international standards) enacted by EDANA (EDANA Recommended Test Methods). In the present specification, unless particularly stated otherwise, the physical properties of the water-absorbing resin are measured according to ERT of the version of year 2002.

[1-6. Others]

According to the present specification, the expression "X to Y" representing a range means "X or more and Y or less" .

According to the present specification, unless particularly annotated otherwise, a unit of mass, "t (ton)", means "Metric ton". The unit "ppm" means "ppm by mass". The "mass" and "weight", "parts by mass" and "parts by weight", "% by mass" and "% by weight", and "ppm by mass" and "ppm by weight" are respectively considered to have the same meanings.

According to the present specification, the term "acid (salt)" means "acid and/or a salt thereof". The term "(meth)acryl" means "acryl and/or methacryl".

According to the present specification, the volume unit "liter" may be described as "l" or "L". The unit "% by mass" may be described as "wt%". In a case in which measurement of trace components is carried out, the detection limit or less is described as N.D. (Non Detected).

[2. Water-absorbing sheet]

The water-absorbing sheet of the second aspect of the description is a water-absorbing sheet having a first base material, a second base material, and a water-absorbing layer positioned between the first base material and the second base material, wherein the first base material is a water-permeable sheet positioned on the side where a liquid to be absorbed is introduced, the water-absorbing layer has a particulate water absorbent and an intermediate sheet, the weight average particle size of the particulate water absorbent is 200 to 600 pm, the CRC of the particulate water absorbent is 36 g/g or more, and the permeability of the particulate water absorbent to the intermediate sheet is 60% by mass or more. Hereinafter, for the permeability, % by mass is simply indicated as %. Due to such a configuration, even when introduction of a liquid occurs intermittently for several times (particularly, three or more times) under a situation with no load and the amount of introduction of the liquid becomes large, the return amount can be meaningfully reduced. Particularly, due to such a configuration, very excellent results can be obtained in the measurement of the return amount under particular conditions in the Examples of the present application (in the present specification, also referred to "evaluation of the specific return amount") . That is, when there is intermittent introduction of a liquid for several times (particularly, three or more times), in a conventional configuration, the liquid amount is more than or equal to the set amount of absorption, and excessive "return" occurs. In contrast, in the second aspect of the description, the permeability of the particulate water absorbent to the intermediate sheet is 60% or higher. When a combination of a particulate water absorbent and an intermediate sheet capable of realizing such a permeability is used, the intermediate sheet can easily capture the particulate water absorbent present in the water-absorbing sheet. As the intermediate sheet captures a large amount of the particulate water absorbent, the particulate water absorbent diffuses in the water-absorbing sheet, and gaps between the particles of the particulate water absorbent present in the water-absorbing sheet are likely to become vacant. If the permeability is lower than 60%, the particulate water absorbent existing between the intermediate sheet and the base materials is not easily captured by the intermediate sheet, the probability of the particulate water absorbent existing between the intermediate sheet and the base materials increases, and the particulate water absorbent exists compactly between the intermediate sheet and the base material. Then, a gel blocking phenomenon occurs between the particles of the particulate water absorbent that have absorbed water, liquid diffusibility is decreased, and excessive "return amount" occurs. In the second aspect of the description, since the permeability is 60% or higher, as the particulate water absorbent existing between the intermediate sheet and the base materials diffuses widely into the intermediate sheet, gaps between the particles of the particulate water absorbent are likely to become vacant, the performance of the particulate water absorbent can be exhibited at the maximum level, and even as a water-absorbing sheet, there is a technical effect in which the area capable of performing water absorption can be utilized more widely. Therefore, the "evaluation of the specific return amount" can be achieved with excellence. Furthermore, when the CRC is 36 g/g or more, there is a tendency that the diffusibility of a liquid is generally likely to be decreased; however, since the permeability is 60% or higher in the second aspect of the description, the intended problems of the second aspect of the description can be solved, while an advantage that the amount of water absorption is large while maintaining the diffusibility of the liquid is best utilized. Here, it should be additionally remarked that a water-absorbing sheet or an absorbent article, which has been designed so as to suppress the return amount under general conditions, does not necessarily give excellent results in regard to the "evaluation of the specific return amount" of the present application. Furthermore, the water-absorbing sheet according to an embodiment of the second aspect of the description is suitable as, for example, an absorbent article (for example, a diaper) which an infant who has just learned to run and still has a small urinary bladder uses during a period of time for actively moving around, such as daytime; however, of course, the usage form is not limited to this. Here, it is considered that as long as the porosity is increased, the permeability can be brought to 60% or higher. However, hypothetically it is not always true that an intermediate sheet having a high porosity can permeate a large amount of the particulate water absorbent. A method of realizing a permeability to be 60% or higher will be described below; however, a method of controlling (when the constituent member of the intermediate sheet is fibers) the fiber diameter, or (when the intermediate sheet is a nonwoven fabric) superposing a plurality of sheets of a nonwoven fabric is considered suitable. That is, a permeability of 60% or higher can be realized by appropriately adjusting the properties of the member constituting the intermediate sheet, the surface state thereof, complicatedness of the network structure, the fiber diameter, the fused state between fibers, the basis weight, thickness, and the like. A person who attempts to carry out the second aspect of the description can adjust the permeability, for example, in a case in which an air-through nonwoven fabric is used as the intermediate sheet, by changing the heat treatment conditions for the air-through nonwoven fabric, and the fiber diameter and the density. Even in the case of using intermediate sheets of different types, a permeability of 60% or higher can be realized by appropriately modifying a factor involved in the adjustment of the permeability such as described above.

In the following description, embodiments of the second aspect of the description will be described with reference to the attached drawings. Incidentally, identical reference signs will be assigned to identical elements in the description of the drawings, and any overlapping explanations will not be repeated. Furthermore, the dimensional ratios of the drawings are exaggerated for the convenience of explanation and may be different from the actual ratios.

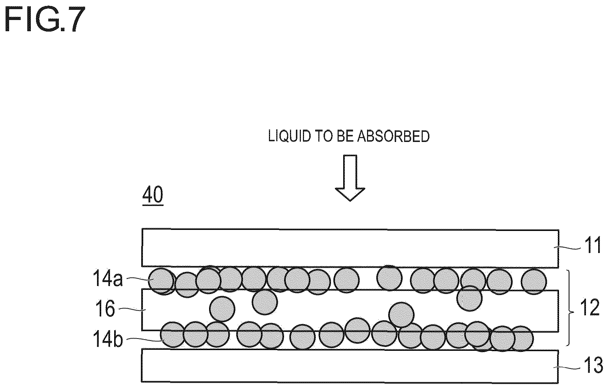

Fig. 7 is a schematic diagram illustrating a cross-section of a water-absorbing sheet 40 according to an embodiment of the second aspect of the description. A first base material 11 is positioned on the side where a liquid to be absorbed is introduced. That is, at least the first base material is disposed on the discharge side of the liquid (for example, the skin side in a paper diaper). A water-absorbing layer 12 is disposed between the first base material 11 and a second base material 13. Incidentally, in Fig. 7, the second base material 13 is positioned only on the side opposite to the side where the liquid to be absorbed is introduced, with respect to the water-absorbing layer 12 interposed therebetween; however, for example, the area of the second base material 13 may be designed to be larger than that of the first base material 11 so that the second base material 13 may be folded up so as to wrap the first base material 11. By doing so, fall-off of particulate water absorbents 14a and 14b can be suppressed. In the water-absorbing sheet 40 of Fig. 7, the first particulate water absorbent 14a localized on the side of a surface of the first base material 11, the surface being arranged to face the second base material 13, and the second particulate water absorbent 14b localized on the side of a surface of the second base material 13, the surface being arranged to face the first base material 11, are localized so as to interpose an intermediate sheet 16; however, the particulate water absorbent 14a and the particulate water absorbent 14b may be uniformized. The water-absorbing layer 12 has a particulate water absorbent 14 and an intermediate sheet 16. Since this intermediate sheet 16 is such that the permeability of the particulate water absorbent 14 is 60% or higher, the intermediate sheet has a structure that can easily capture the particulate water absorbent 14a and the particulate water absorbent 14b. Therefore, within this intermediate sheet 16, large quantities of the particulate water absorbent 14a and the particulate water absorbent 14b are present. That is, as the particulate water absorbent 14a and the particulate water absorbent 14b diffuse significantly into the intermediate sheet 16 and exist in the water-absorbing sheet, the intended effects of the second aspect of the description are provided. In a preferred embodiment of the second aspect of the description, the particulate water absorbent 14a and the particulate water absorbent 14b diffuse into the intermediate sheet 16 while maintaining a localized state as far as possible.

As described above, in the form of Fig. 7, the water-absorbing layer 12 has a particulate water absorbent 14a fixed to the first base material 11, and a particulate water absorbent 14b fixed to the second base material; however, portions of the particulate water absorbents 14a and 14b can be detached from the respective sheets. Therefore, the water-absorbing "layer" does not refer only to a continuous body such as a sheet but may have any form so long as it exists with a certain thickness between the first base material 11 and the second base material 13. Regarding the method of fixing particulate water absorbents to the respective base materials, for example, an adhesive may be used. A method for producing a water-absorbing sheet using an adhesive will be described in detail in [3.].

The water-absorbing sheet according to an embodiment of the second aspect of the description can be made thinner than the absorbent bodies used in the conventional type absorbent articles. In a case in which the water-absorbing sheet is used for a disposable diaper, the thickness is, for example, at 40%RH to 50%RH, preferably 15 mm or less, more preferably 10 mm or less, even more preferably 7 mm or less, particularly preferably 5 mm or less, and most preferably 4 mm or less. On the other hand, in view of the strength of the water-absorbing sheet and the diameters of the particulate water absorbents, the lower limit of the thickness is 0.2 mm or more, preferably 0.3 mm or more, and more preferably 0.5 mm or more. The thickness of the water-absorbing sheet used in the Examples of the present application was 3 mm to 5 mm under the above-described conditions.

Incidentally, the thickness of the water-absorbing sheet is measured using a Dial Thickness Gauge large-sized type (thickness measuring machine) (manufactured by Ozaki Manufacturing Co., Ltd., product No. : J-B, gauge head: anvils upper and lower $\phi$ 50 mm) . Regarding the measurement sites, an absorbent body was divided into three equal parts in the longitudinal direction, and the respective center parts (a point positioned at an intersection point obtained by drawing diagonal lines from the corners of the absorbent body) were set as the measurement sites. For example, in the case of a water-absorbing sheet measuring 36 cm in the longitudinal direction and 10 cm in the width direction, the measurement positions are such that, with respect to the length of 36 cm in the longitudinal direction, three points such as a point 6 cm away from the left end in the longitudinal direction and 5 cm away from both ends in the width direction (designated as left) ; a point 18 cm away from the left end in the longitudinal direction and 5 cm away from both ends in the width direction (designated as center) ; and a point 30 cm away from the left end in the longitudinal direction and 5 cm away from both ends in the width direction (designated as right), correspond to the measurement sites. The measurement score is obtained by making measurement two times at each site, and the measured value of thickness is designated as the average value of six points in total. Regarding a specific procedure, a water-absorbing sheet is stuck flatly on a plate having a uniform thickness so that no crease or distortion is produced at the measurement sites of the water-absorbing sheet, and the plate is mounted on a lower gauge head of a thickness measuring machine . Next, an upper gauge head of the thickness measuring machine is approached to a position 2 to 3 mm high from the water-absorbing sheet, subsequently the handle is slowly released from the hands, and the thickness combining the water-absorbing sheet and the plate is measured. The thickness of the water-absorbing sheet is determined by formula: $T1 = T2 - T0$ (T0: thickness of plate (mm) , T1: thickness of water-absorbing sheet (mm), T2: thickness of water-absorbing sheet and plate (mm)).

In order to further impart liquid passability, diffusibility, flexibility, and the like to the water-absorbing sheet, the surface of the water-absorbing sheet may be appropriately subjected to embossing processing. The region that is subjected to embossing processing may be the entire face of the water-absorbing sheet surface or may be a portion thereof. By providing a continuously embossing-processed region in the longitudinal direction of the water-absorbing sheet, a liquid can be caused to diffuse easily in the longitudinal direction. Furthermore, the particulate water absorbents may be scattered on the entire surface of the water-absorbing sheet, or particulate water absorbent-absent regions may be provided in some parts. In the case of providing particulate water absorbent-absent regions, it is preferable to provide the absent regions in a channel form (striped shape) in the longitudinal direction of the water-absorbing sheet. As such, when embossing-processed regions and/or particulate water absorbent-absent regions are provided consecutively in the longitudinal direction, those regions accomplish the role as passages for allowing large quantities of liquid to flow through (liquid conveyance passages). The embossing-processed regions and/or the particulate water absorbent-absent regions may be provided in a linear shape, may be provided in a curved shape, or may be provided in a wavy shape.

In the following description, various members constituting the water-absorbing sheet will be described in detail.

[2-1. First base material, second base material, and intermediate sheet]

The first base material is a water-permeable sheet positioned on the side where a liquid to be absorbed is introduced. Incidentally, the liquid to be absorbed is not limited to water and may be urine, blood, sweat, feces, wastewater, moisture, ice, a mixture of water and an organic solvent and/or an inorganic solvent, rain water, underground water, or the like. The liquid to be absorbed is not particularly limited so long as it includes water. Preferred examples include urine, menstrual blood, sweat, and other body fluids.

The lower limit of the permeability of the particulate water absorbent to the intermediate sheet in the water-absorbing sheet according to an embodiment of the second aspect of the description is not particularly limited as long as it is 60% or higher; however, the lower limit is preferably 65% or higher, more preferably 70% or higher, even more preferably 75% or higher, still more preferably 80% or higher, even more preferably 85% or higher, still more preferably 87% or higher, even more preferably 89% or higher, still more preferably 90% or higher, even more preferably 91% or higher, still more preferably 92% or higher, even more preferably 93% or higher, still more preferably 94% or higher, and even more preferably 95% or higher. By having such a lower limit, the capture ratio of the particulate water absorbent becomes high, and the intended effects of the second aspect of the description are easily exhibited. Furthermore, the upper limit of the permeability of the particulate water absorbent to the intermediate sheet in the water-absorbing sheet according to an embodiment of the second aspect of the description is theoretically 100%; however, the upper limit is preferably 99. 9% or lower, more preferably 99.5% or lower, even more preferably 99% or lower, still more preferably 98% or lower, and even more preferably lower than 97% . By having such a permeability, the intermediate sheet can capture a large amount of the particulate water absorbent.

The porosity of the intermediate sheet in the water-absorbing sheet according to an embodiment of the second aspect of the description is preferably 80% to 99.9%, more preferably 85% to 99.9%, even more preferably 90% to 99.9%, particularly preferably 98.0% (or 98.1%) to 99.9%, and most preferably 98.2% (98.3% or 98.4%) to 99.5% (or 99.0%). Incidentally, as described above, since a permeability of 60% or higher can be realized by means of the properties of the member constituting the intermediate sheet, the surface state thereof, the complicatedness of the network structure, the fiber diameter, the fused state between fibers, the basis weight, thickness, and the like, there is no direct correlation between porosity and permeability.

The materials of the first base material, the second base material, and the intermediate sheet used for the water-absorbing sheet according to an embodiment of the second aspect of the description are each independently preferably a nonwoven fabric. The raw material of the nonwoven fabric is not particularly limited; however, from the viewpoints of liquid penetrability, flexibility, and the strength of the water-absorbing sheet, polyolefin fibers (polyethylene (PE), polypropylene (PP), and the like), polyester fibers (polyethylene terephthalate (PET), polytrimethylene terephthalate (PTT), polyethylene naphthalate (PEN), and the like), polyamide fibers (nylon and the like), rayon fibers, pulp (cellulose) fibers, and the like are preferred. Furthermore, nonwoven fabrics of other synthetic fibers, and nonwoven fabrics produced by mixing synthetic fibers with cotton, silk, hemp, pulp (cellulose) fibers and the like are also similarly preferred. The nonwoven fabric described above may be a nonwoven fabric including only one kind of the above-mentioned fibers or a nonwoven fabric combining two or more kinds of fibers. It is preferable that the nonwoven fabrics used for the first base material and the second base material are produced by an air-laid method. Furthermore, pulp (cellulose) fibers are preferred.

With regard to the water-absorbing sheet according to an embodiment of the second aspect of the description, the nonwoven fabric used for the intermediate sheet is preferably an air-through nonwoven fabric. Furthermore, it is preferable that the nonwoven fabric used for the intermediate sheet is bulky, and specifically, the thickness under no load is preferably 1.3 mm or more, more preferably 1.5 mm or more, even more preferably 1.7 mm or more, still more preferably 1.9 mm or more, even more preferably 2.1 mm or more, still more preferably 2.3 mm or more, and even more preferably 2.5 mm or more. The nonwoven fabric described above may include a small amount of pulp fibers to the extent that does not increase the thickness of the water-absorbing sheet.

The upper limit of the thickness of the nonwoven fabric used for the intermediate sheet is also not particularly limited; however, for example, the thickness under no load is preferably 4.9 mm or less, more preferably 4.8 mm or less, even more preferably 4.7 mm or less, still more preferably 4.6 mm or less, and particularly preferably less than 4.0 mm. With regard to the water-absorbing sheet according to an embodiment of the second aspect of the description, the basis weight of the intermediate sheet is preferably 30 to 60 $g/m^2$, more preferably 40 to 59 $g/m^2$, and even more preferably 42 to 55 $g/m^2$, per sheet of the intermediate sheet.

With regard to the water-absorbing sheet according to an embodiment of the second aspect of the description, the thickness of the intermediate sheet is thicker than the thickness of either the first base material or the second base material. More suitably, the thickness of the intermediate sheet is thicker than the thickness of both the first base material and the second base material. Such an embodiment contributes to water retention in the early stage of liquid absorption and conduces to the reduction of leakage, the first base material and the second base material can be designed to be thin, and the overall thickness of the water-absorbing sheet can be made thinner. The ratio of the thickness of the intermediate sheet with respect to the arithmetic mean of the thicknesses of the first base material and the second base material is preferably 1.5 to 100, more preferably 2 to 80, even more preferably 3 to 50, and still more preferably 3.2 to 10.

As mentioned above, the nonwoven fabric used for the water-absorbing sheet according to an embodiment of the second aspect of the description is preferably a hydrophilic nonwoven fabric in order to increase the water permeability; however, the nonwoven fabric or the fibers as the material of the nonwoven fabric may be hydrophilized using a hydrophilizing agent (surfactant or the like).

Regarding examples of the hydrophilizing agent include anionic surfactants (aliphatic sulfonic acid salts, higher alcohol sulfuric acid ester salts, and the like), cationic surfactants (quaternary ammonium salts and the like), nonionic surfactants (polyethylene glycol fatty acid esters, polyglycerin fatty acid esters, sorbitan fatty acid esters, and the like), silicone-based surfactants (polyoxyalkylene-modified silicone, and the like), stain release agents including polyester-based, polyamide-based, acrylic, and urethane-based resins, and the like are used.

The first base material used for the water-absorbing sheet according to an embodiment of the second aspect of the description is a water-permeable sheet because it is located on the side where the liquid to be absorbed is introduced; however, it is preferable that the second base material and the intermediate sheet are also water-permeable sheets having water permeability, and the two may be of the same type or may be of different types. With regard to the water-permeability for the water-permeable sheet, the water permeability coefficient (JIS A1218:2009) is preferably 1 $\times$ $10^{-5}$ cm/sec or higher. This water permeability coefficient is more preferably 1 $\times$ $10^{-4}$ cm/sec or higher, even more preferably 1 $\times$ $10^{-3}$ cm/sec or higher, still more preferably 1 $\times$ $10^{-2}$ cm/sec or higher, and even more preferably 1 $\times$ $10^{-1}$ cm/sec or higher. The water-permeable sheet used for the water-absorbing sheet according to an embodiment of the second aspect of the description is preferably a hydrophilic nonwoven fabric. By being a hydrophilic nonwoven fabric, the intended effects of the second aspect of the description can be efficiently provided.

It is more desirable as the thicknesses of the first base material and the second base material are thinner to the extent of having strength as a water-absorbing sheet, and for each sheet of the base material, the thicknesses are each independently appropriately selected in the ranges of 0.01 to 2 mm, 0.02 to 1 mm, 0.03 to 0.9 mm, and 0.05 to 0.8 mm. The basis weights of the first base material and the second base material are each independently preferably 5 to 300 $g/m^2$, more preferably 8 to 200 $g/m^2$, even more preferably 10 to 100 $g/m^2$, and still more preferably 11 to 50 $g/m^2$, per sheet of the base material.

[2-2. Water-absorbing layer]

The water-absorbing layer in the water-absorbing sheet according to an embodiment of the second aspect of the description includes a particulate water absorbent and an intermediate sheet. When the water-absorbing layer has an intermediate sheet, there is an advantage that the first particulate water absorbent and the second particulate water absorbent are likely to be localized more efficiently on the first base material side and the second base material side, respectively. Furthermore, when the intermediate sheet constitutes a layer such as an air layer, there is an advantage that even if there is reversion from the water-absorbing sheet, the skin may not feel the reversion. Since the explanation about the intermediate sheet is as described above, further explanation will not be repeated here. Incidentally, localization of the first particulate water absorbent and the second particulate water absorbent can be realized by, for example, appropriately utilizing an adhesive, as described in the following section [3.].

(Particulate water absorbent)

The water-absorbing layer in the water-absorbing sheet according to an embodiment of the second aspect of the description includes a first particulate water absorbent and a second particulate water absorbent. Incidentally, unless particularly stated otherwise, when it is simply described as particulate water absorbent, this means a mixture of a first particulate water absorbent and a second particulate water absorbent, or at least one of a first particulate water absorbent and a second particulate water absorbent. Furthermore, in a case in which the first particulate water absorbent and/or the second particulate water absorbent are mixtures of a plurality of kinds of particulate water absorbents, the following description is an explanation of the physical properties of these mixtures.

"CRC" (ERT441.2-02)

The term "CRC" is an acronym for "Centrifuge Retention Capacity" and means a water absorption ratio with no load (may also be referred to as "water absorption ratio") of a particulate water absorbent. Specifically, CRC refers to a water absorption ratio (unit: g/g) obtained by introducing 0.2 g of a particulate water absorbent into a nonwoven fabric bag, subsequently immersing the bag in a large excess of a 0.9 mass% aqueous solution of sodium chloride for 30 minutes so as to cause the particulate water absorbent to freely swell, and then dehydrating the particulate water absorbent in a centrifuge (250 G).

The CRC of the second particulate water absorbent in the water-absorbing sheet according to an embodiment of the second aspect of the description is preferably 30 to 50 g/g, more preferably 36 to 45 g/g, and even more preferably 37 to 44 g/g. By being such an embodiment, excellent results are obtained in the evaluation of the specific return amount.

The CRC of the first particulate water absorbent in the water-absorbing sheet according to an embodiment of the second aspect of the description is preferably 30 to 50 g/g, more preferably 31 to 48 g/g, and even more preferably 32 to 45 g/g. By being such an embodiment, excellent results are obtained in the evaluation of the specific return amount.

With regard to the water-absorbing sheet according to an embodiment of the second aspect of the description, the CRC of the second particulate water absorbent to the CRC of the first particulate water absorbent is preferably more than 1 and 2 or less, more preferably 1.03 to 1.5, and even more preferably 1.05 to 1.4. As such, as the CRC of the lower layer (layer of the second particulate water absorbent) is set to be higher than the CRC of the upper layer (layer of the first particulate water absorbent), the evaluation of the specific return amount can be made more excellent; however, the second aspect of the description is not limited to such an embodiment.

The lower limit of the CRC of the particulate water absorbent (CRC of a mixture of the first particulate water absorbent and the second particulate water absorbent) in the water-absorbing sheet according to an embodiment of the second aspect of the description is 36 g/g or more. When the CRC is less than 36 g/g, the amount of water absorption is insufficient, and there is a risk that excellent results cannot be obtained in the evaluation of the specific return amount. On the other hand, when the CRC is 36 g/g or more, the liquid diffusibility generally tends to be easily decreased; however, in the second aspect of the description, since the permeability is 60% or higher, the intended problems of the second aspect of the description can be solved while making the best of the advantage that the amount of water absorption is large, while still maintaining the liquid diffusibility. The lower limit of the CRC of the particulate water absorbent (CRC of a mixture of the first particulate water absorbent and the second particulate water absorbent) in the water-absorbing sheet according to an embodiment of the second aspect of the description is preferably 36.1 g/g or more, more preferably 36.2 g/g or more, even more preferably 36.3 g/g or more, and still more preferably 36.4 g/g or more. By being such an embodiment, excellent results are obtained in the evaluation of the specific return amount. The upper limit is preferably 50 g/g or less, more preferably 48 g/g or less, and even more preferably 45 g/g or less.

"AAP" (ERT442.2-02)

"AAP" is an acronym for "Absorption Against Pressure" and means the water absorption ratio under pressure of a particulate water absorbent. Specifically, AAP refers to the water absorption ratio (unit: g/g) obtained after swelling 0.9 g of a particulate water absorbent in a large excess of a 0.9 mass% aqueous solution of sodium chloride for one hour under a load of 2.06 kPa (21 g/cm$^2$, 0.3 psi). Furthermore, in ERT442.2-02, it is described as Absorption Under Pressure (AUP); however, the terms substantially have the same contents.

AAP2.1kPa of the second particulate water absorbent in the water-absorbing sheet according to an embodiment of the second aspect of the description is preferably 18 to 40 g/g, more preferably 23 to 33 g/g, even more preferably 24 to 32 g/g, and still more preferably 25 to 29 g/g. By being such an embodiment, excellent results are obtained in the evaluation of the specific return amount.

The AAP2.1kPa of the first particulate water absorbent in the water-absorbing sheet according to an embodiment of the second aspect of the description is preferably 18 to 40 g/g, more preferably 23 to 33 g/g, and even more preferably 24 to 32 g/g. By being such an embodiment, excellent results are obtained in the evaluation of the specific return amount.

The lower limit of the AAP2.1kPa of the particulate water absorbent (AAP2.1kPa of a mixture of the first particulate water absorbent and the second particulate water absorbent) in the water-absorbing sheet according to an embodiment of the second aspect of the description is preferably 18 g/g or more, more preferably 20 g/g or more, and even more preferably 25 g/g or more. By being such an embodiment, excellent results are obtained in the evaluation of the specific return amount. The upper limit is preferably 40 g/g or less, more preferably 38 g/g or less, and even more preferably 35 g/g or less.

"GPR"

Gel permeation rate (Gel Permeation Rate: GPR)

According to the present specification, the "liquid passability" of a particulate water absorbent refers to the flowability of a liquid that passes between swollen gel particles under a load. As an index of this, the gel permeation rate (GPR) is used. The gel permeation rate (GPR) of the particulate water absorbents (first particulate water absorbent and second particulate water absorbent) included in the water-absorbing sheet according to an embodiment of the second aspect of the description is carried out by the following procedure by referring to the saline flow conductivity (SFC) test described in US Patent No. 5849405 and by changing the measurement conditions.

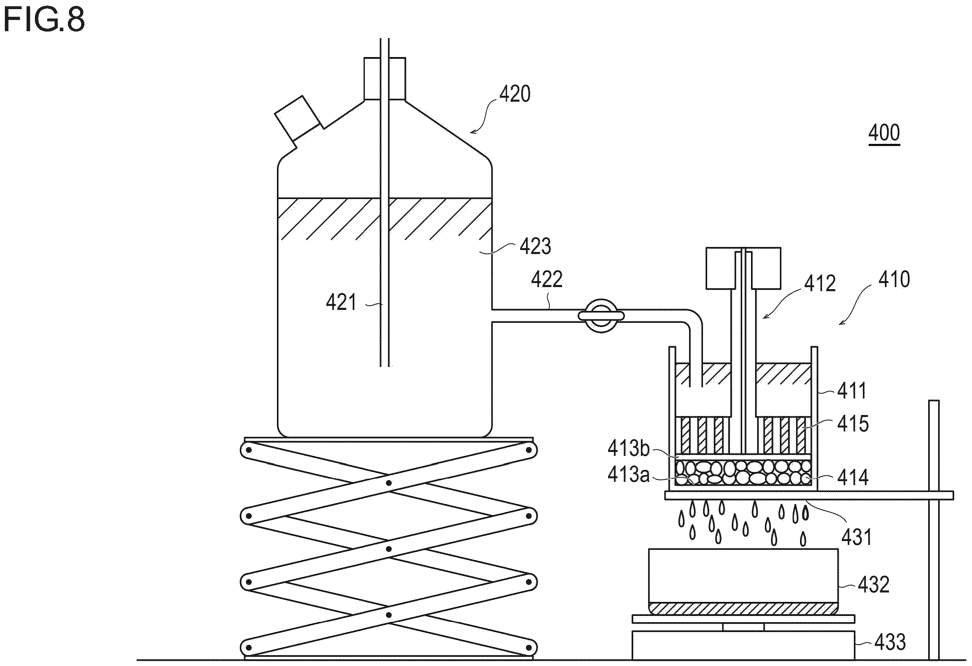

As an apparatus for measurement, an apparatus 400 illustrated in Fig. 8 is used. The apparatus 400 is broadly composed of a container 410 and a tank 420. In the container 410, a cell 411 (inner diameter 6 cm) is provided, and inside the cell 411, a swollen gel 414 (product obtained by causing a particulate water absorbent to absorb water) can be stored, while a liquid 423 can be introduced. Furthermore, by fitting a piston 412 into the cell 411, pressure can be exerted to the swollen gel 414. At the bottom face of the cell 411 and the bottom face of the piston 412, wire gauzes 413a and 413b (wire gauzes made of No. 400 stainless steel, sieve opening 38 μm) are stretched so that the swollen gel 414 (and a particulate water absorbent) cannot pass therethrough. Here, as the liquid 423, a 0.90 mass% aqueous solution of sodium chloride is used. The tank 420 has the liquid 423 accumulated inside . The liquid 423 is introduced into the cell 411 through an L-shaped tube with a cock 422. Furthermore, a glass tube 421 is inserted into the tank 420, and the interior of the glass tube 421 is filled with air. Thereby, the lower end of the glass tube 421 and the liquid surface inside the cell 411 can be made even. That is, so long as the liquid surface of the liquid 423 inside the tank 420 remains upper to the lower end of the glass tube 421, it is possible to maintain the liquid surface inside the cell 411 at a constant level. In the measurement of this time, the difference in elevation between the lower liquid surface of the liquid 423 (that is, lower end of the glass tube 421) inside the tank 420 and the bottom face of the swollen gel 414 was adjusted to 4 cm. That is, according to the apparatus 400, the liquid 423 at a constant positive hydrostatic pressure can be introduced into the cell 411. Since the piston 412 has a hole 415 formed therein, the liquid 423 flows through the hole 415, also further flows through the swollen gel 414 layer, and flows out to the outside of the cell 411. The container 410 is mounted on a stainless steel wire gauze 431 that does not interrupt the passage of the liquid 423. Therefore, the liquid 423 that has flowed out from the cell 411 is finally collected in a capturing container 432. Then, the amount of the liquid 423 collected in the capturing container 432 can be weighed using a Roberval balance 433.

A specific method for measuring the gel permeation rate (GPR) is as follows. Meanwhile, the following operation is carried out at room temperature (20°C to 25°C).

> (1) A particulate water absorbent (0.900 g) is uniformly introduced into a cell 411.
> (2) The particulate water absorbent is caused to absorb a liquid (0.00 mass% to 0.90 mass% (0.9 mass% in the present application) aqueous solution of sodium chloride) with no load for 60 minutes, and a swollen gel 414 is obtained.
> (3) A piston is placed on the swollen gel 414, and the system is brought into a pressurized state at 0.3 psi (2.07 kPa) .
> (4) While the hydrostatic pressure is maintained at a constant value of 3,923 dyne/cm$^2$, a liquid 423 is introduced into the cell 411 and is caused to pass through the swollen gel 414 layer.
> (5) The amount of the liquid 423 that passes through the swollen gel 414 layer is recorded for 3 minutes at an interval of 5 seconds. That is, the flow rate of the liquid 423 that passes through the swollen gel 414 layer is measured. For the measurement, a Roberval balance 433 and a computer (not illustrated in the diagram) are used.
> (6) The flow rates after one minute to after three minutes from the initiation of the flow-through of the liquid 423 are averaged, and the gel permeation rate (GPR) [g/min] is calculated.

With regard to the water-absorbing sheet according to an embodiment of the second aspect of the description, the GPR of the first particulate water absorbent is preferably 5 g/min or more, more preferably 35 g/min or more, even more preferably 55 g/min or more, still more preferably 75 g/min or more, even more preferably 95 g/min or more, and still more preferably 112 g/min or more . By being such an embodiment, the liquid to be absorbed may be easily sent to the second particulate water absorbent after being introduced on the first base material side, and the second particulate water absorbent can be effectively utilized. Thus, excellent results are obtained in the evaluation of the specific return amount. With regard to the water-absorbing sheet according to an embodiment of the second aspect of the description, the upper limit of the GPR of the first particulate water absorbent is not particularly limited; however, from the viewpoint of preventing liquid leakage, the upper limit is 500 g/min or less, 400 g/min or less, or 300 g/min or less, and it also preferable that the upper limit is 120 g/min or less.

With regard to the water-absorbing sheet according to an embodiment of the second aspect of the description, the GPR of the second particulate water absorbent is preferably 1 g/min or more, more preferably 3 g/min or more, even more preferably 5 g/min or more, and still more preferably 10 g/min or more, and the GPR may be 35 g/min or more, or 45 g/min or more. By having such a lower limit, gel blocking is suppressed, diffusibility is increased, and a liquid can be easily absorbed by the absorbent body. With regard to the water-absorbing sheet according to an embodiment of the second aspect of the description, the upper limit of the GPR of the first particulate water absorbent is not particularly limited; however, from the viewpoint of preventing liquid leakage, the upper limit is 300 g/min or less, 200 g/min or less, or 100 g/min or less.

The GPR of the particulate water absorbent (GPR of a mixture of the first particulate water absorbent and the second particulate water absorbent) in the water-absorbing sheet according to an embodiment of the second aspect of the description is preferably 100 g/min or less, more preferably 98 g/min or less, and even more preferably 95 g/min or less. Furthermore, with regard to the water-absorbing sheet according to an embodiment of the second aspect of the description, the lower limit of the GPR of the particulate water absorbent is preferably 1 g/min or more, more preferably 3 g/min or more, even more preferably 5 g/min or more, still more preferably 10 g/min or more, even more preferably 30 g/min or more, still more preferably 50 g/min or more, and even more preferably 70 g/min or more. By being such an embodiment, liquid leakage can be suppressed, and thereby, excellent results can be obtained in the evaluation of the specific return amount. Furthermore, with regard to the water-absorbing sheet according to an embodiment of the second aspect of the description, the GPR of the particulate water absorbent is 70 g/min or more, and the permeability is 92% or higher.

"Degradable soluble content"

The upper limit of the particulate water absorbent in the water-absorbing sheet according to an embodiment of the second aspect of the description is not particularly limited; however, from the viewpoint of liquid leakage during long-term use, the upper limit is each independently preferably less than 50%, more preferably 25% or less, and even more preferably 15% or less. The lower limit is not particularly limited; however, from the viewpoint of liquid leakage during long-term use, for example, the lower limit is about 1% or more, 3% or more, and 5% or more. Incidentally, a method for measuring the degradable soluble content is as follows.

L-ascorbic acid is added to physiological saline that has been prepared in advance so as to obtain a concentration of 0.05% by mass, and a degradation test liquid is produced. Specifically, 0.50 g of L-ascorbic acid was dissolved in 999.5 g of physiological saline, and thereby a degradation test liquid was prepared. 25 ml of the degradation test liquid is added into a glass beaker container having a capacity of 250 ml, 1.0 g of a particulate water absorbent is added thereto, and thereby a swollen gel is formed. The container is tightly sealed by covering up with a plastic wrap, and the swollen gel is left to stand for 16 hours in an atmosphere at 37°C (manufactured by Kusumoto Chemicals, Ltd., ETAC trade name, HISPEC series, used HT320, set to 37°C, set to a wind velocity variator scale of 30) . After 16 hours, 175 ml of physiological saline and a cylindrical stirring bar having a length of 30 mm and a width of 8 mm are charged therein, the swollen gel is degraded and then stirred at 500 rpm for 10 minutes, and extraction from hydrated gel is performed.

20.0 g of a filtrate that is obtained by filtering this extract using a filter paper (Advantec Toyo Kaisha, Ltd., product name: (JIS P 3801, No.2), thickness 0.26 mm, retained particle size 5 pm) is weighed, 30 g of physiological saline is further added thereto, and the mixture is used as a measurement solution.

Regarding the measurement method, physiological saline is subjected to titration up to pH 10 using a 0.1 N aqueous solution of NaOH, subsequently the physiological saline is titrated to pH 2.7 using a 0.1 N aqueous solution of HCl, and blank titers ([bNaOH] ml, [bHCl] ml) are obtained. A similar titration operation is also carried out for the measurement solution, and thereby titers ([NaOH]ml, [HCl]ml) are determined. For example, in the case of a particulate water absorbent including known amounts of acrylic acid and sodium salt thereof, a soluble content in the particulate water absorbent can be calculated by the following calculation formula, based on the average molecular weight of the monomer and the titers obtained by the above-described operation:

Degradable soluble content (mass%) = 0.1 × (average molecular weight) × 200 × 100 × ([HCl] - [bHCl])/1000/1.0/20.0.
In the case of an unknown amount, the average molecular weight of the monomer is calculated using a neutralization ratio determined by titration.

"Surface tension"

Surface tension is the work (free energy) required to increase the surface area of a solid or a liquid, expressed in per unit area. The surface tension as used in the present application refers to the surface tension of an aqueous solution when a particulate water absorbent is dispersed in a 0.90 mass% aqueous solution of sodium chloride. Incidentally, the surface tension of a water absorbent is measured by the following procedure. That is, 50 ml of physiological saline that has been adjusted to 20°C is introduced into a sufficiently washed 100-ml beaker, and first, the surface tension of the physiological saline is measured using a surface tensiometer (K11 automatic surface tensiometer manufactured by Kruss GmbH). Next, into the beaker including the physiological saline after surface tension measurement, which has been adjusted to 20°C, a sufficiently washed stirring bar made of a fluororesin, 25 mm in length, and 0.5 g of a particulate water absorbent are charged, and the mixture is stirred for 4 minutes under the conditions of 500 rpm. After 4 minutes, after stirring is stopped and the hydrated particulate water absorbent settles, the surface tension of the supernatant is measured by performing a similar operation again. Incidentally, in the second aspect of the description, a plate method of using a platinum plate is employed, and the plate is sufficiently washed with deionized water before each measurement and is used after being heated with a gas burner and washed. With regard to the water-absorbing sheet according to an embodiment of the second aspect of the description, the surface tension of the particulate water absorbent is preferably, in the following order, 57 N/m or more, 65 mN/m or more, 66 mN/m or more, 67 mN/m or more, 69 mN/m or more, 70 mN/m or more, or 71 mN/m or more, and most preferably 72 mN/m or more. In the application of a particulate water absorbent to a water-absorbing sheet, the influence of surface tension is more easily exhibited than conventional paper diapers, and as the surface tension satisfies the above-described conditions, the return amount to the paper diaper can be reduced.

With regard to the water-absorbing sheet according to an embodiment of the second aspect of the description, the upper limit of the surface tension of the particulate water absorbent is not particularly limited; however, the upper limit is 73 mN/m or less.

"Particle shape"

It is preferable that the particulate water absorbent according to an embodiment of the second aspect of the description includes particles having an irregular crushed shape as the particle shape. Here, the irregular crushed shape is a crushed-shaped particle whose shape is not regular. Compared to spherical particles obtained by reverse phase suspension polymerization or gas phase polymerization, the irregular crushed shape allows easy fixation to the base material. The particulate water absorbent according to an embodiment of the second aspect of the description is preferably a pulverized product in aqueous solution polymerization. On the other hand, in a case in which the particulate water absorbent is not subjected to a pulverization process, representatively, spherical particles obtainable by reverse phase suspension polymerization, liquid droplet polymerization, by which monomers to be polymerized are sprayed and polymerized, or the like, or a granulated product of spherical particles do not have an irregular crushed shape. According to embodiments of the second aspect of the description, when the shape of the particulate water absorbent is an irregular crushed shape, shape retention of the water-absorbing sheet is easily achieved as compared to particles whose average degree of circularity is high (for example, spherical particles) . According to embodiments of the second aspect of the description, the average degree of circularity of the particulate water absorbent is preferably 0.70 or less, more preferably 0.60 or less, and even more preferably 0.55 or less.

The method for calculating the average degree of circularity is as follows. One hundred or more particles of a particulate water absorbent were randomly selected, various particles of the particulate water absorbent were imaged with an electron microscope (VE-9800 manufactured by KEYENCE CORPORATION) (magnification ratio 50 times), images of the particulate water absorbent were obtained, and the circumference and the area were calculated for each particle using an affiliated image analysis software. The degree of circularity of each particle is determined by the following formula:

```
[Mathematical Formula 5]
```

$$\text{Degree of circularity} = 4 \times \pi \times \text{area}/(\text{circumference})^2$$

and the average value of the values thus obtained is calculated as the average degree of circularity.

With regard to the water-absorbing sheet according to an embodiment of the second aspect of the description, the lower limit of the content of the particulate water absorbent per unit volume of the water-absorbing sheet is, in a preferable order, 50 mg/cm$^3$ or more, 51 mg/cm$^3$ or more, 52 mg/cm$^3$ or more, 53 mg/cm$^3$ or more, 54 mg/cm$^3$ or more, 55 mg/cm$^3$ or more, 57 mg/cm$^3$ or more, 59 mg/cm$^3$ or more, or 60 mg/cm$^3$ or more. With regard to the water-absorbing sheet according to an embodiment of the second aspect of the description, the upper limit of the content of the particulate water absorbent per unit volume of the water-absorbing sheet is also not particularly limited; however, the upper limit is practically 600 mg/cm$^3$ or less, preferably 500 mg/cm$^3$ or less, more preferably 400 mg/cm$^3$ or less, even more preferably 300 mg/cm$^3$ or less, and still more preferably 150 mg/cm$^3$ or less. By adjusting the content of the particulate water absorbent to the range described above, the water-absorbing sheet efficiently exhibits the effects of the second aspect of the description. The contents of the particulate water absorbents per unit volume of the water-absorbing sheet of the water-absorbing sheets used in Examples of the present application were 50 mg/cm$^3$ or more.

The method for producing a particulate water absorbent is not particularly limited as long as it is a method for producing a water absorbent having desired physical properties, and for example, a particulate water absorbent can be appropriately produced by referring to the publications described in the Examples and the like.

Furthermore, in the water-absorbing sheet according to an embodiment of the second aspect of the description, the content weight of the first particulate water absorbent with respect to the content weight of the second particulate water absorbent is preferably 1.0 or less, more preferably 0.5 or less, and even more preferably 0.4 or less. By making the content of the second particulate water absorbent to be equal to, and preferably larger than, the content of the first particulate water absorbent, the second particulate water absorbent that is considered to have a so-called tank-like operating function effectively functions. Thus, excellent results are obtained in the evaluation of the specific return amount.

An embodiment of the second aspect of the description is a water-absorbing sheet having a first base material, a second base material, and a water-absorbing layer positioned between the first base material and the second base material, wherein the first base material is a water-permeable sheet positioned on the side where a liquid to be absorbed is introduced, the water-absorbing layer has a particulate water absorbent and an intermediate sheet, the weight average particle size of the particulate water absorbent is 200 to 600 $\mu$m, and the nonwoven fabric permeability index (NPI) is 60 or higher. According to such an embodiment, a water-absorbing sheet that can meaningfully reduce the return amount even when introduction of a liquid occurs intermittently for several times (particularly, three or more times) under a situation with no load, and the amount of introduction of the liquid becomes large, can be provided.

According to an embodiment of the second aspect of the description, the lower limit of the nonwoven fabric permeability index (NPI) is not particularly limited; however, the lower limit is preferably 70 or more, more preferably 80 or more, and even more preferably 90 or more. By having such a lower limit, the intended effects of the second aspect of the description are efficiently provided. According to an embodiment of the second aspect of the description, the upper limit of the nonwoven fabric permeability index (NPI) is not particularly limited; however, the upper limit is preferably 120 or less, more preferably 110 or less, and even more preferably 100 or less.

Incidentally, the nonwoven fabric permeability index (NPI) described in the present specification is represented by the following formula:

$$-90 - 1.05 \times C - 39.3 \times B + 3.15 \times A - 0.289 \times D - 0.0472 \times (C - 52.1) \times (D - 369)$$
$$+ 3.62 \times (B - 3.48) \times (A - 142) - 0.0142 \times (A - 142) \times (D - 369). \qquad \text{[Mathematical Formula 6]}$$

Here, A represents the specific surface area (mm$^{-1}$) of an intermediate sheet (for example, nonwoven fabric) measured by X-ray CT; B represents the thickness (mm) of an intermediate sheet (for example, nonwoven fabric) ; C represents the basis weight (g/m$^2$) of an intermediate sheet (for example, nonwoven fabric) ; and D represents the average particle size ($\mu$m) (D50) of a particulate water absorbent.

Here, the specific surface area of an intermediate sheet was determined by analyzing an image captured by X-ray CT as described below using an analysis software program.

<Imaging by X-ray CT>

An intermediate sheet cut out into a square shape that measured 10 mm in length and 10 mm in width (thickness was as received) was subjected to measurement using a microfocus X-ray CT system, InspeXio SMX-100CT, manufactured by Shimadzu Corp. The measurement conditions are described below.

· Image transverse size (pixel): 512
· Image longitudinal size (pixel): 512
· X-ray tube voltage (kV): 40
· X-ray tube current ($\mu$A): 50
· Inch size (inch): 4.0
· X-ray filter: None
· SDD (distance between focal point of X-ray source and X-ray detector) (mm): 500
· SRD (distance between focal point of X-ray source and center of rotation of measurement sample) (mm): 40
· Scan mode 1: CBCT
· Scan mode 2: Normal scan
· Scan angle: Full scan
· Number of views: 1,200
· Average number: 5
· Smoothing: YZ

· Slice thickness (mm): 0.012
· Distance between slices (mm): 0.010
· Scaling number: 50
· BHC data: None
· Fine mode: Available
· FOV XY (maximum imaging region XY) (mm): 5.0
· FOV Z (maximum imaging region Z) (mm): 1.5
· Voxel size (mm/voxel): 0.010.

<Calculation of specific surface area>

The imaging data of X-ray CT was subjected to analysis by the following procedure using an analysis software program, TRI/3D-PRT-LRG,manufactured by RatocSystem Engineering Co., Ltd.

1. From the menu, Particle Measurement > 3D Particles > Particle Separation > Giant Particle Separation is selected. EV panel, BC panel, EVC panel, and Giant Particle Separation panel are displayed.
2. L-W is selected at the Binarize tab of the EVC panel, the L value is changed, and a circular-shaped measurement target region is selected. "Execute" is pressed, and this treatment is applied to all slice images (a cylindrical-shaped measurement region is selected as a whole) . "ROI OK" of the Giant Particle Separation panel is pressed.
3. L-W is selected at the Binarize tab of the EVC panel, the L value is set to 37,580, and thereby only fibers are selected. "Execute" is pressed. bD of the BC panel is selected, and "Save" is pressed.
4. Labeling tab is selected at the 3D tab of the EVC panel, "Volume" is selected, and MAX is executed (by this operation, 3D Label Count = 1 is displayed).
5. From the menu, Particle Measurement > Void in 3D Particles > Measurement After Separation is selected. Measurement After Separation panel is displayed. A check for the Removal of Edge Particles is removed, a check is inserted for the Surface Area Calculation of the measurement items, Void Calculation is selected, Binary 5ch is selected at the Calculation ROI Designation, "Register OK" is pressed, and the folder for saving data is selected. "Recent Registered Data Execution" is pressed, and computation processing is executed.
6. From the calculation results, the specific surface area was calculated by the following formula.

Specific surface area (mm$^{-1}$) = Total particle surface area (mm$^2$) / (total particle volume (mm$^3$)

 Incidentally, for the configuration of the calculation software, the expression "Particle" is used; however, the actual values are measurement results obtained with fibers, and there are no problems in the measurement and calculation.
According to an embodiment of the second aspect of the description, the water-absorbing sheet is such that the nonwoven fabric permeability index (NPI) is 60 or higher, and the CRC of the particulate water absorbent included in the water-absorbing layer in the water-absorbing sheet is preferably 34 g/g or more, and more preferably 36 g/g or more. Incidentally, the descriptions including the physical properties and the like concerning the particulate water absorbent (for example, CRC, AAP2.1kPa, GPR, weight average molecular weight, and surface tension) as described in the present specification can also be applied to the particulate water absorbent according to this embodiment, and these can be used as the ground for an amendment of the present embodiment.

[3. Method for producing water-absorbing sheet]

The method for producing a water-absorbing sheet according to an embodiment of the second aspect of the description includes at least one of: (1) a step of scattering a first particulate water absorbent on a first base material, (2) a step of scattering a second particulate water absorbent on a second base material, and (3) a step of scattering a first particulate water absorbent and/or a second particulate water absorbent on an intermediate sheet. As more specific examples of the production method, the following production methods of (a) to (d) may be mentioned.

(a) A first particulate water absorbent (and preferably an adhesive) is uniformly scattered on a first base material. An intermediate sheet is superposed thereon, and the assembly is pressure-bonded. Furthermore, on a surface of the intermediate sheet on the side that does not face the first particulate water absorbent, a second particulate water absorbent (and preferably an adhesive) is uniformly scattered. An intermediate sheet is superposed thereon, and (preferably under heated conditions in which a hot melt is melted, or in a state in which a hot melt is melting) the assembly is pressure-bonded.

(b) A second particulate water absorbent is uniformly scattered on an intermediate sheet. Furthermore, an adhesive is scattered on a second base material. Then, the surface of the intermediate sheet, on which the second particulate water absorbent has been scattered, and the surface of the second base material, on which the adhesive has been scattered, are pressure-bonded. Next, in the intermediate sheet after pressure-bonding, on the surface on the opposite side of the surface on which the second particulate water absorbent has been scattered, a first particulate water absorbent is uniformly scattered. Furthermore, an adhesive is scattered on the first base material. Then, the surface of the intermediate sheet, on which the first particulate water absorbent has been scattered, and the surface of the first base material, on which the adhesive has been scattered, are pressure-bonded. It is preferable that the above-described pressure-bonding is carried out under heated conditions in which a hot melt is melted, or in a state in which a hot melt is melting.

(c) An adhesive is scattered on a second base material. Next, a second particulate water absorbent is uniformly scattered thereon. Next, an intermediate sheet is placed thereon, and the assembly is pressure-bonded. Next, an adhesive is scattered on a surface of the intermediate sheet on the side that does not face the second particulate water absorbent. Next, a first particulate water absorbent is uniformly scattered thereon. Next, a first base material is placed thereon, and the assembly is pressure-bonded. It is preferable that the above-described pressure-bonding is carried out under heated conditions in which a hot melt is melted, or in a state in which a hot melt is melting.

(d) An adhesive is scattered on a second base material. Next, a second particulate water absorbent is uniformly scattered thereon. Next, an intermediate sheet is placed thereon, and the assembly is pressure-bonded. Next, on the surface of the intermediate sheet on the side that does not face the second particulate water absorbent, a first particulate water absorbent is uniformly scattered. Furthermore, an adhesive is scattered on a first base material. Then, the surface of the intermediate sheet, on which the first particulate water absorbent has been scattered, and the surface of the first base material, on which the adhesive has been scattered, are pressure-bonded. It is preferable that the above-described pressure-bonding is carried out under heated conditions in which a hot melt is melted, or in a state in which a hot melt is melting.

As a step other than those described above, for the purpose of improving the feel of the water-absorbing sheet to the touch and enhancing the liquid absorption performance, the water-absorbing sheet may be subjected to embossing processing. The embossing processing may be carried out simultaneously when the first base material and the second base material are pressure-bonded or may be carried out after sheet production.

In the method for producing a water-absorbing sheet according to an embodiment of the second aspect of the description, additives (adeodorant, fibers, an antibacterial agent, a gel stabilizer, and the like) may be appropriately incorporated. The amount of incorporation of the additives is preferably 0% to 50% by mass, and more preferably 0% to 10% by mass, with respect to the mass of the particulate water absorbent. In the above-described production method, a particulate water absorbent that has been mixed with additives in advance may be used, or additives may be added in the middle of the production process.

The dimension of the water-absorbing sheet to be produced can be appropriately designed. Usually, the transverse width is 10 cm to 10 m, and the length is several dozen meters (m) to several thousand meters (m) (for a continuous sheet or a roll form) . The water-absorbing sheet thus produced is used after being cut out according to the purpose (size of the absorbent body to be used).

In addition to the examples described above, the method for producing a water-absorbing sheet is disclosed in the following patent literatures: WO 2012/174026 A, WO 2013/078109 A, WO 2015/041784 A, WO 2011/117187 A, WO 2012/001117 A, WO 2012/024445 A, WO 2010/004894 A, WO 2010/004895 A, WO 2010/076857 A, WO 2010/082373 A, WO 2010/113754 A, WO 2010/143635 A, WO 2011/043256 A, WO 2011/086841 A, WO 2011/086842 A, WO 2011/086843 A, WO 2011/086844 A, WO 2011/117997 A, WO 2011/118409 A, WO 2011/136087 A, WO 2012/043546 A, WO 2013/099634 A, WO 2013/099635A, JP2010-115406A, JP2002-345883A, JPH6-315501 A, JP H6-190003 A, JP H6-190002 A, JP H6-190001 A, JP H2-252558 A, JP H2-252560 A, and JP H2-252561 A. The methods for producing a water-absorbing sheet disclosed in these literatures are also appropriately referred to.

With regard to the water-absorbing sheet according to an embodiment of the second aspect of the description, as a method of fixing base materials together or fixing a base material with a particulate water absorbent, (i) an adhesive may be used, and if necessary, pressure-bonding may be used, (ii) various binders dissolved or dispersed in water, a water-soluble polymer, or a solvent may be used, or (iii) base materials may be heat-sealed at the melting point of the material of the base materials themselves. Preferably, fixing is achieved using an adhesive.

The adhesive to be used may be a solution type; however, from the viewpoint of the effort to remove the solvent, the problem of remaining solvent, and the problem of productivity, a hot melt adhesive that exhibits high productivity and does not have a problem of residual solvent is preferred. In the second aspect of the description, the hot melt adhesive may be incorporated in advance at the surface of the base material or the particulate water absorbent, or separately, a hot melt adhesive may be used during the production process for a water-absorbing sheet. The form or melting point of the hot melt adhesive can be appropriately selected, and the hot melt adhesive may be in a particulate form, may be in a fibrous form, may be in a net form, may be in a film form, or may be in a liquid form melted by heating. The melting temperature or the softening point of the hot melt adhesive is preferably 50°C to 200°C, or 60°C to 180°C. In the case of using a particulate adhesive, a particulate adhesive in which the particle size is about 0.01 to 2 times, 0.02 to 1 time, or 0.05 to 0.5 times, the average particle size of the particulate water absorbent, is used.

Regarding the method of using a hot melt adhesive in the production of the water-absorbing sheet according to an embodiment of the second aspect of the description, the following example may be mentioned. A water-absorbing sheet can be produced by uniformly scattering a mixture of a particulate water absorbent and a hot melt adhesive on a base material (for example, a nonwoven fabric), laminating another sheet of base material thereon, and heating and pressure-bonding the laminate at near the melting temperature of the hot melt adhesive.

The hot melt adhesive to be used for the second aspect of the description can be appropriately selected; however, preferably, one or more kinds selected from an ethylene-vinyl acetate copolymer adhesive, a styrene-based elastomer adhesive, a polyolefin-based adhesive, a polyester-based adhesive, and the like can be appropriately used.

Specific examples include, as polyolefin-based adhesives, polyethylene, polypropylene, and atactic polypropylene; as styrene-based elastomer adhesives, a styrene-isoprene block copolymer (SIS), a styrene-butadiene block copolymer (SBS), a styrene-isobutylene block copolymer (SIBS), a styrene-ethylene-butylene-styrene block copolymer (SEES), a styrene-butadiene rubber (SBR), and the like; copolymerized polyolefins, and the like; as polyester-based adhesives, polyethylene terephthalate (PET), polybutylene terephthalate (PBT), copolymerized polyesters, and the like; as ethylene-vinyl acetate copolymer adhesives, an ethylene-vinyl acetate copolymer (EVA) adhesive; an ethylene-ethyl acrylate copolymer (EEA), an ethylene-butyl acrylate copolymer (EBA), and the like.

With regard to the water-absorbing sheet according to an embodiment of the second aspect of the description and/or a method for producing the same, it is preferable that the water-absorbing sheet includes an adhesive, and the adhesive is preferably a hot melt adhesive. The amount of use (content) of the adhesive (forexample, a hotmelt adhesive) is preferably more than 0 and 3.0 times, and more preferably 0.05 to 2.0 times, the total mass of the first particulate water absorbent and the second particulate water absorbent. When the content of the adhesive (particularly, a hot melt adhesive) is too large, not only it is disadvantageous in view of the cost and in view of the mass of the water-absorbing sheet (increase in the mass of a paper diaper), but also there is a possibility that the particulate water absorbent may be subjected to the regulation of swelling and lower the water absorption power of the water-absorbing sheet.

[4. Absorbent article]

The absorbent article according to an embodiment of the second aspect of the description has a structure in which the water-absorbing sheet described in [2] is interposed between a liquid-permeable sheet and a liquid-impermeable sheet. Therefore, according to the second aspect of the description, there is provided an absorbent article having the water-absorbing sheet interposed between a liquid-permeable sheet and a liquid-impermeable sheet, the liquid-permeable sheet being positioned on the first base material side, and the liquid-impermeable sheet being positioned on the second base material side. Specific examples of the absorbent article include a disposable diaper, an incontinence pad, a sanitary napkin, a pet sheet, a drip sheet for food, a water sealant for electric power cables, and the like. By having such a configuration, excellent results are obtained in the evaluation of the specific return amount.

Regarding the liquid-permeable sheet and the liquid-impermeable sheet, those known in the technical field of absorbent articles can be used without particular limitations. Furthermore, the absorbent article can be produced according to a known method.

[EXAMPLES]

The second aspect of the description will be described in more detail using the following Examples and Comparative Examples. However, the technical scope of the second aspect of the description is not intended to be limited to the following Examples only. Furthermore, in the following Examples, unless particularly stated otherwise, operations were carried out under the conditions of room temperature (25°C)/relative humidity of 40% to 50%RH.

<Production Example>

With reference to the Production Examples, Examples, and Comparative Examples described in the following patent literatures, particulate water absorbents (1) to (4) of polyacrylic acid (salt)-based resins were obtained by appropriately adjusting CRC by means of the amount of an internal crosslinking agent. The physical properties of the particulate water absorbents thus obtained are presented in Table 1.

WO 2014/034897 A
WO 2017/170605 A

WO 2016/204302 A
WO 2014/054656 A
WO 2015/152299 A
WO 2018/062539 A
WO 2012/043821 A.

[Production Example of acrylic acid]

Commercially available acrylic acid (acrylic acid dimer 2, 000 ppm, acetic acid 500 ppm, propionic acid 500 ppm, and p-methoxyphenol 200 ppm) was supplied to the column bottom of a high-boiling point impurities separating column having fifty weirless perforated plates and was distilled at a reflux ratio of 1. After removal of maleic acid, a dimer including acrylic acid (acrylic acid dimer), and the like, crystallization is further carried out, and thereby acrylic acid (acrylic acid dimer 20 ppm, acetic acid 50 ppm, propionic acid 50 ppm, furfural 1 ppm or less, and protoanemonin 1 ppm or less) was obtained. After performing further distillation, 50 ppm of p-methoxyphenol was added thereto.

[Method for producing aqueous solution of sodium acrylate]

1,390 g of the acrylic acid was neutralized at 20°C to 40°C using 48% caustic soda according to Example 9 of US Patent No. 5210298, and thereby a 100% neutralized aqueous solution of sodium acrylate at a concentration of 37% was obtained.

<Particulate water absorbent 1>

In 5,500 g (monomer concentration 39.0% by mass) of an aqueous solution of sodium acrylate having a neutralization ratio of 73 mol%, which was obtained by mixing the acrylic acid obtained in the above-described Production Example of acrylic acid, an aqueous solution of sodium acrylate obtained by the above-described method for producing an aqueous solution of sodium acrylate using the acrylic acid, and deionized water, 4.54 g of polyethylene glycol diacrylate (average number of added moles of ethylene oxide 9) was dissolved, and a reaction liquid was obtained. Next, the reaction liquid was supplied to a reactor formed by attaching a lid to a jacketed stainless steel double-arm type kneader having an internal volume of 10 L and having two sigma-type blades, the system was purged with nitrogen gas while maintaining the reaction liquid at 30°C, and dissolved oxygen in the reaction liquid was removed. Subsequently, while the reaction liquid was stirred, 31.65 g of a 10 mass% aqueous solution of sodium persulfate and 38.95 g of a 1 mass% aqueous solution of L-ascorbic acid were added thereto, and after approximately 1 minute, polymerization was initiated. Forty minutes after the initiation of polymerization, a hydrated gel-like polymer was taken out. The hydrated gel-like polymer thus obtained was finely divided into particles that measured about 2 to 4 mm. This finely divided hydrated gel-like polymer was spread on a 50-mesh (mesh size 300 pm) wire gauze and was subjected to hot air drying for 65 minutes at 175°C. Next, the dried product was pulverized using a roll mill, further classified with a wire gauze having a sieve opening of 600 pm, and compounded. Thereby, water-absorbing resin (1-1) having an irregular crushed shape with an average particle size of 380 μm was obtained.

Into 100 parts by mass of the water-absorbing resin (1-1) thus obtained, 3.83 parts by mass of an aqueous solution of a surface crosslinking agent including 0.03 parts by mass of ethylene glycol diglycidyl ether, 0.3 parts by mass of 1,4-butanediol, 0.5 parts by mass of propylene glycol, and 3.0 parts by mass of water was sprayed and mixed. The mixture was subjected to a heating treatment for 40 minutes at a heat medium temperature of 210°C using a paddle type mixing and heat-treating machine, and surface-crosslinked water-absorbing resin (1-2) was obtained. 1.0 part by mass of water was sprayed and mixed into 100 parts by mass of the surface-crosslinked water-absorbing resin (1-2) thus obtained, the mixture was cured in a sealed container for one hour at 60°C and then was passed through a sieve having a sieve opening of 710 pm. Thus, water-absorbing resin (1-3) was obtained. 0.3 parts by mass of Aerosil 200 (hydrophilic amorphous silica, manufactured by Nippon Aerosil Co., Ltd.) was added to the water-absorbing resin (1-3) and mixed, and thereby the water-absorbing resin thus obtained was produced as particulate water absorbent (1).

<Particulate water absorbent 2>

The particulate water absorbent 1 was classified and subjected to particle size compounding, and thereby particulate water absorbent (2) having an average particle size of 420 μm was obtained.

<Particulate water absorbent 3>

In 5,500 g (monomer concentration 33.0% by mass) of an aqueous solution of sodium acrylate having a neutralization ratio of 73 mol%, which was obtained by mixing the acrylic acid obtained in the above-described Production Example of acrylic acid, an aqueous solution of sodium acrylate obtained by the above-described method for producing an aqueous solution of sodium acrylate using the acrylic acid, and deionized water, 4.17 g of polyethylene glycol diacrylate (average number of added moles of ethylene oxide 9) was dissolved, and a reaction liquid was obtained. Next, the reaction liquid was supplied to a reactor formed by attaching a lid to a jacketed stainless steel double-arm type kneader having an internal volume of 10 L and having two sigma-type blades, the system was purged with nitrogen gas while maintaining the reaction liquid at 30°C, and dissolved oxygen in the reaction liquid was removed. Subsequently, while the reaction liquid was stirred, 26.78 g of a 10 mass% aqueous solution of sodium persulfate and 32.96 g of a 1 mass% aqueous solution of L-ascorbic acid were added thereto, and after approximately 1 minute, polymerization was initiated. Forty minutes after the initiation of polymerization, 181.5 g of a water-absorbing resin fine powder having a size of 150 pm or less was added thereto, the gel was cracked for 10 minutes by rotating the blades of the kneader at a high speed (130 rpm), and then a hydrated gel-like polymer was taken out. The hydrated gel-like polymer thus obtained was finely divided into particles that measured about 1 to 2 mm. This finely divided hydrated gel-like polymer was spread on a 50-mesh (mesh size 300 pm) wire gauze and was subjected to hot air drying for 65 minutes at 175°C. Next, the dried product was pulverized using a roll mill, further classified with a wire gauze having a sieve opening of 600 pm, andprepared. Thereby, water-absorbing resin (3-1) having an irregular crushed shape with an average particle size of 350 $\mu$m was obtained.

Into 100 parts by mass of the water-absorbing resin (3-1) thus obtained, 3.83 parts by mass of an aqueous solution of a surface crosslinking agent including 0.03 parts by mass of ethylene glycol diglycidyl ether, 0.3 parts by mass of 1,4-butanediol, 0.5 parts by mass of propylene glycol, and 3.0 parts by mass of water was sprayed and mixed. The mixture was subjected to a heating treatment for 40 minutes at a heat medium temperature of 195°C using a paddle type mixing and heat-treating machine, and surface-crosslinked water-absorbing resin (3-2) was obtained. 1.0 part by mass of water was sprayed and mixed into 100 parts by mass of the surface-crosslinked water-absorbing resin (3-2) thus obtained, the mixture was cured in a sealed container for one hour at 60°C and then was passed through a sieve having a sieve opening of 710 pm. Thus, water-absorbing resin (3-3) was obtained. 0.3 parts by mass of Aerosil 200 (hydrophilic amorphous silica, manufactured by Nippon Aerosil Co., Ltd.) was added to the water-absorbing resin (3-3) and mixed, and thereby the water-absorbing resin thus obtained was produced as particulate water absorbent (3).

<Particulate water absorbent 4>

The particulate water absorbent 3 was classified and subjected to particle size compounding, and thereby particulate water absorbent (4) having an average particle size of 420 $\mu$m was obtained.

<Particulate water absorbent 5>

A water-absorbing resin was taken out from commercially available disposable diaper urine collection pads for adult (LIFREE urine collection pad for The Free Without Worry Paper Pants for night use (manufactured by Unicharm Corp. , purchased in September 2018). At the time of extraction, only the water-absorbing resin was taken out so as not to be mixed with cotton-like pulp and the like. The extracted water-absorbing resin had a particle shape obtained by granulating spherical particles. This water-absorbing resin was designated as particulate water absorbent (5).

[Extraction of water-absorbing resin from commercially available disposable diaper 1]

A water-absorbing resin was taken out from commercially available disposable diapers (MOONY AIRFIT (L size, Lot No. 201512163072), manufactured by Unicharm Corp., purchased in May 2016). At the time of extraction, only the water-absorbing resin was taken out so as not to be mixed with cotton-like pulp and the like. The extracted water-absorbing resin had a particle shape obtained by granulating spherical particles. This water-absorbing resin was designated as particulate water absorbent (T1). The physical properties of the particulate water absorbent (T1) are presented in Table 1.

[EXAMPLES]

[Example 1]

On a surface of an air-through nonwoven fabric (1) made of olefins as a main component (corresponding to the intermediate sheet) having a thickness of 2.6 mm under no load, which had been cut into a size of 10 cm in length and 40 cm in width, 6.0 g (scattering amount: 150 g/m$^2$) of the particulate water absorbent (3) (corresponding to the second particulate water absorbent) was uniformly scattered.

Next, on a surface of a nonwoven fabric (B) (made of pulp fibers as a main component, thickness under no load: 0.7 mm, produced by an air-laid method, corresponding to the second base material, basis weight: 42 g/m$^2$) that had been cut into a size of 10 cm in length and 40 cm in width), 0.3 to 0.5 g of an adhesive including styrene-butadiene rubber (spray adhesive 77, manufactured by 3M Japan, Ltd.) was uniformly scattered (scattering amount: 7.5 to 12.5 g/m$^2$).

Next, the surface of the nonwoven fabric (1) where the particulate water absorbent had been scattered, and the surface of the nonwoven fabric (B) where the adhesive had been scattered, were superposed so as to be paired (so as to be in contact), and the assembly was pressurized and pressure-bonded.

Next, on the surface of the nonwoven fabric (1) on the side that did not face the particulate water absorbent, 6.0 g (scattering amount: 150 g/m$^2$) of the particulate water absorbent (1) (corresponding to the first particulate water absorbent) was uniformly scattered. Furthermore, on the resultant, a nonwoven fabric (A) (made of pulp fibers as a main component, thickness under no load: 0.7 mm, produced by an air-laid method, corresponding to the first base material, basis weight: 42 g/m$^2$) , on which 0.3 to 0.5 g of an adhesive including styrene-butadiene rubber (spray adhesive 77, manufactured by 3M Japan, Ltd.) had been uniformly scattered (scattering amount: 7.5 to 12.5 g/m$^2$), was superposed such that the surface of the nonwoven fabric (1) where the particulate water absorbent had been scattered, and the surface of the nonwoven fabric (A) where the adhesive had been scattered would be paired (so as to be in contact with each other), and the assembly was pressurized and pressure-bonded. In this manner, water-absorbing sheet (1) was obtained.

[Example 2]

With regard to Example 1, as shown in Table 2, a nonwoven fabrics (2) (made of olefins as a main component, air-through nonwoven fabric) was used instead of the nonwoven fabric (1), and thereby water-absorbing sheet (2) was obtained.

[Example 3]

With regard to Example 1, as shown in Table 2, a nonwoven fabric (3) (made of olefins as a main component, air-through nonwoven fabric) was used instead of the nonwoven fabric (1), and thereby water-absorbing sheet (3) was obtained.

[Example 4]

With regard to Example 1, as shown in Table 2, a nonwoven fabric (4) (made of olefins as a main component, air-through nonwoven fabric) was used instead of the nonwoven fabric (1), and thereby water-absorbing sheet (4) was obtained.

[Example 5]

With regard to Example 1, the particulate water absorbent (2) was used instead of the particulate water absorbent (1), the particulate absorbent (4) was used instead of the particulate water absorbent (3), and thereby water-absorbing sheet (5) was obtained.

[Example 6]

With regard to Example 1, the particulate absorbent (5) (containing olefins as a main component, air-through nonwoven fabric) was used instead of the particulate water absorbent (3), and thereby water-absorbing sheet (6) was obtained.

[Comparative Example 1]

With regard to Example 6, as shown in Table 2, a nonwoven fabric (5) was used instead of the nonwoven fabric (1), the particulate absorbent (T1) was used instead of the particulate water absorbent (5), and thereby comparative water-absorbing sheet (1) was obtained.

An evaluation (measurement of reversion amount) of absorbent bodies of the water-absorbing sheets thus obtained was carried out, and the results are presented in Table 3.

[Method for evaluating absorbent sheet, or the like]

<Reversion amount>

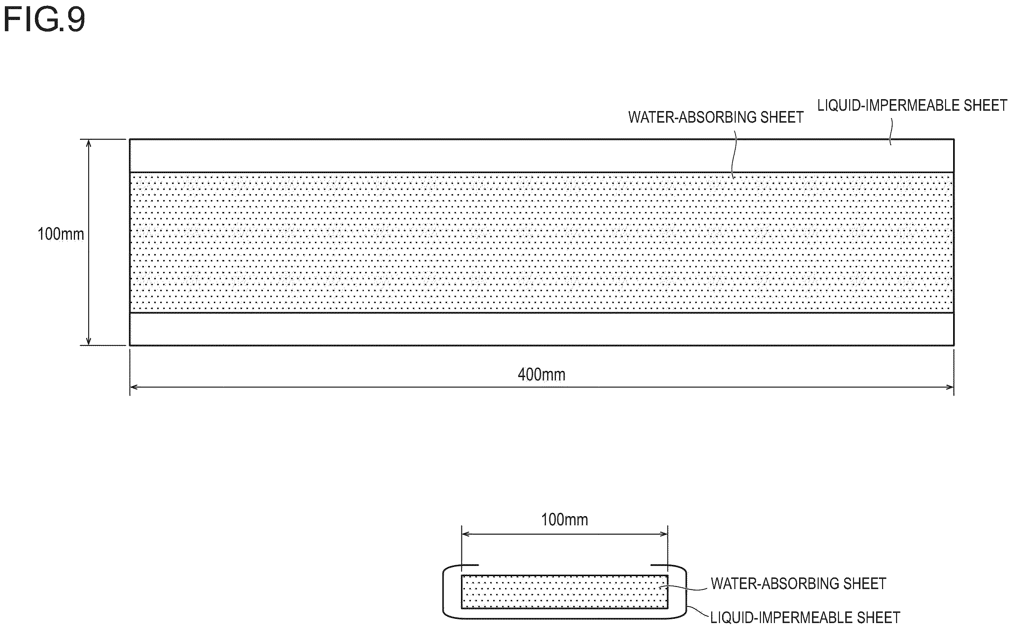

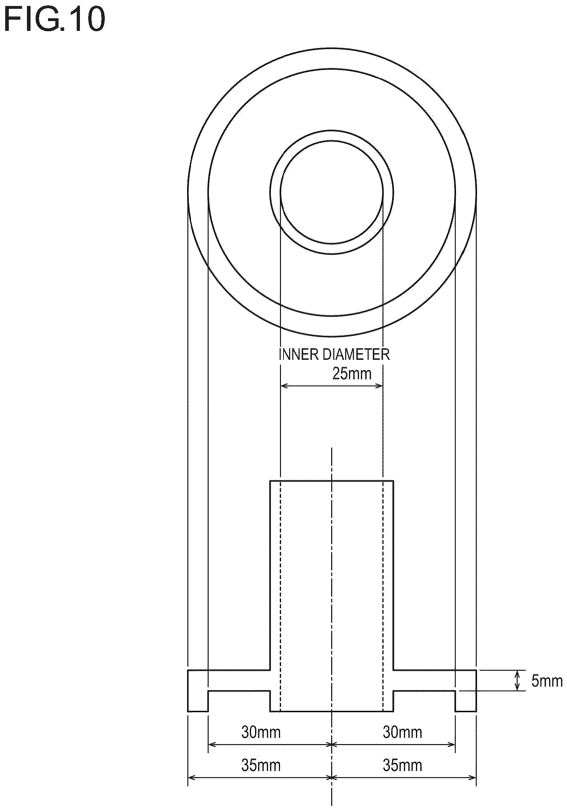

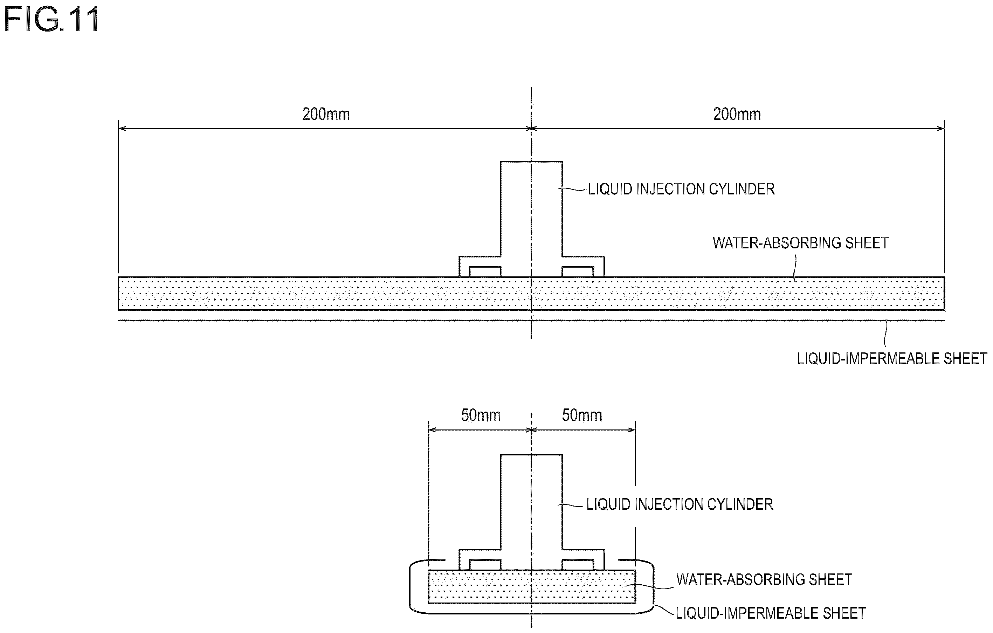

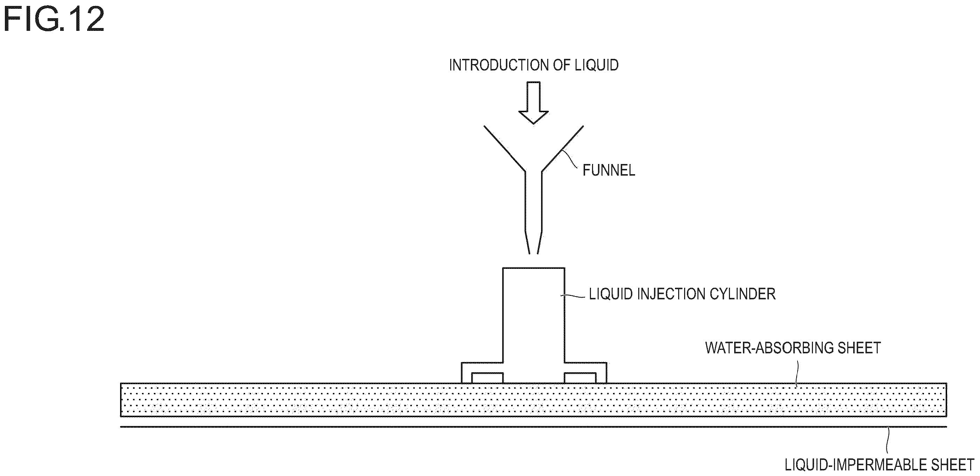

A water-absorbing sheet that had been cut into a size of 10 cm in length and 40 cm in width as illustrated in Fig. 9 was wrapped with a liquid-impermeable sheet having a size of 14 cm in length and 40 cm in width, such that an opening would be produced at the top. The water-absorbing sheet wrapped with the liquid-impermeable sheet was placed on a flat surface, and a liquid injection cylinder (Fig. 10) was placed thereon at the center of the water-absorbing sheet as illustrated in Fig. 11. In this state, 80 g of a 0.9 wt% aqueous solution of sodium chloride at 23°C was charged into the liquid injection cylinder using a funnel that enabled liquid charge at a flow rate of 7 ml/second (Fig. 12). Ten minutes after the liquid charge, 20 sheets of filter paper (Model No. 2, manufactured by Advantec MFS, Inc.; circular-shaped product having a diameter of 110 mm), whose weight had been measured in advance, were placed at the center of the water-absorbing sheet, a circular-shaped weight (1,200 g) having a diameter of 100 mm was further placed thereon, and this was retained for 1 minute. After 1 minute, the weight was removed, and the reversion amount first time (g) was measured from a weight increment of the filter paper. One minute after the removal of the weight, a similar operation (10 minutes after the charge of liquid, filter paper and a weight (1,200 g) were placed and retained for 1 minute. After 1 minute, the weight was removed, the measurement of the reversion amount was repeated, and the reversion amount second time (g) and the reversion amount third time (g) were measured. Meanwhile, as the reversion amount third time (g) is smaller, the sample is evaluated to be superior. The reversion amounts of the various water-absorbing sheets and the various comparative water-absorbing sheets are presented in Table 3.

<Method for measuring permeability of particulate absorbent>

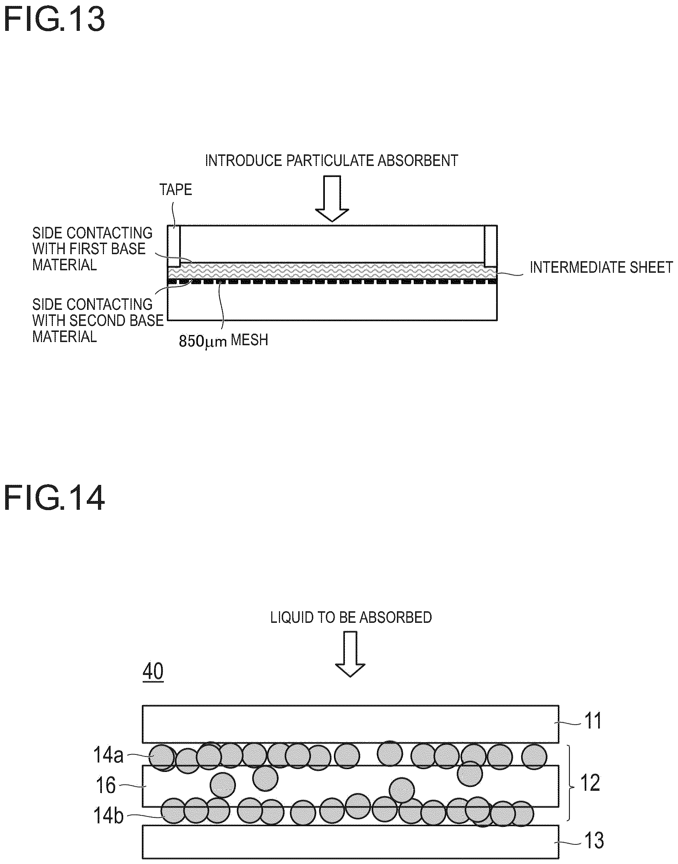

In a JIS standard sieve (The IIDA TESTING SIEVE: inner diameter 80 mm; JIS Z8801-1 (2000)) having a sieve opening of 850 $\mu$m, or a sieve corresponding to a JIS standard sieve, the nonwoven fabric (1) (corresponding to the intermediate sheet) that had been cut into a diameter of 80 mm was installed as illustrated in Fig. 13, and the circumference was fixed with a tape (an area having at least a diameter of 75 mm or more, which is permeable to particles, is secured). Furthermore, at this time, with regard to the form of the water-absorbing sheet, the nonwoven fabric (1) was installed inside the sieve such that the surface of the nonwoven fabric (1) that was in contact with the nonwoven fabric (A) (first base material) would face upward. For the nonwoven fabric (1), a nonwoven fabric taken out from a water-absorbing sheet by the method that will be described below may also be used. 10.0 g of a particulate water absorbent was charged onto the nonwoven fabric (1) in the sieve, and the particulate water absorbent was shaken for 5 minutes under the conditions of room temperature (20°C to 25°C) and a relative humidity of 50%RH, using a low-tap type sieve shaker (ES-65 type sieve shaker manufactured by Sieve Factory Iida Co., Ltd.; speed of rotation 230 rpm, number of mechanical shocks 130 rpm). For the particulate water absorbent, a particulate water absorbent taken out from a water-absorbing sheet by the method that will be described below may also be used. After shaking, the mass (W (g)) of the particulate water absorbent that had passed through the nonwoven fabric (1) and the JIS standard sieve was measured, and the permeability of the particulate absorbent was calculated by the following formula (i) . Meanwhile, measurement was performed three times, and the average value thereof was calculated. Incidentally, the shaking conditions were set in consideration of practical production conditions for a water-absorbing sheet, practical conveyance conditions for a manufactured water-absorbing sheet, and the like. Here, when the permeability of the particulate water absorbent to the intermediate sheet is 100%, it may also be considered that all of the particulate water absorbent would spill over in the water-absorbing sheet without being captured at the intermediate sheet. However, since these shaking conditions are set to be stronger than the cases of practical manufacture, conveyance, and the like but are designed not to be excessively strong, a situation such as described in the left side does not occur. By using an intermediate sheet having a predetermined permeability in the second aspect of the description, a large amount of a particulate water absorbent present in the water-absorbing sheet can be captured by the effect of practical shaking during practical manufacture and conveyance. [Mathematical Formula 7] Permeability of particulate absorbent (mass%) = (W/10.0) $\times$ 100                    Formula (i)

The permeabilities of the nonwoven fabric (2) through the nonwoven fabric (5) were also similarly measured.

[Method for extracting particulate absorbent from water-absorbing sheet and extracting nonwoven fabric used in intermediate sheet]

An upper nonwoven fabric (corresponding to the first base material) and a lower nonwoven fabric (corresponding to the second base material) were torn off from a water-absorbing sheet, and thereby a particulate absorbent and an intermediate sheet were taken out. The particulate absorbent stuck to the upper and lower nonwoven fabrics and the intermediate sheet was also all taken out. When the upper and lower nonwoven fabrics were torn off, they were torn off after cooling the water-absorbing sheet, and sufficiently weakening the adhesiveness of the adhesive (hot melt adhesive or spray adhesive) adhering the nonwoven fabrics and the particulate water absorbent. By going through this procedure, the nonwoven fabrics can be taken out without changing the fibers or structure thickness of the intermediate sheet, and it is possible to measure the permeability accurately. Regarding the method for cooling the water-absorbing sheet, various measures such as placing the water-absorbing sheet in a constant temperature chamber at -10°C or lower for a certain time period, blowing a cooling spray, or applying liquid nitrogen may be considered; however, the cooling method is not particularly limited so long as the method is carried out without changing the fibers, structure, and thickness of the intermediate sheet, and under the conditions in which the particulate water absorbent included in the water-absorbing sheet does not absorb moisture.

In a case in which the extracted particulate water absorbent has absorbed moisture, the percentage water content may be adjusted to 10% by mass or less, and preferably 5% $\pm$ 2% by mass by, for example, drying, and then the permeability and the various physical properties defined in the present application may be measured. The drying conditions for adjusting the percentage water content are not particularly limited so long as they are conditions in which decomposition or denaturation of the water-absorbing resin (particulate water absorbent) does not occur; however, drying under reduced pressure is desirable.

[Table 3]

|  | CRC [g/g] | AAP2.1 kPa [g/g] | GPR [g/min] | Average particle size [$\mu$m] | Surface tension [mN/m] |
|---|---|---|---|---|---|
| Particulate water absorbent (1) | 35 | 28 | 114 | 380 | 72 |
| Particulate water absorbent (2) | 37 | 26 | 131 | 420 | 72 |
| Particulate water absorbent (3) | 38 | 28 | 48 | 350 | 72 |
| Particulate water absorbent (4) | 40 | 25 | 65 | 420 | 72 |
| Particulate water absorbent (5) | 37 | 30 | 13 | 360 | 58 |
| Particulate water absorbent (T1) | 33 | 33 | 2 | 350 | 58 |

[Table 4]

|  | Basis weight (g/m$^2$) | Thickness (mm) | Porosity (%) |
|---|---|---|---|
| Nonwoven fabric (1) | 50.6 | 2.6 | 98.0 |
| Nonwoven fabric (2) | 51.0 | 4.0 | 98.7 |
| Nonwoven fabric (3) | 52.5 | 3.7 | 98.5 |
| Nonwoven fabric (4) | 43.2 | 4.6 | 99.0 |
| Nonwoven fabric (5) | 72 | 4.7 | 98.4 |

[Table 5]

| | First particulate water absorbent | Second particulate water absorbent | Intermediate sheet thickness (mm) | Content ratio of first/ second particulate water absorbents | Weight average particle size (μm) | CRC of particulate water absorbent (g/g) | AAP of particulate water absorbent (g/g) | GPR of particulate water absorbent (g/min) | Intermediate layer sheet | Nonwoven fabric permeability index (NPI) | Permeability of first and second particulate water absorbents % | Third time return amount (g) |
|---|---|---|---|---|---|---|---|---|---|---|---|---|
| Example 1 | Particulate water absorbent (1) | Particulate water absorbent (3) | 2.6 | 1 | 360 | 36.5 | 28 | 81 | Nonwoven fabric (1) | 96 | 96 | 4.0 |
| Example 2 | Particulate water absorbent (1) | Particulate water absorbent (3) | 4.0 | 1 | 360 | 36.5 | 28 | 81 | Nonwoven fabric (2) | 98 | 91 | 4.5 |
| Example 3 | Particulate water absorbent (1) | Particulate water absorbent (3) | 3.7 | 1 | 360 | 36.5 | 28 | 81 | Nonwoven fabric (3) | 94 | 88 | 5.3 |
| Example 4 | Particulate water absorbent (1) | Particulate water absorbent (3) | 4.6 | 1 | 360 | 36.5 | 28 | 81 | Nonwoven fabric (4) | 74 | 79 | 5.9 |
| Example 5 | Particulate water absorbent (2) | Particulate water absorbent (4) | 2.6 | 1 | 420 | 38.5 | 25 | 98 | Nonwoven fabric (1) | 92 | 90 | 6.1 |
| Example 6 | Particulate water absorbent (1) | Particulate water absorbent (5) | 2.6 | 1 | 370 | 36 | 29 | 64 | Nonwoven fabric (1) | 95 | 94 | 5.7 |
| Comparative Example 1 | Particulate water absorbent (1) | Particulate water absorbent (T1) | 4.7 | 1 | 360 | 34 | 31 | 58 | Nonwoven fabric (5) | 40 | 40 | 8.9 |

EP 3 834 789 B1

49

[Reference Signs List]

11: first base material
12: water-absorbing layer
13: second base material
14: particulate water absorbent
14a: first particulate water absorbent
14b: second particulate water absorbent
16: intermediate sheet
40: water-absorbing sheet
400: apparatus
410: container
411: cell
412: piston
413a, 413b: wire gauze
414: swollen gel (product obtained by causing a particulate water absorbent to absorb water)
415: hole
420: tank
421: glass tube
422: L-shaped tube with a cock glass tube
423: liquid
431: stainless steel wire gauze
432: capturing container
433: Roberval balance a weight average particle size of the particulate water

The above description is an explanation for the "second aspect of the present description".

In the following description, an explanation about the "third aspect of the present description" will be carried out.

<Third aspect of present description>

The above-described problems are solved by a water-absorbing sheet having a first base material, a second base material, and a water-absorbing layer positioned between the first base material and the second base material, wherein the first base material is a water-permeable sheet positioned on the side where a liquid to be absorbed is introduced, the water-absorbing layer has an intermediate sheet; a first particulate water absorbent localized on a side of a surface of the first base material, the surface being arranged to face the second base material; and a second particulate water absorbent localized on a side of a surface of the second base material, the surface being arranged to face the first base material, the permeability of a mixture of the first particulate water absorbent and the second particulate water absorbent to the intermediate sheet is lower than 60%, and the GPR of the second particulate water absorbent is 45.0 g/min or more.

[1. Definitions of terms]

[1-1. Water-absorbing sheet]

The "water-absorbing sheet" according to the third aspect of the description refers to a structure in which a water-absorbing resin (particulate water absorbent) and an intermediate sheet are supported between two or more sheets of long base materials. The water-absorbing sheet may use an adhesive, or may use a hot melt adhesive, for the adhesion between at least any of the base materials, the intermediate sheet, and the particulate water absorbent. The water-absorbing sheet may include other components (a fiber component, an antibacterial agent, a deodorant, and the like) in addition to the particulate water absorbent. Furthermore, the water-absorbing sheet may also include another sheet in addition to the two sheets of base material having the particulate water absorbent and the like interposed therebetween.

Usually, a water-absorbing sheet is in the form of a continuous sheet or a roll form obtained by winding the continuous sheet. When the water-absorbing sheet is used, a continuous sheet is cut out into an appropriate shape (rectangular shape or the like) and then is used as an absorbent body such as a disposable diaper. On the other hand, a conventional disposable diaper of a water-absorbing resin at a high concentration (for example, a disposable diaper in which the absorbent body is pulpless) uses an absorbent body that is mold-made for each sheet of the disposable diaper. Therefore, such an absorbent body differs from the water-absorbing sheet of the third aspect of the description in view of the technical properties.

[1-2. Water-absorbing resin]

The "water-absorbing resin" according to the present specification refers to a polymer gelling agent in which the water-swellability (CRC) defined in ERT441.2-02 is 5 g/g or more, and the water-soluble component (Ext) defined in ERT470.2-02 is 50% by mass or less.

The water-absorbing resin is preferably a hydrophilic crosslinked polymer by crosslink polymerizing an unsaturated monomer having a carboxyl group. The shape of the water-absorbing resin is a sheet form, a fibrous form, a film form, a particulate form, a gel form, or the like. The water-absorbing sheet according to an embodiment of the third aspect of the description uses a particulate water-absorbing resin.

The "water-absorbing resin" according to the present specification is not limited to an embodiment in which the total amount (100% by mass) is composed only of the water-absorbing resin. The water-absorbing resin may also be a water-absorbing resin composition including additives and the like. Furthermore, the "water-absorbing resin" according to the present specification is a concept that also includes intermediate bodies in the production process for the water-absorbing resin. For example, a hydrated gel-like crosslinked polymer after polymerization, a dried polymer after drying, a water-absorbing resin powder before surface crosslinking, and the like may also be described as the "water-absorbing resin".

As such, in the present specification, in addition to the water-absorbing resin itself, a water-absorbing resin composition and intermediate bodies may also be collectively referred to and described as "water-absorbing resin".

[1-3. Water absorbent, particulate water absorbent]

The "water absorbent" according to the present specification means an absorbent gelling agent for absorbing an aqueous liquid (liquid), the absorbent gelling agent including a water-absorbing resin as a main component. Here, the aqueous liquid (liquid) is not particularly limited so long as it is water as well as a liquid including water. Examples of the aqueous liquid that the water-absorbing sheet according to an embodiment of the third aspect of the description absorbs include urine, menstrual blood, sweat, and other body fluids.

The "particulate water absorbent" according to the present specification means a water absorbent in the form of particle (powdery form) (since the water-absorbing resin is included as a main component in the water absorbent, the particulate water absorbent corresponds to a water-absorbing resin in the form of particle). In the concept of the "particulate water absorbent", a single grain of the particulate water absorbent and an aggregate of a plurality of particulate water absorbents are all included. The term "particulate" according to the present specification means having the form of particle. Here, the "particle" refers to a relatively small divided body of a material and has a size of several Å to several mm (see "Particles", "McGraw-Hill Dictionary of Scientific and Technical Terms, 3rd Edition", edited by McGraw-Hill Dictionary of Scientific and Technical Terms Editorial Board, Nikkan Kogyo Shimbun, Ltd., 1996, p. 1929). Meanwhile, in the present specification, the "particulate water absorbent" may be described simply as "water absorbent". With Regard to the water-absorbing sheet according to an embodiment of the third aspect of the description, the weight average particle size of the particulate water absorbent is preferably 200 to 600 pm, more preferably 250 to 500 pm, and even more preferably 300 to 450 $\mu$m. Furthermore, with regard to the water-absorbing sheet of the third aspect of the description, it is preferable that 95% by mass or more of the entirety of the particulate water absorbent has a particle size of 850 um or less, it is more preferable that 98% by mass or more of the entirety of the particulate water absorbent has a particle size of 850 pm or less, and it is even more preferable that substantially 100% by mass of the entirety of the particulate water absorbent has a particle size of 850 pm or less. Incidentally, in the Examples of the present application, substantially 100% by mass of the entirety of the particulate water absorbent has a particle size of 850 pm or less. Here, in the present specification, the method for measuring the weight average particle size is calculated by a method similar to "(3) Mass-Average Particle Diameter (D50) and Logarithmic Standard Deviation ($\sigma\zeta$) of Particle Diameter Distribution" described in US Patent No. 7638570, based on the PSD obtained according to the method for measuring the "PSD" defined in ERT420.2-02.

The particulate water absorbent includes a water-absorbing resin as a polymer (alternatively, also referred to as particulate water-absorbing resin or water-absorbing resin particles) as a main component. The particulate water absorbent includes the water-absorbing resin as a polymer at a proportion of 60% to 100% by mass, preferably 70% to 100% by mass, more preferably 80% to 100% by mass, even more preferably 90% to 100% by mass, and particularly preferably 95% to 100% by mass. The remaining portion of the particulate water absorbent may optionally include water, additives (inorganic fine particles, polyvalent metal cations, and the like), and the like. Incidentally, the particulate water absorbents used in the Examples of the present application include about 95% to about 99% by mass of a water-absorbing resin.

That is, the upper limit of the water-absorbing resin in the particulate water absorbent is, for example, 100% by mass, 99% by mass, 97% by mass, 95% by mass, or 90% by mass. Preferably, the particulate water absorbent further includes, in addition to the water-absorbing resin, 0% to 10% by mass of components, particularly water, additives (inorganic fine particles, polyvalent metal cations), and the like.

Incidentally, a preferred percentage water content of the particulate water absorbent is 0.2% to 30% by mass. As described above, a water-absorbing resin composition in which components such as water and additives are integrated and/or mixed with the water-absorbing resin, is also included in the "particulate water absorbent".

Examples of the water-absorbing resin that serves as a main component of the particulate water absorbent include a polyacrylic acid (salt)-based resin, a polysulfonic acid (salt)-based resin, a maleic anhydride (salt)-based resin, a polyacrylamide-based resin, a polyvinylalcohol-based resin, a polyethylene oxide-based resin, a polyaspartic acid (salt)-based resin, a polyglutamic acid (salt)-based resin, a polyalginic acid (salt) -based resin, a starch-based resin, and a cellulose-based resin. Among these, preferably, a polyacrylic acid (salt)-based resin is used as the water-absorbing resin.

[1-4. Polyacrylic acid (salt)]

The "polyacrylic acid (salt)" according to the present specification refers to polyacrylic acid and/or a salt thereof . The polyacrylic acid (salt) is a polymer that includes a repeating unit of acrylic acid and/or a salt thereof (hereinafter, referred to as "acrylic acid (salt) ") as a main component and further includes a graft component as an optional component. The polyacrylic acid (salt) is obtained by polymerization of acrylic acid (salt), hydrolysis of polyacrylamide, polyacrylonitrile, or the like, and the like. Preferably, the polyacrylic acid (salt) is obtained by polymerization of acrylic acid (salt).

Here, the phrase "includes as a main component" implies that the amount of use of acrylic acid (salt) at the time of polymerizing polyacrylic acid (salt) is usually 50 mol% to 100 mol%, preferably 70 mol% to 100 mol%, more preferably 90 mol% to 100 mol%, and even more preferably substantially 100mol%, with respect to the entirety of the monomers (however, excluding an internal crosslinking agent) used for polymerization.

[1-5. EDANA and ERT]

"EDANA" is an acronym for the European Disposables and Nonwovens Associations. "ERT" is an acronym for methods for measuring a water-absorbing resin of the European standards (substantially, international standards) enacted by EDANA (EDANA Recommended Test Methods). In the present specification, unless particularly stated otherwise, the physical properties of the water-absorbing resin are measured according to ERT of the version of year 2002.

[1-6. Others]

According to the present specification, the expression "X to Y" representing a range means "X or more and Y or less" .

According to the present specification, unless particularly annotated otherwise, a unit of mass, "t (ton) ", means "Metric ton". The unit "ppm" means "ppm by mass". The "mass" and "weight", "parts by mass" and "parts by weight", "% by mass" and "% by weight", and "ppm by mass" and "ppm by weight" are respectively considered to have the same meanings.

According to the present specification, the term "acid (salt)" means "acid and/or a salt thereof". The term "(meth)acryl" means "acryl and/or methacryl".

According to the present specification, the volume unit "liter" may be described as "l" or "L". The unit "% by mass" may be described as "wt%". In a case in which measurement of trace components is carried out, the detection limit or less is described as N.D. (Non Detected).

[2. Water-absorbing sheet]

The water-absorbing sheet of the third aspect of the description is a water-absorbing sheet having a first base material, a second base material, and a water-absorbing layer positioned between the first base material and the second base material, wherein the first base material is a water-permeable sheet positioned on the side where a liquid to be absorbed is introduced, the water-absorbing layer has an intermediate sheet; a first particulate water absorbent localized on the side of a surface of the first base material, the surface being arranged to face the second base material; and a second particulate water absorbent localized on the side of a surface of the second base material, the surface being arranged to face the first base material, the permeability of a mixture of the first particulate water absorbent and the second particulate water absorbent (in the present specification, also referred to as "water absorbent mixture") to the intermediate sheet, the permeability being measured according to the method described in the Examples of the present application (in the present specification, also simply referred to as "permeability"), is less than 60%, and the GPR of the second particulate water absorbent is 45.0 g/min or more. By such a configuration, even if introduction of a liquid occurs intermittently for several times (particularly, three or more times) under a situation with no load, and the amount of introduction of the liquid becomes large, the return amount can be meaningfully reduced. Particularly, by such a configuration, excellent results about the measurement of the return amount under particular conditions in the Examples of the present application (in the present specification, also referred to as "evaluation of the specific return amount") can be obtained. That is, when there is intermittent introduction of a liquid for several times (particularly, three or more times), in a conventional configuration, the liquid amount is more than or equal to the set amount of absorption, and excessive "return" occurs. The inventors of the third aspect of the description have found that essentially, when the permeability to the intermediate sheet is 60% by mass or higher, excellent results are obtained in the evaluation of the specific return amount. Hereinafter, regarding the permeability, % by mass may also be referred to simply as %. The reason for this is that when a combination of a particulate water absorbent and an intermediate sheet capable of realizing such a permeability is used, the intermediate sheet can easily capture the particulate water absorbent present in the water-absorbing sheet. As the intermediate sheet captures a large amount of the particulate water absorbent, the particulate water absorbent diffuses in the water-absorbing sheet, and gaps between the particles of the particulate water absorbent present in the water-absorbing sheet are likely to become vacant. I fthe permeability is lower than 60%, the particulate water absorbent existing between the intermediate sheet and the base materials is not easily captured by the intermediate sheet, the probability of the particulate water absorbent existing between the intermediate sheet and the base materials increases, and the particulate water absorbent exists compactly between the intermediate sheet and the base material. Then, a gel blocking phenomenon occurs between the particles of the particulate water absorbent that have absorbed water, liquid diffusibility is decreased, and excessive "return amount" occurs. That is, when the permeability is 60% or higher, as the particulate water absorbent existing between the intermediate sheet and the base materials diffuses widely into the intermediate sheet, gaps between the particles of the particulate water absorbent are likely to become vacant, the performance of the particulate water absorbent can be exhibited at the maximum level, and even as a water-absorbing sheet, there is a technical effect in which the area capable of performing water absorption can be utilized more widely. However, the inventors of the third aspect of the description found that even when the permeability of the particulate water absorbent to the intermediate sheet is lower than 60%, the particulate water absorbent that is incorporated into the intermediate sheet is localized into a first particulate water absorbent and a second particulate water absorbent, and by adjusting the GPR of the second particulate water absorbent to 45.0 g/min or more, excellent results can be obtained in the "evaluation of the specific return amount" . Furthermore, when the permeability is lower than 60%, the intermediate sheet becomes soft, and a technical effect that the texture is improved and the user is likely to spend time comfortably, is also obtained. Here, it should be additionally remarked that a water-absorbing sheet or an absorbent article, which has been designed so as to suppress the return amount under general conditions, does not necessarily give excellent results in regard to the "evaluation of the specific return amount" of the present application. Furthermore, the water-absorbing sheet according to an embodiment of the third aspect of the description is suitable as, for example, an absorbent article (for example, a diaper) which an infant who has just learned to run and still has a small urinary bladder uses during aperiodof time for activelymoving around, such as daytime; however, of course, the usage form is not limited to this. A method of realizing the permeability to be lower than 60% will be described below; however, a method of controlling (when the constituent member of the intermediate sheet is fibers) the fiber diameter, or (when the intermediate sheet is a nonwoven fabric) superposing a plurality of sheets of a nonwoven fabric is considered suitable . That is, a permeability of lower than 60% can be realized by appropriately adjusting the properties of the member constituting the intermediate sheet, the surface state thereof, complicatedness of the network structure, the fiber diameter, the fused state between fibers, the basis weight, thickness, and the like. A person who attempts to carry out the third aspect of the description can adjust the permeability, for example, when an air-through nonwoven fabric is used as the intermediate sheet, by modifying the heat treatment conditions for the air-through nonwoven fabric, the fiber diameter, and the density. Even in a case in which an intermediate sheet of another kind is used as the intermediate sheet, a permeability of lower than 60% can be realized by appropriately modifying a factor that is involved in the adjustment of the permeability such as described above.

In the following description, embodiments of the third aspect of the description will be described with reference to the attached drawings. Incidentally, identical reference signs will be assigned to identical elements in the description of the drawings, and any overlapping explanations will not be repeated. Furthermore, the dimensional ratios of the drawings are exaggerated for the convenience of explanation and may be different from the actual ratios.

Fig. 14 is a schematic diagram illustrating a cross-section of a water-absorbing sheet 40 according to an embodiment of the third aspect of the description. A first base material 11 is positioned on the side where a liquid to be absorbed is introduced. That is, at least the first base material is disposed on the discharge side of the liquid (for example, the skin side in a paper diaper). A water-absorbing layer 12 is disposed between the first base material 11 and a second base material 13. Incidentally, in Fig. 14, the second base material 13 is positioned on the side opposite to the side where the liquid to be absorbed is introduced, with the water-absorbing layer 12 being interposed therebetween; however, for example, the area of the second base material 13 may be designed to be larger than that of the first base material 11 so that the second base material 13 may be folded up so as to wrap the first base material 11. By doing so, fall-off of particulate water absorbents 14a and 14b can be suppressed. The water-absorbing sheet 40 of Fig. 14 has an intermediate sheet 16; a first particulate water absorbent 14a localized on the side of a surface of the first base material 11, the surface being arranged to face the second base material; and a second particulate water absorbent 14b localized on the side of a surface of the second base material 13, the surface being arranged to face the first base material. This intermediate sheet 16 is such that the permeability of the water absorbent mixture is lower than 60%; however, since the water absorbent mixture is localized into the particulate water absorbent 14a and the particulate water absorbent 14a, and the GPR of the second particulate water absorbent 14b is 45.0 g/min or more, even if the particulate water absorbent 14a and the particulate water absorbent 14b are present not in large amounts within the intermediate sheet 16, and the particulate water absorbents are diffused and do not exist in the water-absorbing sheet, the intended effects of the third aspect of the description are provided.

As described above, with regard to the form of Fig. 14, the water-absorbing layer 12 has a particulate water absorbent 14a fixed to the first base material 11, and a particulate water absorbent 14b fixed to the second base material; however, portions of the particulate water absorbents 14a and 14b can be detached from the respective sheets. Therefore, the water-absorbing "layer" does not refer only to a continuous body such as a sheet but may have any form so long as it exists with a certain thickness between the first base material 11 and the second base material 13. Regarding the method of fixing particulate water absorbents to the respective base materials, for example, an adhesive may be used. A method for producing a water-absorbing sheet using an adhesive will be described in detail in [3.].

The water-absorbing sheet according to an embodiment of the third aspect of the description can be made thinner than the absorbent bodies used in the conventional type absorbent articles. In a case in which the water-absorbing sheet is used for a disposable diaper, the thickness is, for example, at 40%RH to 50%RH, preferably 15 mm or less, more preferably 10 mm or less, even more preferably 7 mm or less, particularly preferably 5 mm or less, and most preferably 4 mm or less. On the other hand, in view of the strength of the water-absorbing sheet and the diameters of the particulate water absorbents, the lower limit of the thickness is 0.2 mm or more, preferably 0.3 mm or more, and more preferably 0.5 mm or more. The thickness of the water-absorbing sheet used in the Examples of the present application was 3 mm to 5 mm under the above-described conditions.

Incidentally, the thickness of the water-absorbing sheet is measured using a Dial Thickness Gauge large-sized type (thickness measuring machine) (manufactured by Ozaki Manufacturing Co., Ltd., product No. : J-B, gauge head: anvils upper and lower $\phi$ 50 mm). Regarding the measurement sites, an absorbent body was divided into three equal parts in the longitudinal direction, and the respective center parts (a point positioned at an intersection point obtained by drawing diagonal lines from the corners of the absorbent body) were set as the measurement sites. For example, in the case of a water-absorbing sheet measuring 36 cm in the longitudinal direction and 10 cm in the width direction, the measurement positions are such that, with respect to the length of 36 cm in the longitudinal direction, three points such as a point 6 cm away from the left end in the longitudinal direction and 5 cm away from both ends in the width direction (designated as left) ; a point 18 cm away from the left end in the longitudinal direction and 5 cm away from both ends in the width direction (designated as center) ; and a point 30 cm away from the left end in the longitudinal direction and 5 cm away from both ends in the width direction (designated as right), correspond to the measurement sites. The measurement score is obtained by making measurement two times at each site, and the measured value of thickness is designated as the average value of six points in total. Regarding a specific procedure, a water-absorbing sheet is stuck flatly on a plate having a uniform thickness so that no crease or distortion is produced at the measurement sites of the water-absorbing sheet, and the plate is mounted on a lower gauge head of a thickness measuring machine . Next, an upper gauge head of the thickness measuring machine is approached to a position 2 to 3 mm high from the water-absorbing sheet, subsequently the handle is slowly released from the hands, and the thickness combining the water-absorbing sheet and the plate is measured. The thickness of the water-absorbing sheet is determined by formula: T1 = T2 - T0 (T0: thickness of plate (mm) , T1: thickness of water-absorbing sheet (mm), T2: thickness of water-absorbing sheet and plate (mm)).

In order to further impart liquid passability, diffusibility, flexibility, and the like to the water-absorbing sheet, the surface of the water-absorbing sheet may be appropriately subjected to embossing processing. The region that is subjected to embossing processing may be the entire face of the water-absorbing sheet surface or may be a portion thereof. By providing a continuously embossing-processed region in the longitudinal direction of the water-absorbing sheet, a liquid can be caused to diffuse easily in the longitudinal direction. Furthermore, the particulate water absorbents may be scattered over the entire surface of the water-absorbing sheet, or particulate water absorbent-absent regions may be provided in some parts. In the case of providing particulate water absorbent-absent regions, it is preferable to provide the absent regions in a channel form (striped shape) in the longitudinal direction of the water-absorbing sheet. As such, when embossing-processed regions and/or particulate water absorbent-absent regions are provided consecutively in the longitudinal direction, those regions accomplish the role as passages for allowing large quantities of liquid to flow through (liquid conveyance passages). The embossing-processed regions and/or the particulate water absorbent-absent regions may be provided in a linear shape, may be provided in a curved shape, or may be provided in a wavy shape.

In the following description, various members constituting the water-absorbing sheet will be described in detail.

[2-1. First base material, second base material, and intermediate sheet]

The first base material is a water-permeable sheet positioned on the side where the liquid to be absorbed is introduced. Incidentally, the liquid to be absorbed is not limited to water and may be urine, blood, sweat, feces, wastewater, moisture, ice, a mixture of water and an organic solvent and/or an inorganic solvent, rain water, underground water, or the like. The liquid to be absorbed is not particularly limited so long as it includes water. Preferred examples include urine, menstrual blood, sweat, and other body fluids.

The upper limit of the permeability of the water absorbent mixture to the intermediate sheet in the water-absorbing sheet according to an embodiment of the third aspect of the description is not particularly limited so long as the upper limit is lower than 60%; however, the upper limit may be 59% or lower, 57% or lower, 55% or lower, 53% or lower, 51% or lower, 49% or lower, 47% or lower, 45% or lower, or 43% or lower. The lower limit is preferably 10% or higher, 20% or higher, even more preferably 30% or higher, still more preferably 35% or higher, even more preferably higher than 40%, still more preferably 42% or higher, even more preferably 44% or higher, still more preferably 46% or higher, even more preferably 48% or higher, and even more preferably 50% or higher. By having such a lower limit, the capture ratio of the particulate water absorbent becomes high, and the intended effects of the third aspect of the description are easily exhibited.

The porosity of the water absorbent mixture to the intermediate sheet in the water-absorbing sheet according to an embodiment of the third aspect of the description is preferably 80% to 99.9%, more preferably 85% to 99.9%, even more preferably 90% to 99.9%, particularly preferably 98.0% (or 98.1%) to 99.9%, and most preferably 98.2% (98.3% or 98.4%) to 99.5% (or 99.0%). Incidentally, as described above, since a permeability of lower than 60% can be realized by means of the properties of the member constituting the intermediate sheet, the surface state thereof, the complicatedness of the network structure, the fiber diameter, the fused state between fibers, the basis weight, thickness, and the like, there is no direct correlation between porosity and permeability.

The materials of the first base material, the second base material, and the intermediate sheet used for the water-absorbing sheet according to an embodiment of the third aspect of the description are each independently preferably a nonwoven fabric. The raw material of the nonwoven fabric is not particularly limited; however, from the viewpoints of liquid penetrability, flexibility, and the strength of the water-absorbing sheet, polyolefin fibers (polyethylene (PE), polypropylene (PP), and the like), polyester fibers (polyethylene terephthalate (PET), polytrimethylene terephthalate (PTT), polyethylene naphthalate (PEN), and the like), polyamide fibers (nylon and the like), rayon fibers, pulp (cellulose) fibers, and the like are preferred. Furthermore, nonwoven fabrics of other synthetic fibers, and nonwoven fabrics produced by mixing synthetic fibers with cotton, silk, hemp, pulp (cellulose) fibers and the like are also similarly preferred. The nonwoven fabric described above may be a nonwoven fabric including only one kind of the above-mentioned fibers or a nonwoven fabric combining two or more kinds of fibers. It is preferable that the nonwoven fabrics used for the first base material and the second base material are produced by an air-laid method. Furthermore, pulp (cellulose) fibers are preferred.

With regard to the water-absorbing sheet according to an embodiment of the third aspect of the description, the nonwoven fabric used for the intermediate sheet is preferably an air-through nonwoven fabric. Furthermore, it is preferable that the nonwoven fabric used for the intermediate sheet is bulky, and specifically, the thickness under no load is preferably 1.3 mm or more, more preferably 1.5 mm or more, even more preferably 1.7 mm or more, still more preferably 1.9 mm or more, even more preferably 2.1 mm or more, still more preferably 2.3 mm or more, even more preferably 2.5 mm or more, still more preferably 3 mm or more, even more preferably 5 mm or more, and still more preferably 7 mm or more. The nonwoven fabric described above may include a small amount of pulp fibers to the extent that does not increase the thickness of the water-absorbing sheet. The upper limit of the thickness of the nonwoven fabric used for the intermediate sheet is also not particularly limited; however, for example, the thickness under no load is preferably about 14 mm or less, more preferably 13 mm or less, even more preferably 12 mm or less, still more preferably 11 mm or less, and even more preferably 10 mm or less.

With regard to the water-absorbing sheet according to an embodiment of the third aspect of the description, the basis weight of the intermediate sheet is preferably 47 $g/m^2$ or more, more preferably 50 $g/m^2$ or more, even more preferably 55 $g/m^2$ or more, still more preferably 61 $g/m^2$ or more, even more preferably 70 $g/m^2$ or more, still more preferably 80 $g/m^2$ or more, even more preferably 90 $g/m^2$ or more, and still more preferably 95 $g/m^2$ or more, per sheet of the intermediate sheet. The upper limit is not particularly limited; however, the upper limit is preferably 200 $g/m^2$ or less, more preferably 150 $g/m^2$ or less, and even more preferably 120 $g/m^2$ or less.

With regard to the water-absorbing sheet according to an embodiment of the third aspect of the description, the thickness of the intermediate sheet is thicker than the thickness of either the first base material or the second base material. More suitably, the thickness of the intermediate sheet is thicker than the thickness of the both the first base material and the second base material. Such an embodiment contributes to water retention in the early stage of liquid absorption and conduces to the reduction of leakage, the first base material and the second base material can be designed to be thin, and the overall thickness of the water-absorbing sheet can be made thinner. The ratio of the thickness of the intermediate sheet with respect to the arithmetic mean of the thicknesses of the first base material and the second base material is preferably 1.5 to 100, more preferably 2 to 80, even more preferably 3 to 50, and still more preferably 3.2 to 15.

As mentioned above, the nonwoven fabric used for the water-absorbing sheet according to an embodiment of the third aspect of the description is preferably a hydrophilic nonwoven fabric in order to increase the water permeability; however, the nonwoven fabric or the fibers as the material of the nonwoven fabric may be hydrophilized using a hydrophilizing agent (surfactant or the like).

Regarding examples of the hydrophilizing agent include anionic surfactants (aliphatic sulfonic acid salts, higher alcohol sulfuric acid ester salts, and the like), cationic surfactants (quaternary ammonium salts and the like), nonionic surfactants (polyethylene glycol fatty acid esters, polyglycerin fatty acid esters, sorbitan fatty acid esters, and the like), silicone-based surfactants (polyoxyalkylene-modified silicone, and the like), stain release agents including polyester-based, polyamide-based, acrylic, and urethane-based resins, and the like are used.

The first base material used for the water-absorbing sheet according to an embodiment of the third aspect of the description is a water-permeable sheet because it is located on the side where the liquid to be absorbed is introduced; however, it is preferable that the second base material and the intermediate sheet are also water-permeable sheets having water permeability, and the two may be of the same type or may be of different types. With regard to the water-permeability for the water-permeable sheet, the water permeability coefficient (JIS A1218:2009) is preferably $1 \times 10^{-5}$ cm/sec or higher. This water permeability coefficient is more preferably $1 \times 10^{-4}$ cm/sec or higher, even more preferably $1 \times 10^{-3}$ cm/sec or higher, still more preferably $1 \times 10^{-2}$ cm/sec or higher, and even more preferably $1 \times 10^{-1}$ cm/sec or higher. The water-permeable sheet used for the water-absorbing sheet according to an embodiment of the third aspect of the description is preferably a hydrophilic nonwoven fabric. By being a hydrophilic nonwoven fabric, the intended effects of the third aspect of the description can be efficiently provided.

It is more desirable as the thicknesses of the first base material and the second base material are thinner to the extent of having strength as a water-absorbing sheet, and for each sheet of the base material, the thicknesses are each independently appropriately selected in the ranges of 0.01 to 2 mm, 0.02 to 1 mm, 0.03 to 0.9 mm, and 0.05 to 0.8 mm. The basis weights of the first base material and the second base material are each independently preferably 5 to 300 g/m$^2$, more preferably 8 to 200 g/m$^2$, even more preferably 10 to 100 g/m$^2$, and still more preferably 11 to 50 g/m$^2$, per sheet of the base material.

[2-2. Water-absorbing layer]

The water-absorbing layer in the water-absorbing sheet according to an embodiment of the third aspect of the description has an intermediate sheet; a first particulate water absorbent localized on the side of a surface of the first base material, the surface being arranged to face the second base material; and a second particulate water absorbent localized on the side of a surface of the second base material, the surface being arranged to face the first base material. As the water-absorbing layer has an intermediate sheet, there is also an advantage that the first particulate water absorbent and the second particulate water absorbent are likely to be localized more efficiently on the first base material side and the second base material side, respectively. Furthermore, when the intermediate sheet constitutes a layer such as an air layer, there is an advantage that even if there is reversion from the water-absorbing sheet, the skin may not feel the reversion. Since the explanation about the intermediate sheet is as described above, further explanation will not be repeated here. Incidentally, localization of the first particulate water absorbent and the second particulate water absorbent can also be realized by, for example, appropriately utilizing an adhesive, as described in the following section [3.].

(Particulate water absorbent)

The water-absorbing layer in the water-absorbing sheet according to an embodiment of the third aspect of the description includes a first particulate water absorbent and a second particulate water absorbent. Incidentally, unless particularly stated otherwise, when it is simply described as particulate water absorbent, this means a mixture of a first particulate water absorbent and a second particulate water absorbent, or at least one of a first particulate water absorbent and a second particulate water absorbent. Furthermore, in a case in which the first particulate water absorbent and/or the second particulate water absorbent are mixtures of a plurality of kinds of particulate water absorbents, the following description is an explanation of the physical properties of these mixtures.

"CRC" (ERT441.2-02)

The term "CRC" is an acronym for "Centrifuge Retention Capacity" and means a water absorption ratio with no load (may also be referred to as "water absorption ratio") of a particulate water absorbent. Specifically, CRC refers to a water absorption ratio (unit: g/g) obtained by introducing 0.2 g of a particulate water absorbent into a nonwoven fabric bag, subsequently immersing the bag in a large excess of a 0.9 mass% aqueous solution of sodium chloride for 30 minutes so as to cause the particulate water absorbent to freely swell, and then dehydrating the particulate water absorbent in a centrifuge (250 G).

The CRC of the second particulate water absorbent in the water-absorbing sheet according to an embodiment of the third aspect of the description is preferably 30 to 50 g/g, more preferably 31 to 45 g/g, and even more preferably 32 to 42 g/g. By being such an embodiment, excellent results are obtained in the evaluation of the specific return amount.

The CRC of the first particulate water absorbent in the water-absorbing sheet according to an embodiment of the third aspect of the description is preferably 30 to 50 g/g, more preferably 31 to 48 g/g, and even more preferably 32 to 45 g/g. By being such an embodiment, excellent results are obtained in the evaluation of the specific return amount.

The lower limit of the CRC of the particulate water absorbent (CRC of a mixture of the first particulate water absorbent and the second particulate water absorbent) in the water-absorbing sheet according to an embodiment of the third aspect of the description is preferably 30 g/g or more, more preferably 32 g/g or more, and even more preferably 33 g/g or more. By being such an embodiment, excellent results are obtained in the evaluation of the specific return amount. The upper limit is preferably 48 g/g or less, and more preferably 45 g/g or less.

"AAP" (ERT442.2-02)

"AAP" is an acronym for "Absorption Against Pressure" and means the water absorption ratio under pressure of a particulate water absorbent. Specifically, AAP refers to the water absorption ratio (unit: g/g) obtained after swelling 0.9 g of a particulate water absorbent in a large excess of a 0.9 mass% aqueous solution of sodium chloride for one hour under a load of 2.06 kPa (21 g/cm$^2$, 0.3 psi). Furthermore, in ERT442.2-02, it is described as Absorption Under Pressure (AUP); however, the terms substantially have the same contents.

AAP2.1kPa of the second particulate water absorbent in the water-absorbing sheet according to an embodiment of the third aspect of the description is preferably 18 to 40 g/g, more preferably 23 to 34 g/g, and even more preferably 24 to 33 g/g. By being such an embodiment, excellent results are obtained in the evaluation of the specific return amount.

The AAP2.1kPa of the first particulate water absorbent in the water-absorbing sheet according to an embodiment of the third aspect of the description is preferably 18 to 40 g/g, more preferably 23 to 36 g/g, and even more preferably 24 to 34 g/g. By being such an embodiment, excellent results are obtained in the evaluation of the specific return amount.

"Water absorption time determined according to Vortex method"

The term "Vortex" according to the third aspect of the description is the water absorption time determined according to the "Testing method for water absorption rate of super absorbent polymers" described in JIS K7224-1996. Regarding a specific measurement method, 0.02 parts by weight of Edible Blue No. 1, which is a food additive, is added to 1,000 parts by weight of a 0.90 wt% aqueous solution of sodium chloride that has been prepared in advance, and the mixture is adjusted to a liquid temperature of 30°C. 50 ml of the 0.90 wt% aqueous solution of sodium chloride that has been colored blue is weighed in a 100-ml beaker, and in the middle of stirring the aqueous solution at 600 rpm with a cylindrical stirring bar having a length of 40 mm and a width of 8 mm, 2.00 g of a particulate water absorbent is charged thereinto. The water absorption time (seconds) is measured. Regarding the termination point, according to the criteria described in the "Manual for testing method for water absorption rate of super absorbent polymers" described in JIS K7224-year 1996, the time taken by the water absorbent to absorb physiological saline until the test liquid covers the stirrer tip is measured as the water absorption time (seconds).

With regard to the water-absorbing sheet according to an embodiment of the third aspect of the description, the water absorption time of the second particulate water absorbent as determined according to the vortex method is preferably 10 seconds or more, more preferably 20 seconds or more, even more preferably 25 seconds or more, and still more preferably 28 seconds or more. With regard to the water-absorbing sheet according to an embodiment of the third aspect of the description, the upper limit of the water absorption time of the second particulate water absorbent as determined according to the vortex method is not particularly limited; however, from the viewpoint of preventing liquid leakage, for example, the upper limit may be 100 seconds or less, 80 seconds or less, or 60 seconds or less.

With regard to the water-absorbing sheet according to an embodiment of the third aspect of the description, the water absorption time of the first particulate water absorbent as determined according to the vortex method is preferably 10 seconds or more, more preferably 20 seconds or more, even more preferably 25 seconds or more, and still more preferably 28 seconds or more. As such, by regulating the water absorption time of the first particulate water absorbent as determined according to the vortex method to be meaningfully long, the diffusibility in the planar direction can be increased, and the water absorption volume possessed by the first particulate water absorbent can be effectively utilized. Thus, excellent results are obtained in the evaluation of the specific return amount. Furthermore, there is a technical effect that the liquid to be absorbed is easily sent to the second particulate water absorbent after being introduced on the first base material side, and thus the second particulate water absorbent can be effectively utilized. With regard to the water-absorbing sheet according to an embodiment of the third aspect of the description, the upper limit of the water absorption time of the first particulate water absorbent as determined according to the vortex method is not particularly limited; however, from the viewpoint of preventing liquid leakage, for example, the upper limit may be 100 seconds or less, 80 seconds or less, or 60 seconds or less. The water absorption time determined according to the vortex method can be controlled by the specific surface area and the like of the water-absorbing resin particles. When the specific surface area of the water-absorbing resin particles becomes larger, the water absorption time tends to be shortened.

"GPR"

Gel permeation rate (Gel Permeation Rate: GPR)

According to the present specification, the "liquid passability" of a particulate water absorbent refers to the flowability of a liquid that passes between swollen gel particles under a load. As an index of this, the gel permeation rate (GPR) is used. The gel permeation rate (GPR) of the particulate water absorbents (first particulate water absorbent and second particulate water absorbent) included in the water-absorbing sheet according to an embodiment of the third aspect of the description is carried out by the following procedure by referring to the saline flow conductivity (SFC) test described in US Patent No. 5849405 and by changing the measurement conditions.

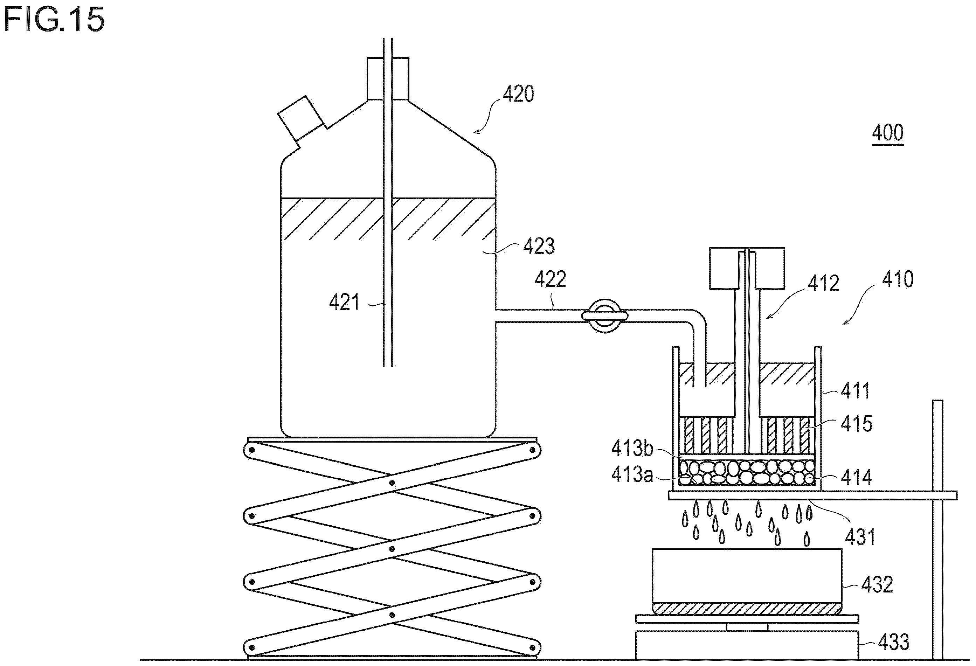

As an apparatus for measurement, an apparatus 400 illustrated in Fig. 15 is used. The apparatus 400 is broadly composed of a container 410 and a tank 420. In the container 410, a cell 411 (inner diameter 6 cm) is provided, and inside the cell 411, a swollen gel 414 (product obtained by causing a particulate water absorbent to absorb water) can be stored, while a liquid 423 can be introduced. Furthermore, by fitting a piston 412 into the cell 411, pressure can be exerted to the swollen gel 414. At the bottom face of the cell 411 and the bottom face of the piston 412, wire gauzes 413a and 413b (wire gauzes made of No. 400 stainless steel, sieve opening 38 $\mu$m) are stretched so that the swollen gel 414 (and a particulate water absorbent) cannot pass therethrough. Here, as the liquid 423, a 0.90 mass% aqueous solution of sodium chloride is used. The tank 420 has the liquid 423 accumulated inside . The liquid 423 is introduced into the cell 411 through an L-shaped tube with a cock 422. Furthermore, a glass tube 421 is inserted into the tank 420, and the interior of the glass tube 421 is filled with air. Thereby, the lower end of the glass tube 421 and the liquid surface inside the cell 411 can be made even. That is, so long as the liquid surface of the liquid 423 inside the tank 420 remains upper to the lower end of the glass tube 421, it is possible to maintain the liquid surface inside the cell 411 at a constant level. In the measurement of this time, the difference in elevation between the lower liquid surface of the liquid 423 (that is, lower end of the glass tube 421) inside the tank 420 and the bottom face of the swollen gel 414 was adjusted to 4 cm. That is, according to the apparatus 400, the liquid 423 at a constant positive hydrostatic pressure can be introduced into the cell 411. Since the piston 412 has a hole 415 formed therein, the liquid 423 flows through the hole 415, also further flows through the swollen gel 414 layer, and flows out to the outside of the cell 411. The container 410 is mounted on a stainless steel wire gauze 431 that does not interrupt the passage of the liquid 423. Therefore, the liquid 423 that has flowed out from the cell 411 is finally collected in a capturing container 432. Then, the amount of the liquid 423 collected in the capturing container 432 can be weighed using a Roberval balance 433.

A specific method for measuring the gel permeation rate (GPR) is as follows. Meanwhile, the following operation is carried out at room temperature (20°C to 25°C).

(1) A particulate water absorbent (0.900 g) is uniformly introduced into a cell 411.
(2) The particulate water absorbent is caused to absorb a liquid (0.00 mass% to 0.90 mass% (0.9 mass% in the present application) aqueous solution of sodium chloride) with no load for 60 minutes, and a swollen gel 414 is obtained.
(3) A piston is placed on the swollen gel 414, and the system is brought into a pressurized state at 0.3 psi (2.07 kPa) .
(4) While the hydrostatic pressure is maintained at a constant value of 3,923 dyne/cm$^2$, a liquid 423 is introduced into the cell 411 and is caused to pass through the swollen gel 414 layer.
(5) The amount of the liquid 423 that passes through the swollen gel 414 layer is recorded for 3 minutes at an interval of 5 seconds. That is, the flow rate of the liquid 423 that passes through the swollen gel 414 layer is measured. For the measurement, a Roberval balance 433 and a computer (not illustrated in the diagram) are used.
(6) The flow rates after one minute to after three minutes from the initiation of the flow-through of the liquid 423 are averaged, and the gel permeation rate (GPR) [g/min] is calculated.

With regard to the water-absorbing sheet according to an embodiment of the third aspect of the description, the GPR of the first particulate water absorbent is more preferably 20 g/min or more, even more preferably 35 g/min or more, still more preferably 45 g/min or more, even more preferably 50 g/min or more, still more preferably 55 g/min or more, even more preferably 75 g/min or more, still more preferably 95 g/min or more, even more preferably 118 g/min or more, still more preferably 150 g/min or more, even more preferably 160 g/min or more, still more preferably 165 g/min or more, and even more preferably 175 g/min or more. By being such an embodiment, the liquid to be absorbed may be easily sent to the second particulate water absorbent after being introduced on the first base material side, and the second particulate water absorbent can be effectively utilized. Thus, excellent results are obtained in the evaluation of the specific return amount. With regard to the water-absorbing sheet according to an embodiment of the third aspect of the description, the upper limit of the GPR of the first particulate water absorbent is not particularly limited; however, from the viewpoint of preventing liquid leakage, the upper limit is 500 g/min or less, 400 g/min or less, or 300 g/min or less.

With regard to the water-absorbing sheet according to an embodiment of the third aspect of the description, the GPR of the second particulate water absorbent is 45 g/min or more. Since an intermediate sheet having a permeability of lower than 60% is used in the third aspect of the description, when the GPR of the second particulate water absorbent is less than 45 g/min, the intended problems of the third aspect of the description cannot be solved. To describe more specifically, in the first place, when the permeability is 60% or higher, the particulate water absorbent is widely distributed in the intermediate sheet, and the liquid diffusibility can be increased. However, when the permeability is lower than 60%, the liquid diffusibility by the intermediate sheet becomes insufficient. Thus, in the third aspect of the description, the second particulate water absorbent is made responsible for liquid diffusibility. That is, after a liquid to be absorbed passes through the first particulate water absorbent and is introduced into the second particulate water absorbent, liquid diffusion is appropriately carried out by the second particulate water absorbent that can have, so to speak, a tank-like action, the water absorption area is effectively utilized, and thereby the second particulate water absorbent absorbs the liquid so as not to spill. Therefore, even if the permeability is lower than 60%, the intended problems of the third aspect of the description can be solved. With regard to the water-absorbing sheet according to an embodiment of the third aspect of the description, the GPR of the second particulate water absorbent may be 45 g/min or more, preferably 46 g/min or more, more preferably 47 g/min or more, preferably 50 g/min or more, even more preferably 55 g/min or more, still more preferably 75 g/min or more, even more preferably 95 g/min or more, still more preferably 118 g/min or more, even more preferably 150 g/min or more, still more preferably 160 g/min or more, even more preferably 165 g/min or more, and still more preferably 175 g/min or more. By having such a lower limit, gel blocking is suppressed, diffusibility is increased, and the liquid is easily absorbed by the absorbent body. With regard to the water-absorbing sheet according to an embodiment of the third aspect of the description, the upper limit of the GPR of the first particulate water absorbent is not particularly limited; however, from the viewpoint of preventing liquid leakage, the upper limit is 500 g/min or less, 400 g/min or less, or 300 g/min or less.

With regard to the water-absorbing sheet according to an embodiment of the third aspect of the description, the GPR of the second particulate water absorbent is 50 g/min or more, the content of the first particulate water absorbent with respect to the content of the second particulate water absorbent in the water-absorbing sheet is 0.4 or less, and the permeability is higher than 40%. By being such an embodiment, the intended effects of the third aspect of the description can be made superior. Furthermore, according to the present embodiment, the GPR of the first particulate water absorbent is 50 g/min or more. By being such an embodiment, the intended effects of the third aspect of the description can be made superior.

"Degradable soluble content"

The upper limits of the degradable soluble content of the first particulate water absorbent and the degradable soluble content of the second particulate water absorbent in the water-absorbing sheet according to an embodiment of the third aspect of the description are not particularly limited; however, from the viewpoint of liquid leakage during long-term use, the upper limits are each independently preferably less than 50%, more preferably 25% or less, and even more preferably 15% or less. The lower limits are not particularly limited; however, from the viewpoint of liquid leakage during long-term use, for example, the lower limits are about 1% or more, 3% or more, and 5% or more. Incidentally, a method for measuring the degradable soluble content is as follows.

L-ascorbic acid is added to physiological saline that has been prepared in advance so as to obtain a concentration of 0.05% by mass, and a degradation test liquid is produced. Specifically, 0.50 g of L-ascorbic acid was dissolved in 999.5 g of physiological saline, and thereby a degradation test liquid was prepared. 25 ml of the degradation test liquid is added into a glass beaker container having a capacity of 250 ml, 1.0 g of a particulate water absorbent is added thereto, and thereby a swollen gel is formed. The container is tightly sealed by covering up with a plastic wrap, and the swollen gel is left to stand for 16 hours in an atmosphere at 37°C (manufactured by Kusumoto Chemicals, Ltd., ETAC trade name, HISPEC series, used HT320, set to 37°C, set to a wind velocity variator scale of 30) . After 16 hours, 175 ml of physiological saline and a cylindrical stirring bar having a length of 30 mm and a width of 8 mm are charged therein, the swollen gel is degraded and then stirred at 500 rpm for 10 minutes, and extraction from hydrated gel is performed.

20.0 g of a filtrate that is obtained by filtering this extract using a filter paper (Advantec Toyo Kaisha, Ltd., product name: (JIS P 3801, No. 2), thickness 0.26 mm, retained particle size 5 pm) is weighed, 30 g of physiological saline is further added thereto, and the mixture is used as a measurement solution.

Regarding the measurement method, physiological saline is subjected to titration up to pH 10 using a 0.1 N aqueous solution of NaOH, subsequently the physiological saline is titrated to pH 2.7 using a 0.1 N aqueous solution of HCl, and blank titers ([bNaOH] ml, [bHCl] ml) are obtained. A similar titration operation is also carried out for the measurement solution, and thereby titers ([NaOH]ml, [HCl]ml) are determined. For example, in the case of a particulate water absorbent including known amounts of acrylic acid and sodium salt thereof, a soluble content in the particulate water absorbent can be calculated by the following calculation formula, based on the average molecular weight of the monomer and the titers obtained by the above-described operation:

Degradable soluble content (mass%) = $0.1 \times$ (average molecular weight) $\times 200 \times 100 \times$ ([HCl] - [bHCl])/1000/1.0/20.0.

In the case of an unknown amount, the average molecular weight of the monomer is calculated using a neutralization ratio determined by titration.

"Surface tension"

Surface tension is the work (free energy) required to increase the surface area of a solid or a liquid, expressed in per unit area. The surface tension as used in the present application refers to the surface tension of an aqueous solution when a particulate water absorbent is dispersed in a 0.90 mass% aqueous solution of sodium chloride. Incidentally, the surface tension of a water absorbent is measured by the following procedure. That is, 50 ml of physiological saline that has been adjusted to 20°C is introduced into a sufficiently washed 100-ml beaker, and first, the surface tension of the physiological saline is measured using a surface tensiometer (K11 automatic surface tensiometer manufactured by Kruss GmbH). Next, into the beaker including the physiological saline after surface tension measurement, which has been adjusted to 20°C, a sufficiently washed stirring bar made of a fluororesin, 25 mm in length, and 0.5 g of a particulate water absorbent are charged, and the mixture is stirred for 4 minutes under the conditions of 500 rpm. After 4 minutes, after stirring is stopped and the hydrated particulate water absorbent settles, the surface tension of the supernatant is measured by performing a similar operation again. Incidentally, in the third aspect of the description, a plate method of using a platinum plate is employed, and the plate is sufficiently washed with deionized water before each measurement and is used after being heated with a gas burner and washed. With regard to the water-absorbing sheet according to an embodiment of the third aspect of the description, the surface tension of at least one of the first particulate water absorbent and the second particulate water absorbent is preferably, in the following order, 65 mN/m or more, 66 mN/m or more, 67 mN/m or more, 69 mN/m or more, 70 mN/m or more, or 71 mN/m or more, and most preferably 72 mN/m or more. In the application of a particulate water absorbent to a water-absorbing sheet, the influence of surface tension is more easily exhibited than conventional paper diapers, and as the surface tension satisfies the above-described conditions, the return amount to the paper diaper can be reduced. With regard to the water-absorbing sheet according to an embodiment of the third aspect of the description, the upper limit of the surface tension of at least one of the first particulate water absorbent and the second particulate water absorbent is not particularly limited; however, the upper limit is 73 mN/m or less.

"Particle shape"

It is preferable that at least one of the first particulate water absorbent and the second particulate water absorbent in the water-absorbing sheet according to an embodiment of the third aspect of the description includes particles having an irregular crushed shape as the particle shape. Here, the irregular crushed shape is a crushed-shaped particle whose shape is not definite. Compared to spherical particles obtained by reverse phase suspension polymerization or gas phase polymerization, the irregular crushed shape allows easy fixation to the base material. The particulate water absorbent according to an embodiment of the third aspect of the description is preferably a pulverized product in aqueous solution polymerization. On the other hand, in a case in which the particulate water absorbent is not subjected to a pulverization process, representatively, spherical particles obtainable by reverse phase suspension polymerization, liquid droplet polymerization, by which monomers to be polymerized are sprayed and polymerized, or the like, or a granulated product of spherical particles do not have an irregular crushed shape. According to embodiments of the third aspect of the description, when the shape of the particulate water absorbent is an irregular crushed shape, shape retention of the water-absorbing sheet is easily achieved as compared to particles whose average degree of circularity is high (for example, spherical particles). According to embodiments of the third aspect of the description, the average degree of circularity of at least one of the first particulate water absorbent and the second particulate water absorbent is preferably 0.70 or less, more preferably 0.60 or less, and even more preferably 0.55 or less.

The method for calculating the average degree of circularity is as follows. One hundred or more particles of a particulate water absorbent were randomly selected, various particles of the particulate water absorbent were imaged with an electron microscope (VE-9800 manufactured by KEYENCE CORPORATION) (magnification ratio 50 times), images of the particulate water absorbent were obtained, and the circumference and the area were calculated for each particle using an affiliated image analysis software. The degree of circularity of each particle is determined by the following formula:

[Mathematical Formula 8]

$$\text{Degree of circularity} = 4 \times \pi \times \text{area}/(\text{circumference})^2$$

and the average value of the values thus obtained is calculated as the average degree of circularity.

With regard to the water-absorbing sheet according to an embodiment of the third aspect of the description, the lower limit of the content of the particulate water absorbent per unit volume of the water-absorbing sheet is, in a preferable order, 50 mg/cm$^3$ or more, 51 mg/cm$^3$ or more, 52 mg/cm$^3$ or more, 53 mg/cm$^3$ or more, 54 mg/cm$^3$ or more, 55 mg/cm$^3$ or more, 57 mg/cm$^3$ or more, 59 mg/cm$^3$ or more, or 60 mg/cm$^3$ or more. Furthermore, with regard to the water-absorbing sheet according to an embodiment of the third aspect of the description, the upper limit of the content of the particulate water absorbent per unit volume of the water-absorbing sheet is also not particularly limited; however, the upper limit is practically 600 mg/cm$^3$ or less, preferably 500 mg/cm$^3$ or less, more preferably 400 mg/cm$^3$ or less, even more preferably 300 mg/cm$^3$ or less, and still more preferably 150 mg/cm$^3$ or less. By adjusting the content of the particulate water absorbent to the range described above, the water-absorbing sheet efficiently exhibits the effects of the third aspect of the description. The contents of the particulate water absorbents per unit volume of the water-absorbing sheet of the water-absorbing sheets used in Examples of the present application were 50 mg/cm$^3$ or more.

The method for producing a particulate water absorbent is not particularly limited as long as it is a method for producing a water absorbent having desired physical properties, and for example, a particulate water absorbent can be appropriately produced by referring to the publications described in the Examples and the like.

Furthermore, in the water-absorbing sheet according to an embodiment of the third aspect of the description, the content weight of the first particulate water absorbent with respect to the content weight of the second particulate water absorbent is preferably 1.0 or less, more preferably 0.5 or less, and even more preferably 0.4 or less. By making the content of the second particulate water absorbent to be equal to, and preferably larger than, the content of the first particulate water absorbent, the second particulate water absorbent that is considered to have a so-called tank-like operating function effectively functions. Thus, excellent results are obtained in the evaluation of the specific return amount.

An embodiment of the third aspect of the description is a water-absorbing sheet having a first base material, a second base material, and a water-absorbing layer positioned between the first base material and the second base material, wherein the first base material is a water-permeable sheet positioned on the side where a liquid to be absorbed is introduced, the water-absorbing layer has an intermediate sheet; a first particulate water absorbent localized on the side of a surface of the first base material, the surface being arranged to face the second base material; and a second particulate water absorbent localized on the side of a surface of the second base material, the surface being arranged to face the first base material, the permeability of a mixture of the first particulate water absorbent and the second particulate water absorbent to the intermediate sheet is lower than 60%, the GPR of the second particulate water absorbent is 45.0 g/min or more, and the nonwoven fabric permeability index (NPI) is lower than 60%. According to such an embodiment, due to such a configuration, there can be provided a water-absorbing sheet that can meaningfully reduce the return amount even when introduction of a liquid occurs intermittently for several times (particularly, three or more times) under a situation with no load, and the amount of introduction of the liquid becomes large.

According to an embodiment of the third aspect of the description, the upper limit of the nonwoven fabric permeability index (NPI) is not particularly limited; however, the upper limit is preferably 55 or less, and more preferably 50 or less. By having such a lower limit, the intended effects of the third aspect of the description are efficiently provided. According to an embodiment of the third aspect of the description, the lower limit of the nonwoven fabric permeability index (NPI) is not particularly limited; however, the lower limit is preferably 10 or more, more preferably 20 or more, and even more preferably 30 or more.

Incidentally, the nonwoven fabric permeability index (NPI) described in the present specification is represented by the following formula:

-90 - 1.05 × C - 39.3 × B + 3.15 × A - 0.289 × D - 0.0472 × (C - 52.1) × (D - 369)
+ 3.62 × (B - 3.48) × (A - 142) - 0.0142 × (A - 142) × (D - 369).          [Mathematical Formula 9]

Here, A represents the specific surface area (mm$^{-1}$) of an intermediate sheet (for example, nonwoven fabric) measured byX-ray CT; B represents the thickness (mm) of an intermediate sheet (for example, nonwoven fabric) ; C represents the basis weight (g/m$^2$) of an intermediate sheet (for example, nonwoven fabric) ; and D represents the average particle size ($\mu$m) (D50) of a particulate water absorbent.

Here, the specific surface area of an intermediate sheet was determined by analyzing an image captured by X-ray CT as described below using an analysis software program.

<Imaging by X-ray CT>

An intermediate sheet cut out into a square shape that measured 10 mm in length and 10 mm in width (thickness was as received) was subjected to measurement using a microfocus X-ray CT system, InspeXio SMX-100CT, manufactured by Shimadzu Corp. The measurement conditions are described below.

· Image transverse size (pixel): 512
· Image longitudinal size (pixel): 512
· X-ray tube voltage (kV): 40
· X-ray tube current ($\mu$A): 50
· Inch size (inch): 4.0
· X-ray filter: None
· SDD (distance between focal point of X-ray source and X-ray detector) (mm) : 500
· SRD (distance between focal point of X-ray source and center of rotation of measurement sample) (mm): 40
· Scan mode 1: CBCT
· Scan mode 2: Normal scan
· Scan angle: Full scan
· Number of views: 1,200
· Average number: 5
· Smoothing: YZ
· Slice thickness (mm): 0.012
· Distance between slices (mm): 0.010
· Scaling number: 50
· BHC data: None
· Fine mode: Available
· FOV XY (maximum imaging region XY) (mm): 5.0
· FOV Z (maximum imaging region Z) (mm): 1.5
• Voxel size (mm/voxel): 0.010.

<Calculation of specific surface area>

The imaging data of X-ray CT was subjected to analysis by the following procedure using an analysis software program, TRI/3D-PRT-LRG,manufactured by RatocSystem Engineering Co., Ltd.

1. From the menu, Particle Measurement > 3D Particles > Particle Separation > Giant Particle Separation is selected. EV panel, BC panel, EVC panel, and Giant Particle Separation panel are displayed.
2. L-W is selected at the Binarize tab of the EVC panel, the L value is changed, and a circular-shaped measurement target region is selected. "Execute" is pressed, and this treatment is applied to all slice images (a cylindrical-shaped measurement region is selected as a whole) . "ROI OK" of the Giant Particle Separation panel is pressed.
3. L-W is selected at the Binarize tab of the EVC panel, the L value is set to 37,580, and thereby only fibers are selected. "Execute" is pressed. bD of the BC panel is selected, and "Save" is pressed.
4. Labeling tab is selected at the 3D tab of the EVC panel, "Volume" is selected, and MAX is executed (by this operation, 3D Label Count = 1 is displayed).
5. From the menu, Particle Measurement > Void in 3D Particles > Measurement After Separation is selected. Measurement After Separation panel is displayed. A check for the Removal of Edge Particles is removed, a check is inserted for the Surface Area Calculation of the measurement items, Void Calculation is selected, Binary 5ch is selected at the Calculation ROI Designation, "Register OK" is pressed, and the folder for saving data is selected. "Recent Registered Data Execution" is pressed, and computation processing is executed.
6. From the calculation results, the specific surface area was calculated by the following formula.

Specific surface area (mm$^{-1}$ = Total particle surface area (mm$^2$) / (Total particle volume (mm$^3$)

Incidentally, for the configuration of the calculation software, the expression "Particle" is used; however, the actual values are measurement results obtained with fibers, and there are no problems in the measurement and calculation.

[3. Method for producing water-absorbing sheet]

The method for producing a water-absorbing sheet according to an embodiment of the third aspect of the description includes at least one of: (1) a step of scattering a first particulate water absorbent on a first base material, (2) a step of scattering a second particulate water absorbent on a second base material, and (3) a step of scattering a first particulate water absorbent and/or a second particulate water absorbent on an intermediate sheet. As more specific examples of the production method, the following production methods of (a) to (d) may be mentioned.

(a) A first particulate water absorbent (and preferably an adhesive) is uniformly scattered on a first base material. An intermediate sheet is superposed thereon, and the assembly is pressure-bonded. Furthermore, on a surface of the intermediate sheet on the side that does not face the first particulate water absorbent, a second particulate water absorbent (and preferably an adhesive) is uniformly scattered. An intermediate sheet is superposed thereon, and (preferably under heated conditions in which a hot melt is melted, or in a state in which a hot melt is melting) the assembly is pressure-bonded.

(b) A second particulate water absorbent is uniformly scattered on an intermediate sheet. Furthermore, an adhesive is scattered on a second base material. Then, the surface of the intermediate sheet, on which the second particulate water absorbent has been scattered, and the surface of the second base material, on which the adhesive has been scattered, are pressure-bonded. Next, in the intermediate sheet after pressure-bonding, on the surface on the opposite side of the surface on which the second particulate water absorbent has been scattered, a first particulate water absorbent is uniformly scattered. Furthermore, an adhesive is scattered on the first base material. Then, the surface of the intermediate sheet, on which the first particulate water absorbent has been scattered, and the surface of the first base material, on which the adhesive has been scattered, are pressure-bonded. It is preferable that the above-described pressure-bonding is carried out under heated conditions in which a hot melt is melted, or in a state in which a hot melt is melting.

(c) An adhesive is scattered on a second base material. Next, a second particulate water absorbent is uniformly scattered thereon. Next, an intermediate sheet is placed thereon, and the assembly is pressure-bonded. Next, an adhesive is scattered on a surface of the intermediate sheet on the side that does not face the second particulate water absorbent. Next, a first particulate water absorbent is uniformly scattered thereon. Next, a first base material is placed thereon, and the assembly is pressure-bonded. It is preferable that the above-described pressure-bonding is carried out under heated conditions in which a hot melt is melted, or in a state in which a hot melt is melting.

(d) An adhesive is scattered on a second base material. Next, a second particulate water absorbent is uniformly scattered thereon. Next, an intermediate sheet is placed thereon, and the assembly is pressure-bonded. Next, on the surface of the intermediate sheet on the side that does not face the second particulate water absorbent, a first particulate water absorbent is uniformly scattered. Furthermore, an adhesive is scattered on a first base material. Then, the surface of the intermediate sheet, on which the first particulate water absorbent has been scattered, and the surface of the first base material, on which the adhesive has been scattered, are pressure-bonded. It is preferable that the above-described pressure-bonding is carried out under heated conditions in which a hot melt is melted, or in a state in which a hot melt is melting.

As a step other than those described above, for the purpose of improving the feel of the water-absorbing sheet to the touch and enhancing the liquid absorption performance, the water-absorbing sheet may be subjected to embossing processing. The embossing processing may be carried out simultaneously when the first base material and the second base material are pressure-bonded or may be carried out after sheet production.

In the method for producing a water-absorbing sheet according to an embodiment of the third aspect of the description, additives (adeodorant, fibers, an antibacterial agent, a gel stabilizer, and the like) may be appropriately incorporated. The amount of incorporation of the additives is preferably 0% to 50% by mass, and more preferably 0% to 10% by mass, with respect to the mass of the particulate water absorbent. In the above-described production method, a particulate water absorbent that has been mixed with additives in advance may be used, or additives may be added in the middle of the production process.

The dimension of the water-absorbing sheet to be produced can be appropriately designed. Usually, the transverse width is 10 cm to 10 m, and the length is several dozen meters (m) to several thousand meters (m) (for a continuous sheet or a roll form) . The water-absorbing sheet thus produced is used after being cut out according to the purpose (size of the absorbent body to be used).

In addition to the examples described above, the method for producing a water-absorbing sheet is disclosed in the following patent literatures: WO 2012/174026 A, WO 2013/078109 A, WO 2015/041784 A, WO 2011/117187 A, WO 2012/001117 A, WO 2012/024445 A, WO 2010/004894 A, WO 2010/004895 A, WO 2010/076857 A, WO 2010/082373 A, WO 2010/113754 A, WO 2010/143635 A, WO 2011/043256 A, WO 2011/086841 A, WO 2011/086842 A, WO 2011/086843 A, WO 2011/086844 A, WO 2011/117997 A, WO 2011/118409 A, WO 2011/136087 A, WO 2012/043546 A, WO 2013/099634 A, WO 2013/099635A, JP2010-115406A, JP2002-345883A, JPH6-315501 A, JP H6-190003 A, JP H6-190002 A, JP H6-190001 A, JP H2-252558 A, JP H2-252560 A, and JP H2-252561 A. The methods for producing a water-absorbing sheet disclosed in these literatures are also appropriately referred to.

With regard to the water-absorbing sheet according to an embodiment of the third aspect of the description, as a method of fixing base materials together or fixing a base material with a particulate water absorbent, (i) an adhesive may be used, and if necessary, pressure-bonding may be used, (ii) various binders dissolved or dispersed in water, a water-soluble polymer, or a solvent may be used, or (iii) base materials may be heat-sealed at the melting point of the material of the base materials themselves. Preferably, fixing is achieved using an adhesive.

The adhesive to be used may be a solution type; however, from the viewpoint of the effort to remove the solvent, the problem of remaining solvent, and the problem of productivity, a hot melt adhesive that exhibits high productivity and does not have a problem of residual solvent is preferred. In the third aspect of the description, the hot melt adhesive may be incorporated in advance at the surface of the base material or the particulate water absorbent, or separately, a hot melt adhesive may be used during the production process for a water-absorbing sheet. The form or melting point of the hot melt adhesive can be appropriately selected, and the hot melt adhesive may be in a particulate form, may be in a fibrous form, may be in a net form, may be in a film form, or may be in a liquid form melted by heating. The melting temperature or the softening point of the hot melt adhesive is preferably 50°C to 200°C, or 60°C to 180°C. In the case of using a particulate adhesive, a particulate adhesive in which the particle size is about 0.01 to 2 times, 0.02 to 1 time, or 0.05 to 0.5 times, the average particle size of the particulate water absorbent, is used.

Regarding the method of using a hot melt adhesive in the production of the water-absorbing sheet according to an embodiment of the third aspect of the description, the following example may be mentioned. A water-absorbing sheet can be produced by uniformly scattering a mixture of a particulate water absorbent and a hot melt adhesive on a base material (for example, a nonwoven fabric), laminating another sheet of base material thereon, and heating and pressure-bonding the laminate at near the melting temperature of the hot melt adhesive.

The hot melt adhesive to be used for the third aspect of the description can be appropriately selected; however, preferably, one or more kinds selected from an ethylene-vinyl acetate copolymer adhesive, a styrene-based elastomer adhesive, a polyolefin-based adhesive, a polyester-based adhesive, and the like can be appropriately used.

Specific examples include, as polyolefin-based adhesives, polyethylene, polypropylene, and atactic polypropylene; as styrene-based elastomer adhesives, a styrene-isoprene block copolymer (SIS), a styrene-butadiene block copolymer (SBS), a styrene-isobutylene block copolymer (SIBS), a styrene-ethylene-butylene-styrene block copolymer (SEES), a styrene-butadiene rubber (SBR), and the like; copolymerized polyolefins, and the like; as polyester-based adhesives, polyethylene terephthalate (PET), polybutylene terephthalate (PBT), copolymerized polyesters, and the like; as ethylene-vinyl acetate copolymer adhesives, an ethylene-vinyl acetate copolymer (EVA) adhesive; an ethylene-ethyl acrylate copolymer (EEA), an ethylene-butyl acrylate copolymer (EBA), and the like.

With regard to the water-absorbing sheet according to an embodiment of the third aspect of the description and/or a method for producing the same, it is preferable that the water-absorbing sheet includes an adhesive, and the adhesive is preferably a hot melt adhesive. The amount of use (content) of the adhesive (forexample, a hotmelt adhesive) is preferably more than 0 and 3.0 times, and more preferably 0.05 to 2.0 times, the total mass of the first particulate water absorbent and the second particulate water absorbent. When the content of the adhesive (particularly, a hot melt melt adhesive) is too large, not only it is disadvantageous in view of the cost and in view of the mass of the water-absorbing sheet (increase in the mass of a paper diaper), but also there is a possibility that the particulate water absorbent may be subjected to the regulation of swelling and lower the water absorption power of the water-absorbing sheet.

[4. Absorbent article]

The absorbent article according to an embodiment of the third aspect of the description has a structure in which the water-absorbing sheet described in [2] is interposed between a liquid-permeable sheet and a liquid-impermeable sheet. Therefore, according to the third aspect of the description, there is provided an absorbent article having the water-absorbing sheet interposed between a liquid-permeable sheet and a liquid-impermeable sheet, the liquid-permeable sheet being positioned on the first base material side, and the liquid-impermeable sheet being positioned on the second base material side. Specific examples of the absorbent article include a disposable diaper, an incontinence pad, a sanitary napkin, a pet sheet, a drip sheet for food, a water sealant for electric power cables, and the like. By having such a configuration, excellent results are obtained in the evaluation of the specific return amount.

Regarding the liquid-permeable sheet and the liquid-impermeable sheet, those known in the technical field of absorbent articles can be used without particular limitations. Furthermore, the absorbent article can be produced according to a known method.

[EXAMPLES]

The third aspect of the description will be described in more detail using the following Examples and Comparative Examples. However, the technical scope of the third aspect of the description is not intended to be limited to the following Examples only. Furthermore, in the following Examples, unless particularly stated otherwise, operations were carried out under the conditions of room temperature (25°C)/relative humidity of 40% to 50%RH.

<Production Example>

With reference to the Production Examples, Examples, and Comparative Examples described in the following patent literatures, particulate water absorbents (1) and (2) of polyacrylic acid (salt)-based resins were obtained by appropriately adjusting CRC by means of the amount of an internal crosslinking agent. The physical properties of the particulate water absorbents thus obtained are presented in Table 1.

> WO 2014/034897 A
> WO 2017/170605 A
> WO 2016/204302 A
> WO 2014/054656 A
> WO 2015/152299 A
> WO 2018/062539 A
> WO 2012/043821 A.

[Production Example of acrylic acid]

Commercially available acrylic acid (acrylic acid dimer 2, 000 ppm, acetic acid 500 ppm, propionic acid 500 ppm, and p-methoxyphenol 200 ppm) was supplied to the column bottom of a high-boiling point impurities separating column having fifty weirless perforated plates and was distilled at a reflux ratio of 1. After removal of maleic acid, a dimer including acrylic acid (acrylic acid dimer), and the like, crystallization is further carried out, and thereby acrylic acid (acrylic acid dimer 20 ppm, acetic acid 50 ppm, propionic acid 50 ppm, furfural 1 ppm or less, and protoanemonin 1 ppm or less) was obtained. After performing further distillation, 50 ppm of p-methoxyphenol was added thereto.

[Method for producing aqueous solution of sodium acrylate]

1,390 g of the acrylic acid was neutralized at 20°C to 40°C using 48% caustic soda according to Example 9 of US Patent No. 5210298, and thereby a 100% neutralized aqueous solution of sodium acrylate at a concentration of 37% was obtained.

<Particulate water absorbent 1>

In 5, 500 g (monomer concentration 33.0% by mass) of an aqueous solution of sodium acrylate having a neutralization ratio of 73 mol%, which was obtained by mixing the acrylic acid obtained in the above-described Production Example of acrylic acid, an aqueous solution of sodium acrylate obtained by the above-described method for producing an aqueous solution of sodium acrylate using the acrylic acid, and deionized water, 5.71 g of polyethylene glycol diacrylate (average number of added moles of ethylene oxide 9) was dissolved, and a reaction liquid was obtained. Next, the reaction liquid was supplied to a reactor formed by attaching a lid to a jacketed stainless steel double-arm type kneader having an internal volume of 10 L and having two sigma-type blades, the system was purged with nitrogen gas while maintaining the reaction liquid at 30°C, and dissolved oxygen in the reaction liquid was removed. Subsequently, while the reaction liquid was stirred, 26.78 g of a 10 mass% aqueous solution of sodium persulfate and 32.96 g of a 1 mass% aqueous solution of L-ascorbic acid were added thereto, and after approximately 1 minute, polymerization was initiated. Forty minutes after the initiation of polymerization, 184.3 g of a water-absorbing resin fine powder having a size of 150 $\mu$m or less was added thereto, the gel was cracked for 10 minutes by rotating the blades of the kneader at a high speed (130 rpm), and then a hydrated gel-like polymer was taken out. The hydrated gel-like polymer thus obtained was finely divided into particles that measured about 1 to 2 mm. This finely divided hydrated gel-like polymer was spread on a 50-mesh (mesh size 300 pm) wire gauze and was subjected to hot air drying for 65 minutes at 175°C. Next, the dried product was pulverized using a roll mill, further classified with a wire gauze having a sieve opening of 600 $\mu$m, andprepared. Thereby, water-absorbing resin (1-1) having an irregular crushed shape with an average particle size of 350 $\mu$m was obtained.

Into 100 parts by mass of the water-absorbing resin (1-1) thus obtained, 3.83 parts by mass of an aqueous solution of a surface crosslinking agent including 0.03 parts by mass of ethylene glycol diglycidyl ether, 0.3 parts by mass of 1,4-butanediol, 0.5 parts by mass of propylene glycol, and 3.0 parts by mass of water was sprayed and mixed. The mixture was subjected to a heating treatment for 40 minutes at a heat medium temperature of 210°C using a paddle type mixing and heat-treating machine, and surface-crosslinked water-absorbing resin (1-2) was obtained. 1.0 part by mass of water was sprayed and mixed into 100 parts by mass of the surface-crosslinked water-absorbing resin (1-2) thus obtained, the mixture was cured in a sealed container for one hour at 60°C and then was passed through a sieve having a sieve opening of 710 $\mu$m. Thus, water-absorbing resin (1-3) was obtained. 0.3 parts by mass of Aerosil 200 (hydrophilic amorphous silica, manufactured by Nippon Aerosil Co., Ltd.) was added to the water-absorbing resin (1-3) and mixed, and thereby the water-absorbing resin thus obtained was produced as particulate water absorbent (1).

<Particulate water absorbent 2>

In 5,500 g (monomer concentration 33.0% by mass) of an aqueous solution of sodium acrylate having a neutralization ratio of 73 mol%, which was obtained by mixing the acrylic acid obtained in the above-described Production Example of acrylic acid, an aqueous solution of sodium acrylate obtained by the above-described method for producing an aqueous solution of sodium acrylate using the acrylic acid, and deionized water, 4.17 g of polyethylene glycol diacrylate (average number of added moles of ethylene oxide 9) was dissolved, and a reaction liquid was obtained. Next, the reaction liquid was supplied to a reactor formed by attaching a lid to a jacketed stainless steel double-arm type kneader having an internal volume of 10 L and having two sigma-type blades, the system was purged with nitrogen gas while maintaining the reaction liquid at 30°C, and dissolved oxygen in the reaction liquid was removed. Subsequently, while the reaction liquid was stirred, 26.78 g of a 10 mass% aqueous solution of sodium persulfate and 32.96 g of a 1 mass% aqueous solution of L-ascorbic acid were added thereto, and after approximately 1 minute, polymerization was initiated. Forty minutes after the initiation of polymerization, 181.5 g of a water-absorbing resin fine powder having a size of 150 $\mu$m or less was added thereto, the gel was cracked for 10 minutes by rotating the blades of the kneader at a high speed (130 rpm), and then a hydrated gel-like polymer was taken out. The hydrated gel-like polymer thus obtained was finely divided into particles that measured about 1 to 2 mm. This finely divided hydrated gel-like polymer was spread on a 50-mesh (mesh size 300 pm) wire gauze and was subjected to hot air drying for 65 minutes at 175°C. Next, the dried product was pulverized using a roll mill, further classified with a wire gauze having a sieve opening of 600 $\mu$m, andprepared. Thereby, water-absorbing resin (2-1) having an irregular crushed shape with an average particle size of 350 $\mu$m was obtained.

Into 100 parts by mass of the water-absorbing resin (2-1) thus obtained, 3.83 parts by mass of an aqueous solution of a surface crosslinking agent including 0.03 parts by mass of ethylene glycol diglycidyl ether, 0.3 parts by mass of 1,4-butanediol, 0.5 parts by mass of propylene glycol, and 3.0 parts by mass of water was sprayed and mixed. The mixture was subjected to a heating treatment for 40 minutes at a heat medium temperature of 195°C using a paddle type mixing and heat-treating machine, and surface-crosslinked water-absorbing resin (2-2) was obtained. 1.0 part by mass of water was sprayed and mixed into 100 parts by mass of the surface-crosslinked water-absorbing resin (2-2) thus obtained, the mixture was cured in a sealed container for one hour at 60°C and then was passed through a sieve having a sieve opening of 710 $\mu$m. Thus, water-absorbing resin (2-3) was obtained. 0.3 parts by mass of Aerosil 200 (hydrophilic amorphous silica, manufactured by Nippon Aerosil Co., Ltd.) was added to the water-absorbing resin (2-3) and mixed, and thereby the water-absorbing resin thus obtained was produced as particulate water absorbent (2).

[Extraction of water-absorbing resin from commercially available disposable diaper 1]

A water-absorbing resin was taken out from commercially available disposable diapers (MOONY AIRFIT (L size, Lot No. 201512163072), manufactured by Unicharm Corp., purchased in May 2 016) . At the time of extraction, only the water-absorbing resin was taken out so as not to be mixed with cotton-like pulp and the like. The extracted water-absorbing resin had a particle shape obtained by granulating spherical particles. This water-absorbing resin was designated as particulate water absorbent (T1). The physical properties of the particulate water absorbent (T1) are presented in Table 1.

[EXAMPLES]

[Example 1]

On a surface of an air-through nonwoven fabric (1) made of olefins as a main component (corresponding to the intermediate sheet) having a thickness of 8.3 mm under no load, which had been cut into a size of 10 cm in length and 40 cm in width, 9.0 g (scattering amount: 225 g/m$^2$) of the particulate water absorbent (1) (corresponding to the second particulate water absorbent) was uniformly scattered.

Next, on a surface of a nonwoven fabric (B) (made of pulp fibers as a main component, thickness under no load: 0.7 mm, produced by an air-laid method, corresponding to the second base material, basis weight: 42 g/m$^2$) that had been cut into a size of 10 cm in length and 40 cm in width), 0.3 to 0.5 g of an adhesive including styrene-butadiene rubber (spray adhesive 77, manufactured by 3M Japan, Ltd.) was uniformly scattered (scattering amount: 7.5 to 12.5 g/m$^2$).

Next, the surface of the nonwoven fabric (1) where the particulate water absorbent had been scattered, and the surface of the nonwoven fabric (B) where the adhesive had been scattered, were superposed so as to be paired (so as to be in contact), and the assembly was pressurized and pressure-bonded.

Next, on the surface of the nonwoven fabric (1) on the side that did not face the particulate water absorbent, 3.0 g (scattering amount: 75 g/m$^2$) of the particulate water absorbent (1) (corresponding to the first particulate water absorbent) was uniformly scattered.

Furthermore, on the resultant, a nonwoven fabric (A) (made of pulp fibers as a main component, thickness under no load: 0.7 mm, produced by an air-laid method, corresponding to the first base material, basis weight: 42 g/m$^2$), on which 0.3 to 0.5 g of an adhesive including styrene-butadiene rubber (spray adhesive 77, manufactured by 3M Japan, Ltd.) had been uniformly scattered (scattering amount: 7.5 to 12.5 g/m$^2$), was superposed such that the surface of the nonwoven fabric (1) where the particulate water absorbent had been scattered, and the surface of the nonwoven fabric (A) where the adhesive had been scattered would be paired (so as to come into contact with each other), and the assembly was pressurized and pressure-bonded. In this manner, water-absorbing sheet (1) was obtained.

[Example 2]

With regard to Example 1, as shown in Table 2, a nonwoven fabric (2) (made of olefins as a main component, air-through nonwoven fabric) was used instead of the nonwoven fabric (1), the particulate water absorbent (2) was used instead of the particulate water absorbent (1) corresponding to the second particulate water absorbent, and thereby water-absorbing sheet (2) was obtained.

[Example 3]

With regard to Example 1, the particulate water absorbent (2) was used instead of the particulate water absorbent (1) corresponding to the first particulate water absorbent, and thereby water-absorbing sheet (3) was obtained.

[Example 4]

With regard to Example 3, 6. 0 g (scattering amount: 150 g/m$^2$) of the particulate water absorbent (1) and 6.0 g (scattering amount: 150 g/m$^2$) of the particulate water absorbent (2) were used, and thereby water-absorbing sheet (4) was obtained.

[Comparative Example 1]

With regard to Example 1, the particulate water absorbent (T1) was used instead of the particulate water absorbent (1) corresponding to the second particulate water absorbent, and thereby comparative water-absorbing sheet (1) was obtained.

An evaluation (measurement of reversion amount) of absorbent bodies of the water-absorbing sheets thus obtained was carried out, and the results are presented in Table 3.

[Example 5]

With regard to Example 2, as shown in Table 2, the nonwoven fabric (1) was used instead of the nonwoven fabric (2), and thereby water-absorbing sheet (5) was obtained.

[Example 6]

With regard to Example 1, 6. 0 g (scattering amount: 150 g/m$^2$) of the particulate water absorbent (1) and 6.0 g (scattering amount: 150 g/m$^2$) of the particulate water absorbent (2) were used, and thereby water-absorbing sheet (6) was obtained.

[Example 7]

With regard to Example 1, as shown in Table 2, the nonwoven fabric (2) was used instead of the nonwoven fabric (1), and thereby water-absorbing sheet (7) was obtained.

[Method for evaluating absorbent sheet, or the like]

<Reversion amount>

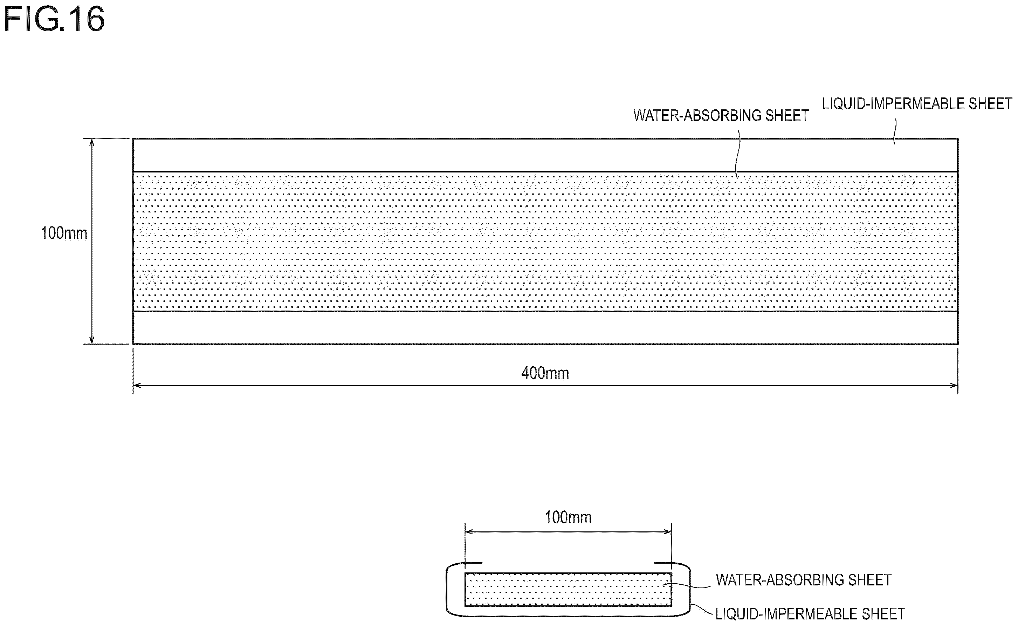

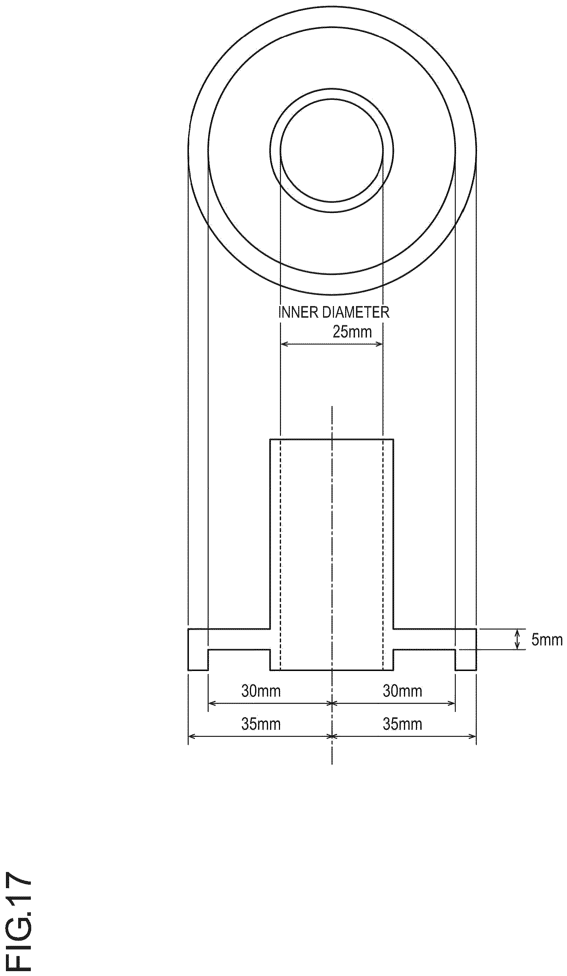

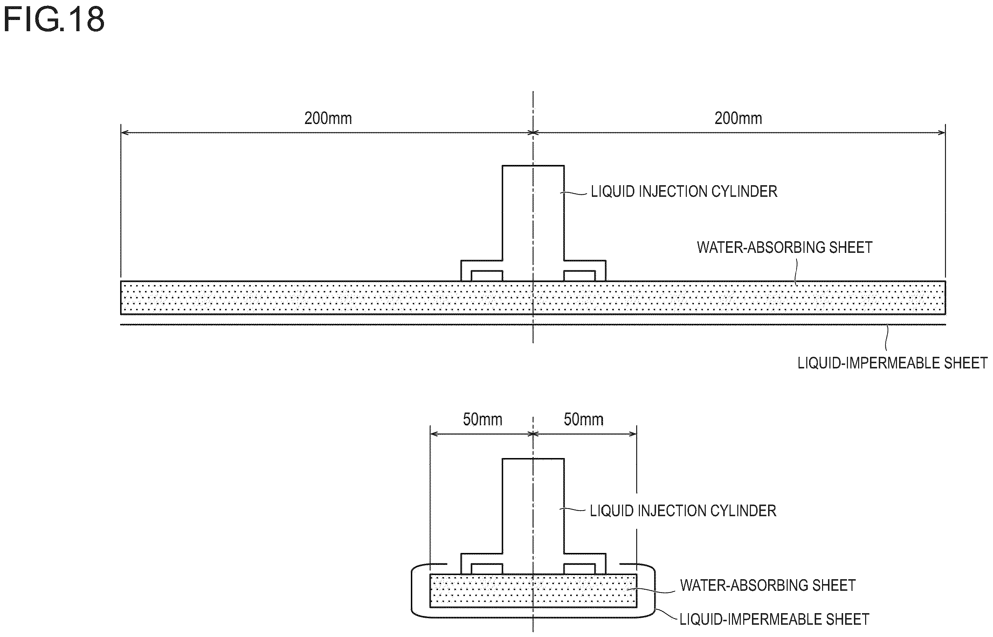

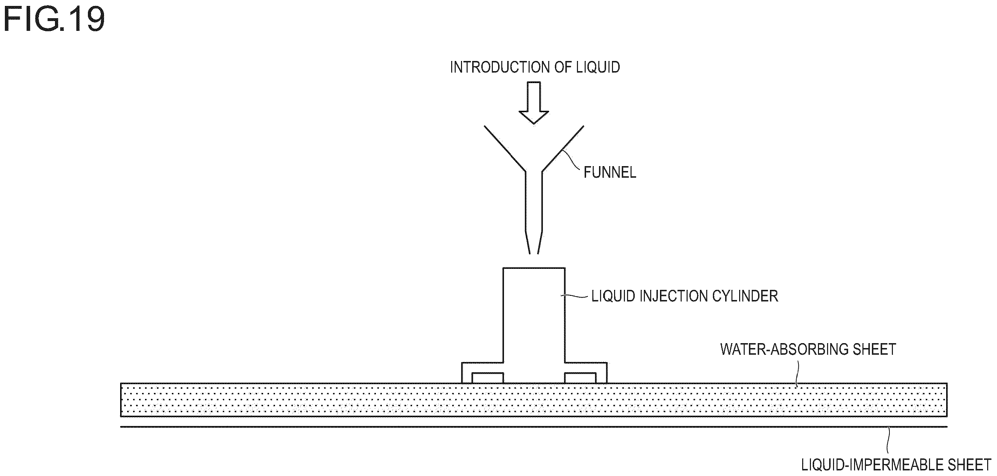

A water-absorbing sheet that had been cut into a size of 10 cm in length and 40 cm in width as illustrated in Fig. 16 was wrapped with a liquid-impermeable sheet having a size of 14 cm in length and 40 cm in width, such that an opening would be produced at the top. The water-absorbing sheet wrapped with the liquid-impermeable sheet was placed on a flat surface, and a liquid injection cylinder (Fig. 17) was placed thereon at the center of the water-absorbing sheet as illustrated in Fig. 18. In this state, 80 g of a 0.9 wt% aqueous solution of sodium chloride at 23°C was charged into the liquid injection cylinder using a funnel that enabled liquid charge at a flow rate of 7 ml/second (Fig. 19). Ten minutes after the liquid charge, 20 sheets of filter paper (Model No. 2, manufactured by Advantec MFS, Inc.; circular-shaped product having a diameter of 110 mm), whose weight had been measured in advance, were placed at the center of the water-absorbing sheet, a circular-shaped weight (1,200 g) having a diameter of 100 mm was further placed thereon, and this was retained for 1 minute. After 1 minute, the weight was removed, and the reversion amount first time (g) was measured from a weight increment of the filter paper. One minute after the removal of the weight, a similar operation (10 minutes after the charge of liquid, filter paper and a weight (1,200 g) were placed and retained for 1 minute. After 1 minute, the weight was removed, the measurement of the reversion amount was repeated, and the reversion amount second time (g) and the reversion amount third time (g) were measured. Meanwhile, as the reversion amount third time (g) is smaller, the sample is evaluated to be superior. The reversion amounts of the various water-absorbing sheets and the various comparative water-absorbing sheets are presented in Table 2.

<Method for measuring permeability of particulate absorbent>

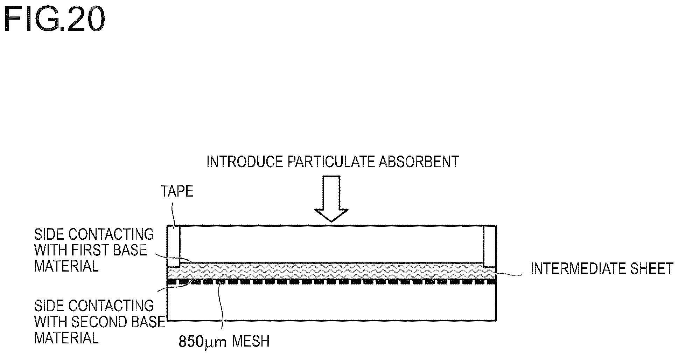

In a JIS standard sieve (The IIDA TESTING SIEVE: inner diameter 80 mm; JIS Z8801-1 (2000)) having a sieve opening of 850 μm, or a sieve corresponding to a JIS standard sieve, the nonwoven fabric (1) (corresponding to the intermediate sheet) that had been cut into a diameter of 80 mmwas installed as illustrated in Fig. 20, and the circumference was fixed with a tape (an area having at least a diameter of 75 mm or more, which is permeable to particles, is secured). Furthermore, at this time, with regard to the form of the water-absorbing sheet, the nonwoven fabric (1) was installed inside the sieve such that the surface of the nonwoven fabric (1) that was in contact with the nonwoven fabric (A) (first base material) would face upward. For the nonwoven fabric (1), a nonwoven fabric taken out from a water-absorbing sheet by the method that will be described below may also be used. 10.0 g of a particulate water absorbent was charged onto the nonwoven fabric (1) in the sieve, and the particulate water absorbent was shaken for 5 minutes under the conditions of room temperature (20°C to 25°C) and a relative humidity of 50%RH, using a low-tap type sieve shaker (ES-65 type sieve shaker manufactured by Sieve Factory Iida Co., Ltd.; speed of rotation 230 rpm, number of mechanical shocks 130 rpm). For the particulate water absorbent, a particulate water absorbent taken out from a water-absorbing sheet by the method that will be described below may also be used. After shaking, the mass (W (g)) of the particulate water absorbent that had passed through the nonwoven fabric (1) and the JIS standard sieve was measured, and the permeability of the particulate absorbent was calculated by the following formula (i) . Meanwhile, measurement was performed three times, and the average value thereof was calculated.
[Mathematical Formula 10] Permeability of particulate absorbant (mass%) = {W/10.0} × 100                Formula (i)

The permeability of the nonwoven fabric (2) was also similarly measured.

[Method for extracting particulate absorbent from water-absorbing sheet and extracting nonwoven fabric used in intermediate sheet]

An upper nonwoven fabric (corresponding to the first base material) and a lower nonwoven fabric (corresponding to the second base material) were torn off from a water-absorbing sheet, and thereby a particulate absorbent and an intermediate sheet were taken out. The particulate absorbent stuck to the upper and lower nonwoven fabrics and the intermediate sheet was also all taken out. When the upper and lower nonwoven fabrics were torn off, they were torn off after cooling the water-absorbing sheet, and sufficiently weakening the adhesiveness of the adhesive (hot melt adhesive or spray adhesive) adhering the nonwoven fabrics and the particulate water absorbent. By going through this procedure, the nonwoven fabrics can be taken out without changing the fibers or structure thickness of the intermediate sheet, and it is possible to measure the permeability accurately. Regarding the method for cooling the water-absorbing sheet, various measures such as placing the water-absorbing sheet in a constant temperature chamber at -10°C or lower for a certain time period, blowing a cooling spray, or applying liquid nitrogen may be considered; however, the cooling method is not particularly limited so long as the method is carried out without changing the fibers, structure, and thickness of the intermediate sheet, and under the conditions in which the particulate water absorbent included in the water-absorbing sheet does not absorb moisture.

Furthermore, in a case in which the extracted particulate water absorbent has absorbed moisture, the percentage water content may be adjusted to 10% by mass or less, and preferably 5% $\pm$ 2% by mass by, for example, drying, and then the permeability and the various physical properties defined in the present application may be measured. The drying conditions for adjusting the percentage water content are not particularly limited so long as they are conditions in which decomposition or denaturation of the water-absorbing resin (particulate water absorbent) does not occur; however, drying under reduced pressure is desirable.

[Table 6]

| Table 1 | | | | | | |
|---|---|---|---|---|---|---|
| | CRC [g/g] | AAP2.1 kPa [g/g] | Weight average particle size [μm] | Vortex [sec] | GPR [g/min] | Surface tension [mN/m] |
| Particulate water absorbent (1) | 33 | 29 | 350 | 30 | 180 | 72 |
| Particulate water absorbent (2) | 38 | 28 | 350 | 32 | 48 | 72 |
| Particulate water absorbent (T1) | 33 | 33 | 350 | 30 | 2 | 58 |

[Table 7]

| Table 2 | | | |
|---|---|---|---|
| | Basis weight (g/m²) | Thickness (mm) | Porosity (5) |
| Nonwoven fabric (1) | 98.0 | 8.3 | 98.8 |
| Nonwoven fabric (2) | 109.0 | 7.4 | 98.5 |

[Table 8]

| | First particulate water absorbent | | Second particulate water absorbent | | Intermediate sheet | Content ratio of first/ second particulate water absorbents | Weight average particle size (μm) | Average CRC (g/g) | NPI index | Permeability (%) | Reversion amount third time (g) |
|---|---|---|---|---|---|---|---|---|---|---|---|
| Table 3 | | | | | | | | | | | |
| | | GPR [g/min] | | GPR [g/min] | | | | | | | |
| Example 1 | Particulate water absorbent (1) | 180 | Particulate water absorbent (1) | 180 | Nonwoven fabric (1) | 0.33 | 350 | 33 | 47 | 52 | 5.5 |
| Example 2 | Particulate water absorbent (1) | 180 | Particulate water absorbent (2) | 48 | Nonwoven fabric (2) | 0.33 | 350 | 37 | 35 | 40 | 6 |
| Example 3 | Particulate water absorbent (2) | 48 | Particulate water absorbent (1) | 180 | Nonwoven fabric (1) | 0.33 | 350 | 34 | 47 | 52 | 5.8 |
| Example 4 | Particulate water absorbent (2) | 48 | Particulate water absorbent (1) | 180 | Nonwoven fabric (1) | 1 | 350 | 36 | 47 | 52 | 6.9 |
| Example 5 | Particulate water absorbent (1) | 180 | Particulate water absorbent (2) | 48 | Nonwoven fabric (1) | 0.33 | 350 | 37 | 47 | 52 | 5.9 |
| Example 6 | Particulate water absorbent (1) | 180 | Particulate water absorbent (1) | 180 | Nonwoven fabric (1) | 1 | 350 | 33 | 47 | 52 | 7.0 |
| Example 7 | Particulate water absorbent (1) | 180 | Particulate water absorbent (1) | 180 | Nonwoven fabric (2) | 0.33 | 350 | 33 | 35 | 40 | 6.8 |
| Comparative Example 1 | Particulate water absorbent (1) | 180 | Particulate water absorbent (T1) | 2 | Nonwoven fabric (1) | 0.33 | 350 | 33 | 47 | 52 | 8 |

[Reference Signs List]

11: first base material
12: water-absorbing layer
13: second base material
14: particulate water absorbent
14a: first particulate water absorbent
14b: second particulate water absorbent
16: intermediate sheet
40: water-absorbing sheet
400: apparatus
410: container
411: cell
412: piston
413a, 413b: wire gauze
414: swollen gel (product obtained by causing a particulate water absorbent to absorb water)
415: hole
420: tank
421: glass tube
422: L-shaped tube with a cock glass tube
423: liquid
431: stainless steel wire gauze
432: capturing container
433: Roberval balance

The above description is an explanation for the "third aspect of the present description".

Claims

1. A water-absorbing sheet comprising:

a first base material;
a second base material; and
a water-absorbing layer positioned between the first base material and the second base material,
wherein the first base material is a water-permeable sheet positioned on a side where a liquid to be absorbed is introduced,
the water-absorbing layer has a first particulate water absorbent localized on a side of a surface of the first base material, the surface being arranged to face the second base material; and a second particulate water absorbent localized on a side of a surface of the second base material, the surface being arranged to face the first base material,
an overall absorption amount as represented by the following formula:

$$[\text{Mathematical Formula 1}]$$
$$CRC \ g/g + 0.44 \times AAP2.1kPa \ g/g$$

of the second particulate water absorbent is 48 g/g or more, and a water absorption time determined according to a vortex method is 35 seconds or more,
a CRC of the second particulate water absorbent is 30 to 50 g/g, and an AAP2.1kPa is 15 to 35 g/g, wherein the CRC is measured according to ERT441.2-02 and the AAP2.1kPa is measured according to ERT442.2-02, and
the first and second particulate water absorbents are fixed to the first and second base materials by means of an adhesive.

2. The water-absorbing sheet according to claim 1, wherein the CRC of the second particulate water absorbent is 36 to 45 g/g.

3. The water-absorbing sheet according to claim 1 or 2, wherein the AAP2.1kPa of the second particulate water absorbent is 23 to 33 g/g.

4. The water-absorbing sheet according to any one of claims 1 to 3, wherein a content of the particulate water absorbents per unit volume of the water-absorbing sheet is 50 mg/cm$^3$ or more.

5. The water-absorbing sheet according to any one of claims 1 to 4, wherein a content weight of the first particulate water absorbent with respect to a content weight of the second particulate water absorbent in the water-absorbing sheet is 1.0 or less and preferably 0.4 or less.

6. The water-absorbing sheet according to any one of claims 1 to 5, wherein a water absorption time of the first particulate water absorbent as determined according to a vortex method is 25 seconds or more, preferably 35 seconds or more and more preferably 48 seconds or more.

7. The water-absorbing sheet according to any one of claims 1 to 6, wherein a water absorption time of the second particulate water absorbent as determined by a vortex method is 40 seconds or more and preferably 43 seconds or more.

8. The water-absorbing sheet according to any one of claims 1 to 7, wherein an overall absorption amount of the second particulate water absorbent is 50.0 g/g or more and preferably 52.0 g/g or more.

9. The water-absorbing sheet according to any one of claims 1 to 8, wherein a GPR of the first particulate water absorbent is 5 g/min or more, preferably 35 g/min or more and more preferably 118 g/min or more.

10. The water-absorbing sheet according to claim 1, wherein the overall absorption amount of the second particulate water absorbent is 50.0 g/g or more, the GPR of the first particulate water absorbent is 5 g/min or more, and the content weight of the second particulate water absorbent with respect to the content weight of the first particulate water absorbent is 0.4 or less and, preferably
the water absorption time of the second particulate water absorbent is 43 seconds or more, and the overall absorption amount of the second particulate water absorbent is 52.0 g/g or more.

11. The water-absorbing sheet according to any one of claims 1 to 10, wherein a surface tension of at least one of the first particulate water absorbent and the second particulate water absorbent is 65 mN/m or more.

12. The water-absorbing sheet according to any one of claims 1 to 11, wherein an average degree of circularity of at least one of the first particulate water absorbent and the second particulate water absorbent is 0.70 or less.

13. The water-absorbing sheet according to any one of claims 1 to 12, wherein at least one of a shape of the first particulate water absorbent and the second particulate water absorbent is an irregular crushed shape.

14. The water-absorbing sheet according to any one of claims 1 to 13, wherein a diffusivity is 38% or higher.

15. The water-absorbing sheet according to any one of claims 1 to 14, wherein the water-permeable sheet is a hydrophilic nonwoven fabric.

16. The water-absorbing sheet according to any one of claims 1 to 15, wherein the water-absorbing layer has an intermediate sheet.

17. The water-absorbing sheet according to any one of claims 1 to 16,
wherein
an amount of use of the adhesive is 0.05 to 2.0 times with respect to a total mass of the first particulate water absorbent and the second particulate water absorbent.

18. An absorbent article comprising the water-absorbing sheet according to any one of claims 1 to 17 interposed between a liquid-permeable sheet and a liquid-impermeable sheet, wherein the liquid-permeable sheet is positioned on the first base material side, and the liquid-impermeable sheet is positioned on the second base material side.

**Patentansprüche**

1.  Wasserabsorbierendes Sheet, das folgendes umfasst:

    ein erstes Basismaterial;
    ein zweites Basismaterial; und
    eine wasserabsorbierende Schicht, die zwischen dem ersten Basismaterial und dem zweiten Basismaterial angeordnet ist,
    wobei das erste Basismaterial ein wasserdurchlässiges Sheet ist, das an einer Seite angeordnet ist, wo eine zu absorbierende Flüssigkeit eingeführt wird,
    die wasserabsorbierende Schicht ein erstes partikelförmiges Wasserabsorptionsmittel aufweist, das sich an einer Seite einer Oberfläche des ersten Basismaterials befindet, wobei die Oberfläche so angeordnet ist, dass sie dem zweiten Basismaterial gegenüberliegt; und ein zweites partikelförmiges Wasserabsorptionsmittel, das sich an einer Seite einer Oberfläche des zweiten Basismaterials befindet, wobei die Oberfläche so angeordnet ist, dass sie dem ersten Basismaterial gegenüberliegt,
    eine gesamte Absorptionsmenge des zweiten partikelförmigen Wasserabsorptionsmittels, die durch die folgende Formel wiedergegeben ist:

    [Mathematische Formel 1]

    $$CRC\ g/g + 0,44 \times AAP2,1kPa\ g/g$$

    48 g/g oder mehr ist und eine nach einer Vortex-Methode bestimmte Wasserabsorptionszeit 35 Sekunden oder mehr ist,
    ein CRC des zweiten partikelförmigen Wasserabsorptionsmittels 30 bis 50 g/g ist und ein AAP2,1kPa 15 bis 35 g/g ist, wobei das CRC gemäß ERT441.2-02 gemessen wird und das AAP2,1kPa gemäß ART442.2-02 gemessen wird, und
    die ersten und zweiten partikelförmigen Wasserabsorptionsmittel mit Hilfe eines Klebstoffs an den ersten und zweiten Basismaterialien befestigt sind.

2.  Wasserabsorbierendes Sheet gemäß Anspruch 1, in dem das CRC des zweiten partikelförmigen Wasserabsorptionsmittels 36 bis 45 g/g ist.

3.  Wasserabsorbierendes Sheet gemäß Anspruch 1 oder 2, in dem das AAP2,1kPa des zweiten partikelförmigen Wasserabsorptionsmittels 23 bis 33 g/g ist.

4.  Wasserabsorbierendes Sheet gemäß mindestens einem der Ansprüche 1 bis 3, in dem ein Anteil der partikelförmigen Wasserabsorptionsmittel pro Einheitsvolumen des wasserabsorbierenden Sheets 50 mg/cm$^3$ oder mehr ist.

5.  Wasserabsorbierendes Sheet gemäß mindestens einem der Ansprüche 1 bis 4, in dem ein Anteilsgewicht des ersten partikelförmigen Wasserabsorptionsmittels in Bezug auf ein Anteilsgewicht des zweiten partikelförmigen Wasserabsorptionsmittels in dem wasserabsorbierenden Sheet 1,0 oder weniger und bevorzugt 0,4 oder weniger ist.

6.  Wasserabsorbierendes Sheet gemäß mindestens einem der Ansprüche 1 bis 5, in dem eine nach einer Vortex-Methode bestimmte Wasserabsorptionszeit des ersten partikelförmigen Wasserabsorptionsmittels 25 Sekunden oder mehr, bevorzugt 35 Sekunden oder mehr und mehr bevorzugt 48 Sekunden oder mehr ist.

7.  Wasserabsorbierendes Sheet gemäß mindestens einem der Ansprüche 1 bis 6, in dem eine nach einer Vortex-Methode bestimmte Wasserabsorptionszeit des zweiten partikelförmigen Wasserabsorptionsmittels 40 Sekunden oder mehr und bevorzugt 43 Sekunden oder mehr ist.

8.  Wasserabsorbierendes Sheet gemäß mindestens einem der Ansprüche 1 bis 7, in dem eine Gesamtabsorptionsmenge des zweiten partikelförmigen Wasserabsorptionsmittels 50,0 g/g oder mehr und bevorzugt 52,0 g/g oder mehr ist.

9.  Wasserabsorbierendes Sheet gemäß mindestens einem der Ansprüche 1 bis 8, in dem ein GPR des ersten partikelförmigen Wasserabsorptionsmittels 5 g/min oder mehr, bevorzugt 35 g/min oder mehr und mehr bevorzugt 118 g/min oder mehr ist.

10. Wasserabsorbierendes Sheet gemäß Anspruch 1, in dem die Gesamtabsorptionsmenge des zweiten partikelförmigen Wasserabsorptionsmittels 50,0 g/g oder mehr ist, das GPR des ersten partikelförmigen Wasserabsorptionsmittels 5 g/min oder mehr ist und das Anteilsgewicht des zweiten partikelförmigen Wasserabsorptionsmittels in Bezug auf das Anteilsgewicht des ersten partikelförmigen Wasserabsorptionsmittels 0,4 oder weniger ist und vorzugsweise
die Wasserabsorptionszeit des zweiten partikelförmigen Wasserabsorptionsmittels 43 Sekunden oder mehr ist und die Gesamtabsorptionsmenge des zweiten partikelförmigen Wasserabsorptionsmittels 52,0 g/g oder mehr ist.

11. Wasserabsorbierendes Sheet gemäß mindestens einem der Ansprüche 1 bis 10, in dem eine Oberflächenspannung des ersten partikelförmigen Wasserabsorptionsmittels und/oder des zweiten partikelförmigen Wasserabsorptionsmittels 65 mN/m oder mehr ist.

12. Wasserabsorbierendes Sheet gemäß mindestens einem der Ansprüche 1 bis 11, in dem ein mittlerer Grad der Rundheit des ersten partikelförmigen Wasserabsorptionsmittels und/oder des zweiten partikelförmigen Wasserabsorptionsmittels 0,70 oder weniger ist.

13. Wasserabsorbierendes Sheet gemäß mindestens einem der Ansprüche 1 bis 12, in dem eine Form des ersten partikelförmigen Wasserabsorptionsmittels und/oder des zweiten partikelförmigen Wasserabsorptionsmittels eine unregelmäßig zerkleinerte Form ist.

14. Wasserabsorbierendes Sheet gemäß mindestens einem der Ansprüche 1 bis 13, in dem ein Diffusionsvermögen 38 % oder höher ist.

15. Wasserabsorbierendes Sheet gemäß mindestens einem der Ansprüche 1 bis 14, in dem das wasserdurchlässige Sheet ein hydrophiles Vliesgewebe ist.

16. Wasserabsorbierendes Sheet gemäß mindestens einem der Ansprüche 1 bis 15, in dem die wasserabsorbierende Schicht ein Zwischensheet aufweist.

17. Wasserabsorbierendes Sheet gemäß mindestens einem der Ansprüche 1 bis 16, in dem
eine Verwendungsmenge des Klebstoffs das 0,05- bis 2,0-fache in Bezug auf eine Gesamtmasse des ersten partikelförmigen Wasserabsorptionsmittels und des zweiten partikelförmigen Wasserabsorptionsmittels ist.

18. Absorbierender Artikel, der das wasserabsorbierende Sheet gemäß mindestens einem der Ansprüche 1 bis 17 zwischen ein flüssigkeitsdurchlässiges Sheet und ein flüssigkeitsundurchlässiges Sheet eingefügt umfasst, wobei das flüssigkeitsdurchlässige Sheet an der ersten Basismaterialseite angeordnet ist und das flüssigkeitsundurchlässige Sheet an der zweiten Basismaterialseite angeordnet ist.


**Revendications**

1. Feuille absorbant l'eau comprenant :

   un premier matériau de base ;
   un second matériau de base ; et
   une couche absorbant l'eau positionnée entre le premier matériau de base et le second matériau de base,
   dans laquelle le premier matériau de base est une feuille perméable à l'eau positionnée sur un côté où un liquide à absorber est introduit,
   la couche absorbant l'eau présente un premier agent particulaire absorbant l'eau localisé sur un côté d'une surface du premier matériau de base, la surface étant agencée pour faire face au second matériau de base ;
   et un second agent particulaire absorbant l'eau localisé sur un côté d'une surface du second matériau de base, la surface étant agencée pour faire face au premier matériau de base,
   une quantité d'absorption globale telle que représentée par la formule suivante :

[Formule mathématique 1]

CRC g/g + 0,44 × AAP2,1kPa g/g

du second agent particulaire absorbant l'eau est de 48 g/g ou plus, et une durée d'absorption d'eau déterminée selon une méthode Vortex est de 35 secondes ou plus,
un CRC du second agent particulaire absorbant l'eau est de 30 à 50 g/g, et un AAP2,1kPa est de 15 à 35 g/g, dans laquelle le CRC est mesuré selon la méthode ERT441.2-02 et l'AAP2,1kPa est mesuré selon la méthode ERT442.2-02, et les premier et second agents particulaires absorbant l'eau sont fixés aux premier et second matériaux de base au moyen d'un adhésif.

2.  Feuille absorbant l'eau selon la revendication 1, dans laquelle le CRC du second agent particulaire absorbant l'eau est de 36 à 45 g/g.

3.  Feuille absorbant l'eau selon la revendication 1 ou la revendication 2, dans laquelle l'AAP2, 1kPa du second agent particulaire absorbant l'eau est de 23 à 33 g/g.

4.  Feuille absorbant l'eau selon l'une quelconque des revendications 1 à 3, dans laquelle une teneur en agents absorbants d'eau particulaires par volume unitaire de la feuille absorbant l'eau est de 50 mg/cm$^3$ ou plus.

5.  Feuille absorbant l'eau selon l'une quelconque des revendications 1 à 4, dans laquelle une teneur en poids du premier agent particulaire absorbant l'eau par rapport à une teneur en poids du second agent particulaire absorbant l'eau dans la feuille absorbant l'eau est de 1,0 ou moins et préférablement de 0,4 ou moins.

6.  Feuille absorbant l'eau selon l'une quelconque des revendications 1 à 5, dans laquelle une durée d'absorption d'eau du premier agent particulaire absorbant l'eau telle que déterminée par une méthode Vortex est de 25 secondes ou plus, préférablement de 35 secondes ou plus et plus préférablement de 48 secondes ou plus.

7.  Feuille absorbant l'eau selon l'une quelconque des revendications 1 à 6, dans laquelle une durée d'absorption d'eau du second agent particulaire absorbant l'eau telle que déterminée par une méthode Vortex est de 40 secondes ou plus et préférablement de 43 secondes ou plus.

8.  Feuille absorbant l'eau selon l'une quelconque des revendications 1 à 7, dans laquelle une quantité d'absorption globale du second agent particulaire absorbant l'eau est de 50,0 g/g ou plus et préférablement de 52,0 g/g ou plus.

9.  Feuille absorbant l'eau selon l'une quelconque des revendications 1 à 8, dans laquelle un GPR du premier agent particulaire absorbant l'eau est de 5 g/min ou plus, préférablement de 35 g/min ou plus et plus préférablement de 118 g/min ou plus.

10. Feuille absorbant l'eau selon la revendication 1, dans laquelle la quantité d'absorption globale du second agent particulaire absorbant l'eau est de 50,0 g/g ou plus, le GPR du premier agent particulaire absorbant l'eau est de 5 g/min ou plus, et la teneur en poids du second agent particulaire absorbant l'eau par rapport à la teneur en poids du premier agent particulaire absorbant l'eau est de 0,4 ou moins et, préférablement
la durée d'absorption d'eau du second agent particulaire absorbant l'eau est de 43 secondes ou plus, et la quantité d'absorption globale du second agent particulaire absorbant l'eau est de 52,0 g/g ou plus.

11. Feuille absorbant l'eau selon l'une quelconque des revendications 1 à 10, dans laquelle une tension superficielle d'au moins un absorbant parmi le premier agent particulaire absorbant l'eau et le second agent particulaire absorbant l'eau est de 65 mN/m ou plus.

12. Feuille absorbant l'eau selon l'une quelconque des revendications 1 à 11, dans laquelle un degré moyen de circularité d'au moins un absorbant parmi le premier agent particulaire absorbant l'eau et le second agent particulaire absorbant l'eau est de 0,70 ou moins.

13. Feuille absorbant l'eau selon l'une quelconque des revendications 1 à 12, dans laquelle au moins une forme parmi le premier agent particulaire absorbant l'eau et le second agent particulaire absorbant l'eau est une forme écrasée irrégulière.

14. Feuille absorbant l'eau selon l'une quelconque des revendications 1 à 13, dans laquelle une diffusivité est de 38 % ou supérieure.

15. Feuille absorbant l'eau selon l'une quelconque des revendications 1 à 14, dans laquelle la feuille perméable à l'eau est un tissu non-tissé hydrophile.

16. Feuille absorbant l'eau selon l'une quelconque des revendications 1 à 15, dans laquelle la couche absorbant l'eau présente une feuille intermédiaire.

17. Feuille absorbant l'eau selon l'une quelconque des revendications 1 à 16, dans laquelle une quantité d'utilisation de l'adhésif est 0,05 à 2,0 fois d'une masse totale du premier agent particulaire absorbant l'eau et du second agent particulaire absorbant l'eau.

18. Article absorbant comprenant la feuille absorbant l'eau selon l'une quelconque des revendications 1 à 17 interposée entre une feuille perméable aux liquides et une feuille imperméable aux liquides, dans lequel la feuille perméable aux liquides est positionnée sur le côté du premier matériau de base, et la feuille imperméable aux liquides est positionnée sur le côté du second matériau de base.

# FIG.1

LIQUID TO BE ABSORBED

⬇

40

14a

16

14b

11

12

13

FIG.2

EP 3 834 789 B1

# FIG.3

EP 3 834 789 B1

# FIG.4

INNER DIAMETER
25mm

5mm

30mm  30mm

35mm  35mm

# FIG.5

# FIG.6

INTRODUCTION OF LIQUID

FUNNEL

LIQUID INJECTION CYLINDER

WATER-ABSORBING SHEET

LIQUID-IMPERMEABLE SHEET

EP 3 834 789 B1

# FIG.7

LIQUID TO BE ABSORBED

40

14a

16

14b

11

12

13

# FIG.8

EP 3 834 789 B1

# FIG.9

WATER-ABSORBING SHEET

LIQUID-IMPERMEABLE SHEET

100mm

400mm

100mm

WATER-ABSORBING SHEET

LIQUID-IMPERMEABLE SHEET

EP 3 834 789 B1

# FIG.10

INNER DIAMETER
25mm

5mm

30mm    30mm

35mm    35mm

# FIG.11

LIQUID INJECTION CYLINDER

WATER-ABSORBING SHEET

LIQUID-IMPERMEABLE SHEET

200mm   200mm

LIQUID INJECTION CYLINDER

50mm   50mm

WATER-ABSORBING SHEET

LIQUID-IMPERMEABLE SHEET

EP 3 834 789 B1

# FIG.12

INTRODUCTION OF LIQUID

FUNNEL

LIQUID INJECTION CYLINDER

WATER-ABSORBING SHEET

LIQUID-IMPERMEABLE SHEET

EP 3 834 789 B1

## FIG.13

INTRODUCE PARTICULATE ABSORBENT

TAPE

SIDE CONTACTING
WITH FIRST BASE
MATERIAL

INTERMEDIATE SHEET

SIDE CONTACTING
WITH SECOND BASE
MATERIAL

850μm MESH

## FIG.14

LIQUID TO BE ABSORBED

40

14a

16

14b

11

12

13

FIG.15

EP 3 834 789 B1

# FIG.16

WATER-ABSORBING SHEET

LIQUID-IMPERMEABLE SHEET

100mm

400mm

100mm

WATER-ABSORBING SHEET

LIQUID-IMPERMEABLE SHEET

EP 3 834 789 B1

INNER DIAMETER
25mm

5mm

30mm 30mm

35mm 35mm

FIG.17

# FIG.18

EP 3 834 789 B1

# FIG.19

INTRODUCTION OF LIQUID

FUNNEL

LIQUID INJECTION CYLINDER

WATER-ABSORBING SHEET

LIQUID-IMPERMEABLE SHEET

EP 3 834 789 B1

# FIG.20

INTRODUCE PARTICULATE ABSORBENT

TAPE

SIDE CONTACTING
WITH FIRST BASE
MATERIAL

INTERMEDIATE SHEET

SIDE CONTACTING
WITH SECOND BASE
MATERIAL

850μm MESH

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2010143635 A
- WO 2013099634 A1
- US 20120203191 A1
- WO 2005092956 A1
- US 5849405 A
- WO 2012174026 A
- WO 2013078109 A
- WO 2015041784 A
- WO 2011117187 A
- WO 2012001117 A
- WO 2012024445 A
- WO 2010004894 A
- WO 2010004895 A
- WO 2010076857 A
- WO 2010082373 A
- WO 2010113754 A
- WO 2011043256 A
- WO 2011086841 A
- WO 2011086842 A
- WO 2011086843 A
- WO 2011086844 A
- WO 2011117997 A
- WO 2011118409 A
- WO 2011136087 A
- WO 2012043546 A
- WO 2013099634 A
- WO 2013099635 A
- JP 2010115406 A
- JP 2002345883 A
- JP H6315501 A
- JP H6190003 A
- JP H6190002 A
- JP H6190001 A
- JP H2252558 A
- JP H2252560 A
- JP H2252561 A
- WO 2014034897 A
- WO 2017170605 A
- WO 2016204302 A
- WO 2014054656 A
- WO 2015152299 A
- WO 2018062539 A
- WO 2012043821 A
- US 5210298 A
- JP 2018150124 A
- JP 2018150125 A
- JP 2018150129 A
- JP 2018185701 A
- JP 2018185706 A
- US 7638570 B

### Non-patent literature cited in the description

- Particles. McGraw-Hill Dictionary of Scientific and Technical Terms. Nikkan Kogyo Shimbun, Ltd, 1996, 1929